# EUROPEAN PATENT SPECIFICATION

(11) **EP 4 010 354 B1**
(45) Date of publication and mention of the grant of the patent: **18.02.2026**
(21) Application number: 20750683.3
(22) Date of filing: 07.08.2020
(51) Int. Cl.: C07K 14/00, C12N 15/11, C12N 15/63

(54) **STEREOSELECTIVE PH RESPONSIVE PEPTIDE DENDRIMERS FOR NUCLEIC ACID TRANSFECTION**
STEREOSELEKTIVE PH-EMPFINDLICHE PEPTIDDENDRIMERE FÜR DIE NUKLEINSÄURETRANSFEKTION
DENDRIMÈRES PEPTIDIQUES STÉRÉOSÉLECTIFS SENSIBLES AU PH POUR LA TRANSFECTION D'ACIDES NUCLÉIQUES

(30) Priority: 07.08.2019 EP 19190626
(43) Date of publication of application: 15.06.2022
(73) Proprietor: Universität Bern, 3012 Bern (CH)
(72) Inventor: REYMOND, Jean-Louis, 1630 Bulle (CH); HEITZ, Marc, 68870 Bartenheim (FR); DARBRE, Tamis, 3063 Ittigen (CH); ZAMOLO, Susanna, 27029 Vigevano (IT)
(74) Representative: Schulz Junghans Patentanwälte PartGmbB
(86) International application number: PCT/EP2020/072329
(87) International publication number: WO 2021/023891

(56) References cited:
- WO-A1-2015/144928
- GANESH R. KOKIL ET AL: "Self-assembling asymmetric peptide-dendrimer micelles - a platform for effective and versatile in vitro nucleic acid delivery", SCIENTIFIC REPORTS, vol. 8, no. 1, 19 March 2018 (2018-03-19), XP055738070, DOI: 10.1038/s41598-018-22902-9
- MARC HEITZ ET AL: "Peptide Dendrimer-Lipid Conjugates as DNA and siRNA Transfection Reagents: Role of Charge Distribution Across Generations", CHIMIA INTERNATIONAL JOURNAL FOR CHEMISTRY, vol. 71, no. 4, 26 April 2017 (2017-04-26), CH, pages 220 - 225, XP055738085, ISSN: 0009-4293, DOI: 10.2533/chimia.2017.220
- THISSA N. SIRIWARDENA ET AL: "Lipidated Peptide Dendrimers Killing Multidrug-Resistant Bacteria", JOURNAL OF THE AMERICAN CHEMICAL SOCIETY, vol. 140, no. 1, 19 December 2017 (2017-12-19), US, pages 423 - 432, XP055738061, ISSN: 0002-7863, DOI: 10.1021/jacs.7b11037
- MARC HEITZ ET AL: "Stereoselective pH Responsive Peptide Dendrimers for siRNA Transfection", BIOCONJUGATE CHEMISTRY, vol. 30, no. 8, 9 August 2019 (2019-08-09), pages 2165 - 2182, XP055738013, ISSN: 1043-1802, DOI: 10.1021/acs.bioconjchem.9b00403

## Description

### Background of the invention

Transfection of cells with nucleic acids enables targeted expression of foreign genes or knock-down of selected proteins and is an essential procedure in biological and biomedical research also envisioned for gene therapy applications. Due to safety concerns with biological transfection agents such as viruses, a broad variety of non-viral transfection reagents have been developed. These reagents are amphiphilic polycations consisting of liposome forming lipids (including lipofectamine L2000, one of the best performing reagent used here as reference), polymers, modified poly(amidoamine), carbosilane or bolaamphiphile dendrimers, nanoparticles, proteins dendrimer conjugates, protein cages, or peptides. They complex nucleic acids to form aggregates of 50-150 nm in size, which enter cells by endocytosis, escape endosomes, and deliver their cargo into the cytosol (for siRNA) or nucleus (for DNA).

GANESH R. KOKIL ET AL: "Self-assembling asymmetric peptide-dendrimer micelles - a platform for effective and versatile in vitro nucleic acid delivery", SCIENTIFIC REPORTS, vol. 8, no. 1, 19 March 2018, DOI: 10.1038/s41598-018-22902-9, disclose a library of lipidated asymmetric peptide dendrimers which varied in charge density and head group (lysine or arginine) chemistry. The peptide dendrimers are used in a method for nucleic acid transfection, such as siRNA transfection.

MARC HEITZ ET AL: "Peptide Dendrimer-Lipid Conjugates as DNA and siRNA Transfection Reagents: Role of Charge Distribution Across Generations", CHIMIA INTERNATIONAL JOURNAL FOR CHEMISTRY, vol. 71, no. 4, 26 April 2017, pages 220-225, DOI: 10.2533/chimia.2017.220D2 report on easily modifiable transfection reagents in the form of peptide dendrimers. These dendrimers self-assemble with DNA or siRNA and lipofectin to form nanoparticles which efficiently enter mammalian cells and liberate their nucleic acid cargo.

Unfortunately, almost all reagents mentioned above have either undisclosed or nonhomogeneous structures, or require complex multistep syntheses with non-standard reagents, which limits reproducibility and transferability of results.

Known Peptide dendrimers are used together with lipofectin as a helper lipid, however these dendrimers were inactive when used alone.

### Description of the invention

### Terms and Definitions

The term "amino acid" relates to naturally occurring amino acids, in particular proteinogenic amino acids, as well as to unnatural amino acids. The amino acid may be in D- or L-configuration.

Based on the chemical properties of their side chains, amino acids are classified, e.g. as being hydrophobic or cationic.

A hydrophobic amino acid in the context of the present specification is any alpha-aminocarboxylic acid having a side chain without hydrogen bond donors or acceptors. Hydrophobic amino acids include, without being limited to leucine (Leu), phenylalanine (Phe), tryptophan (Trp), cysteine (Cys), norleucine (Nle) and amino octanoic acid (Aoc). Cysteine is classified as hydrophobic according to Nagano N, Ota M, Nishikawa K (1999) "Strong hydrophobic nature of cysteine residues in proteins". FEBS Lett. 458 (1): 69-71.

An amino acid comprising a cationic side chain in the context of the present specification is an alpha-amino carboxylic acid having a side chain comprising a chemical functional group present as a cation under physiological pH. Cationic amino acids include, without being limited to, arginine (Arg), histidine (His) and lysine (Lys).

Amino acid sequences are given from N-termini to C-terminus. The terminal carboxy group of a peptide dendrimer mentioned herein may be a carboxylic acid or a carboxylate (-COO⁻). The C-terminus may also be a carboxamide (-CONH₂) group.

Amino acids may be given in the three-letter code (Stryer, Biochemistry, 3rd ed. p. 21) or in the one-letter code. If not indicated otherwise, the amino acid may be in the D- or L-configuration. Particularly in the experimental section, the one-letter code in upper case letters refers to L-amino acid enantiomers or diastereomers while the one-letter code in lower letters refers to D-amino acid enantiomers or diastereomers.

### Description

The present invention relates to a peptide dendrimer of formula 1,

(D³)₈-(B³-D²)₄-(B²-D¹)₂-B¹-Z (1)

with Z being -X(Y¹) (1a), -X(Y²)X(Y²) (1b), -X(Y³)Cys (1c) or -HP (1d) wherein,
- X is selected from Lys, Orn, DAB (2,4 diaminobutyric acid), DAP (2,3 diaminopropionic acid), Glu or Asp,, which is coupled to B¹ via its N-terminus and which is coupled to Y via its side chain,
- Y' is selected from a -C(=O)-Cᵥ-alkyl, a -C(=O)-Cᵥ-alkenyl in case of X being Lys, Orn, DAB or DAP, and v is
   - between 17 and 27 particularly between 19 and 27, more particularly between 21 and 25 in case of X being Lys,
   - between 18 and 28 particularly between 20 and 28, more particularly between 22 and 26 in case of X being Orn,
   - between 19 and 29 particularly between 21 and 29, more particularly between 23 and 27 in case of X being DAB,
   - between 20 and 30 particularly between 22 and 30, more particularly between 24 and 28 in case of X being DAP, or
   Y¹ is selected from a -(NH)-Cₐ-alkyl, a -(NH)-Cₐ-alkenyl, -(N)-(C_{d}-alkyl)₂ or a -(N)-(C_{d}-alkenyl)₂ in case of X being Glu or Asp, wherein
   - the sum of d is between 19 and 29, particularly between 21 and 29, more particularly between 23 and 27 in case of X being Glu,
   - the sum of d is between 20 and 30, particularly between 22 and 30, more particularly between 24 and 28, in case of X being Asp, and wherein
   - a is between 19 and 29 particularly between 21 and 29, more particularly between 23 and 27, in case of X being Glu, or
   - a is between 20 and 30, particularly between 22 and 30, more particularly between 24 and 28, in case of X being Asp,
- each Y² is independently selected from a -C(=O)-C_{w}-alkyl and a -C(=O)-C_{w}-alkenyl, in case of X being Lys, Orn, DAB or DAP and the sum of w is
   - between 18 and 36, particularly between 22 and 36, more particularly between 28 and 36, in case of X being Lys,
   - between 20 and 38, particularly between 24 and 38, more particularly between 30 and 38, in case of X being Orn,
   - between 22 and 40, particularly between 26 and 40, more particularly between 32 and 40, in case of X being DAB,
   - between 24 and 42, particularly between 28 and 42, more particularly between 34 and 42, in case of X being DAP, or
   Y² is selected from a -(NH)-C_{b}-alkyl, a -(NH)-C_{b}-alkenyl, -(N)-(Cₑ-alkyl)₂ or a -(N)-(Cₑ-alkenyl)₂ in case of X being Glu or Asp, wherein
   the sum of e or b is between 22 and 40, particularly between 26 and 40, more particularly between 32 and 40 in case of X being Glu, wherein
   the sum of e or b is between 24 and 42, particularly between 28 and 42, more particularly between 34 and 42, in case of X being Asp,
- Y³ is selected from a -C(=O)-Cₓ-alkyl and a -C(=O)-Cₓ-alkenyl, in case of X being Lys, Orn, DAB or DAP and x is
   - between 15 and 21, particularly between 15 and 17, in case of X being Lys,
   - between 16 and 22, particularly between 16 and 18, in case of X being Orn
   - between 17 and 23, particularly between 17 and 19, in case of X being DAB between 18 and 24, particularly between 18 and 20, in case of X being DAPY³ is selected from a -(NH)-C_{c}-alkyl, a -(NH)-C_{c}-alkenyl, -(N)-(C_{f}-alkyl)₂ or a -(N)-(C_{f}-alkenyl)₂ in case of X being Glu or Asp, wherein
   - the sum of f is between 17 and 23, particularly between 17 and 19, in case of X being Glu,
   - the sum of f is between 18 and 24, particularly between 18 and 20, in case of X being Asp,
   - c is between 17 and 23, particularly between 17 and 19, in case of X being Glu,
   - c is between 18 and 24, particularly between 18 and 20, in case of X being Asp,
- HP is a hydrophobic peptide consisting of 3 to 5 hydrophobic amino acids,
- each B is Lys
- each D independently from any other D is a dipeptide consisting of one hydrophobic amino acid and one cationic amino acid (HC or CH), a dipeptide consisting of two cationic amino acids (CC) or a dipeptide consisting of two hydrophobic amino acids (HH).

In certain embodiments, the invention relates to a peptide dendrimer of formula 1,

(D³)₈-(B³-D²)₄-(B²-D¹)₂-B¹-Z (1)

with Z being -X(Y¹) (1a), -X(Y²)X(Y²) (1b), -X(Y³)Cys (1c), -HP (1d), or -X(Y⁴)Ala, in particular with Z being -X(Y¹) (1a), -X(Y²)X(Y²) (1b), -X(Y³)Cys (1c) or -HP (1d)
wherein,
- X is selected from Lys, Orn, DAB, DAP, Glu or Asp, which is coupled to B¹ via its N-terminus and which is coupled to Y via its side chain,
- Y¹ is selected from a -C(=O)-Cᵥ-alkyl, a -C(=O)-Cᵥ-alkenyl in case of X being Lys, Orn, DAB or DAP, and v is
   - between 15 and 27, particularly between 17 and 27, more particularly between 19 and 27, even more particularly between 21 and 25, in case of X being Lys,
   - between 18 and 28, particularly between 20 and 28, more particularly between 22 and 26, in case of X being Orn,
   - between 19 and 29, particularly between 21 and 29, more particularly between 23 and 27, in case of X being DAB,
   - between 20 and 30, particularly between 22 and 30, more particularly between 24 and 28, in case of X being DAP, or
   Y¹ is selected from a -(NH)-Cₐ-alkyl, a -(NH)-Cₐ-alkenyl, -(N)-(C_{d}-alkyl)₂ or a -(N)-(C_{d}-alkenyl)₂ in case of X being Glu or Asp, wherein
   - the sum of d is between 19 and 29, particularly between 21 and 29, more particularly between 23 and 27 in case of X being Glu,
   - the sum of d is between 20 and 30, particularly between 22 and 30, more particularly between 24 and 28, in case of X being Asp, and wherein
   - a is between 19 and 29 particularly between 21 and 29, more particularly between 23 and 27, in case of X being Glu, or
   - a is between 20 and 30, particularly between 22 and 30, more particularly between 24 and 28, in case of X being Asp,
- each Y² is independently selected from a -C(=O)-C_{w}-alkyl and a -C(=O)-C_{w}-alkenyl, in case of X being Lys, Orn, DAB or DAP and the sum of w is
   - between 18 and 36, particularly between 22 and 36, more particularly between 28 and 36, in case of X being Lys,
   - between 20 and 38, particularly between 24 and 38, more particularly between 30 and 38, in case of X being Orn,
   - between 22 and 40, particularly between 26 and 40, more particularly between 32 and 40, in case of X being DAB,
   - between 24 and 42, particularly between 28 and 42, more particularly between 34 and 42, in case of X being DAP, or
   Y² is selected from a -(NH)-C_{b}-alkyl, a -(NH)-C_{b}-alkenyl, -(N)-(Cₑ-alkyl)₂ or a -(N)-(Cₑ-alkenyl)₂ in case of X being Glu or Asp, wherein
   the sum of e or b is between 22 and 40, particularly between 26 and 40, more particularly between 32 and 40 in case of X being Glu, wherein
   the sum of e or b is between 24 and 42, particularly between 28 and 42, more particularly between 34 and 42, in case of X being Asp,
- Y³ and Y⁴ are independently selected from a -C(=O)-Cₓ-alkyl and a -C(=O)-Cₓ-alkenyl, in case of X being Lys, Orn, DAB or DAP and x is
   - between 15 and 21, particularly between 15 and 17, in case of X being Lys,
   - between 16 and 22, particularly between 16 and 18, in case of X being Orn
   - between 17 and 23, particularly between 17 and 19, in case of X being DAB between 18 and 24, particularly between 18 and 20, in case of X being DAPY³ is selected from a -(NH)-C_{c}-alkyl, a -(NH)-C_{c}-alkenyl, -(N)-(C_{f}-alkyl)₂ or a -(N)-(C_{f}-alkenyl)₂ in case of X being Glu or Asp, wherein
   - the sum of f is between 17 and 23, particularly between 17 and 19, in case of X being Glu,
   - the sum of f is between 18 and 24, particularly between 18 and 20, in case of X being Asp,
   - c is between 17 and 23, particularly between 17 and 19, in case of X being Glu,
   - c is between 18 and 24, particularly between 18 and 20, in case of X being Asp,
- HP is a hydrophobic peptide consisting of 3 to 5 hydrophobic amino acids,
- each B is Lys
- each D independently from any other D is a dipeptide consisting of one hydrophobic amino acid and one cationic amino acid (HC or CH), a dipeptide consisting of two cationic amino acids (CC) or a dipeptide consisting of two hydrophobic amino acids (HH).

In certain embodiments, the invention relates to a peptide dendrimer of formula 1,

(D³)₈-(B³-D²)₄-(B²-D¹)₂-B¹-Z (1)

with Z being -X(Y¹) (1a), -X(Y²)X(Y²) (1b), -X(Y³)Cys (1c) or -HP (1d)
wherein,
- X is selected from Lys, Orn, DAB, DAP, Glu or Asp,, which is coupled to B¹ via its N-terminus and which is coupled to Y via its side chain,
- Y' is selected from a -C(=O)-Cᵥ-alkyl, a -C(=O)-Cᵥ-alkenyl in case of X being Lys, Orn, DAB or DAP, and v is
   - between 15 and 27, particularly between 17 and 27, more particularly between 19 and 27, even more particularly between 21 and 25, in case of X being Lys,
   - between 18 and 28, particularly between 20 and 28, more particularly between 22 and 26, in case of X being Orn,
   - between 19 and 29, particularly between 21 and 29, more particularly between 23 and 27, in case of X being DAB,
   - between 20 and 30, particularly between 22 and 30, more particularly between 24 and 28, in case of X being DAP, or
   Y' is selected from a -(NH)-Cₐ-alkyl, a -(NH)-Cₐ-alkenyl, -(N)-(C_{d}-alkyl)₂ or a -(N)-(C_{d}-alkenyl)₂ in case of X being Glu or Asp, wherein
   - the sum of d is between 19 and 29, particularly between 21 and 29, more particularly between 23 and 27 in case of X being Glu,
   - the sum of d is between 20 and 30, particularly between 22 and 30, more particularly between 24 and 28, in case of X being Asp, and wherein
   - a is between 19 and 29 particularly between 21 and 29, more particularly between 23 and 27, in case of X being Glu, or
   - a is between 20 and 30, particularly between 22 and 30, more particularly between 24 and 28, in case of X being Asp,
- each Y² is independently selected from a -C(=O)-C_{w}-alkyl and a -C(=O)-C_{w}-alkenyl, in case of X being Lys, Orn, DAB or DAP and the sum of w is
   - between 18 and 36, particularly between 22 and 36, more particularly between 28 and 36, in case of X being Lys,
   - between 20 and 38, particularly between 24 and 38, more particularly between 30 and 38, in case of X being Orn,
   - between 22 and 40, particularly between 26 and 40, more particularly between 32 and 40, in case of X being DAB,
   - between 24 and 42, particularly between 28 and 42, more particularly between 34 and 42, in case of X being DAP, or
   Y² is selected from a -(NH)-C_{b}-alkyl, a -(NH)-C_{b}-alkenyl, -(N)-(Cₑ-alkyl)₂ or a -(N)-(Cₑ-alkenyl)₂ in case of X being Glu or Asp, wherein
   the sum of e or b is between 22 and 40, particularly between 26 and 40, more particularly between 32 and 40 in case of X being Glu, wherein
   the sum of e or b is between 24 and 42, particularly between 28 and 42, more particularly between 34 and 42, in case of X being Asp,
- Y³ and Y⁴ are independently selected from a -C(=O)-Cₓ-alkyl and a -C(=O)-Cₓ-alkenyl, in case of X being Lys, Orn, DAB or DAP and x is
   - between 15 and 21, particularly between 15 and 17, in case of X being Lys,
   - between 16 and 22, particularly between 16 and 18, in case of X being Orn
   - between 17 and 23, particularly between 17 and 19, in case of X being DAB between 18 and 24, particularly between 18 and 20, in case of X being DAPY³ is selected from a -(NH)-C_{c}-alkyl, a -(NH)-C_{c}-alkenyl, -(N)-(C_{f}-alkyl)₂ or a -(N)-(C_{f}-alkenyl)₂ in case of X being Glu or Asp, wherein
   - the sum of f is between 17 and 23, particularly between 17 and 19, in case of X being Glu,
   - the sum of f is between 18 and 24, particularly between 18 and 20, in case of X being Asp,
   - c is between 17 and 23, particularly between 17 and 19, in case of X being Glu,
   - c is between 18 and 24, particularly between 18 and 20, in case of X being Asp,
- HP is a hydrophobic peptide consisting of 3 to 5 hydrophobic amino acids,
- each B is Lys
- each D independently from any other D is a dipeptide consisting of one hydrophobic amino acid and one cationic amino acid (HC or CH), a dipeptide consisting of two cationic amino acids (CC) or a dipeptide consisting of two hydrophobic amino acids (HH).

The peptide dendrimer is suitable for the transfection of cells, particularly mammalian cells.

Peptide dendrimers are readily obtained as pure products in a single solid-phase peptide synthesis (SPPS) run followed by purification by HPLC. Peptide dendrimers represent a superior alternative to linear peptides as well-defined and easily transferable transfection reagents.

Dendrimer self-aggregation via intermolecular β-sheet formation at neutral pH enables nucleic acid complexation to form nanoparticles which enter cells by endocytosis. Endosome acidification triggers protonation of amino termini and rearrangement to an α-helical conformation forming smaller dendrimer/nucleic acid nanoparticles, which escape the endosome and release their nucleic acid cargo in the cytosol of the cell.

The β-sheet formation is favored by a hydrophobic core. Therefore, Z is either a hydrophobic moiety comprising acylated amino acids or a hydrophobic peptide consisting of at least 3 or more hydrophobic amino acids.

While the core is important for the self-aggregation, the branching structure of the peptide dendrimer is important for complexation of nucleic acid molecules. Several nucleic acid molecules are complexed by several self-aggregates forming a nanoparticle.

The branching structure of the peptide dendrimer starts with a first trifunctional branching unit B¹, e.g. a lysine, which is bound via its C-terminus to Z and via its N-terminus to one dipeptide D¹ and via its side chain to another dipeptide D¹. Each dipeptide D¹ is bound to further branching units B². The core unit Z may also refer to as generation G⁰. The first branching unit B¹ bound to the dipeptides D¹ forms the first generation G¹, the second branching units B² bound to the dipeptides D² form the second generation G² and the third branching units B³ bound to the dipeptides D³ form the third generation G³.

The core unit Z is a hydrophobic moiety comprising the formulas 1a to 1d. The moiety X is lysine or glutamic acid, is coupled to B¹ via its N-terminus and to a further moiety X or cysteine, via its C-terminus. The side chain of the X is acylated, for example by reaction of the carboxylate group of a fatty acid with the amino group of the side chain or a fatty amine with carboxylate group of the side chain.

The fatty acid or fatty amine may be saturated or unsaturated and linear or branched, particularly saturated and linear.

Alternatively, the core unit Z may be formed by three or more hydrophobic amino acids (hydrophobic peptide). The hydrophobic peptide may comprise various hydrophobic amino acids, e.g. Leu-Phe-Nle, or identical amino acids, e.g. Leu-Leu-Leu.

In certain embodiments X is Lys providing a peptide dendrimer of formula 1,

(D³)₈-(B³-D²)₄-(B²-D¹)₂-B¹-Z (1)

with Z being -X(Y¹) (1a), -X(Y²)X(Y²) (1b), -X(Y³)Cys (1c), -HP (1d), or -X(Y⁴)Ala, in particular with Z being -X(Y¹) (1a), -X(Y²)X(Y²) (1b), -X(Y³)Cys (1c) or -HP (1d), wherein,
- X is Lys, which is coupled to B¹ via its N-terminus and which is coupled to Y via its side chain,
- Y¹ is selected from a -C(=O)-Cᵥ-alkyl, a -C(=O)-Cᵥ-alkenyl, wherein v is between 15 and 27, particularly between 17 and 27, more particularly between 19 and 27, even more particularly between 21 and 25,
- each Y² is independently selected from a -C(=O)-C_{w}-alkyl and a -C(=O)-C_{w}-alkenyl, wherein the sum of w is between 18 and 36, particularly between 22 and 36, more particularly between 28 and 36,
- Y³ and Y⁴ are indenpendetly selected from a -C(=O)-Cₓ-alkyl and a -C(=O)-Cₓ-alkenyl, wherein x is between 15 and 21, particularly between 15 and 17,
- HP is a hydrophobic peptide consisting of 3 to 5 hydrophobic amino acids,
- each B is Lys
- each D independently from any other D is a dipeptide consisting of one hydrophobic amino acid and one cationic amino acid (HC or CH), consisting of two cationic amino acids (CC) or a dipeptide consisting of two hydrophobic amino acids (HH).

In certain embodiments X is Lys providing a peptide dendrimer of formula 1,

(D³)₈-(B³-D²)₄-(B²-D¹)₂-B¹-Z (1)

with Z being -X(Y¹) (1a), -X(Y²)X(Y²) (1b), -X(Y³)Cys (1c) or -HP (1d), wherein,
- X is Lys, which is coupled to B¹ via its N-terminus and which is coupled to Y via its side chain,
- Y¹ is selected from a -C(=O)-Cᵥ-alkyl, a -C(=O)-Cᵥ-alkenyl, wherein v is between 17 and 27 particularly between 19 and 27, more particularly between 21 and 25,
- each Y² is independently selected from a -C(=O)-C_{w}-alkyl and a -C(=O)-C_{w}-alkenyl, wherein the sum of w is between 18 and 36, particularly between 22 and 36, more particularly between 28 and 36,
- Y³ is selected from a -C(=O)-Cₓ-alkyl and a -C(=O)-Cₓ-alkenyl, wherein x is between 15 and 21, particularly between 15 and 17,
- HP is a hydrophobic peptide consisting of 3 to 5 hydrophobic amino acids,
- each B is Lys
- each D independently from any other D is a dipeptide consisting of one hydrophobic amino acid and one cationic amino acid (HC or CH), consisting of two cationic amino acids (CC) or a dipeptide consisting of two hydrophobic amino acids (HH).

High transfection efficiency may be achieved when using peptide dendrimers having a lipidated moiety Z, i.e. Z comprises one or two amino acids X coupled to a fatty acid or fatty amine Y via their side chains. Particularly, the C-terminus of the C-terminally amino acid X is a carboxamide (-C(=O)-NH₂).

In certain embodiments, the peptide dendrimer is of formula 1a

(D³)₈-(B³-D²)₄-(B²-D¹)₂-B¹- X(Y¹) (1a),

wherein,
- X is selected from Lys or Glu, particularly X is Lys, which is coupled to B¹ via its N-terminus and which is coupled to Y via its side chain,
- Y¹ is selected from a -C(=O)-Cᵥ-alkyl, a -C(=O)-Cᵥ-alkenyl in case of X being Lys, and Y¹ is selected from a -(NH)-Cₐ-alkyl, a -(NH)-Cₐ-alkenyl, -(N)-(C_{d}-alkyl)₂ or a -(N)-(C_{d}-alkyl)₂ in case of X being Glu, wherein v is between 15 and 27, and wherein a is between 19 and 29 particularly between 21 and 29, more particularly between 23 and 27, particularly Y¹ is selected from a -C(=O)-Cᵥ-alkyl, a -C(=O)-Cᵥ-alkenyl, wherein v is between 17 and 27 particularly between 19 and 27, more particularly between 21 and 25,
- each B is Lys
- each D independently from any other D is a dipeptide consisting of one hydrophobic amino acid and one cationic amino acid (HC or CH), consisting of two cationic amino acids (CC) or a dipeptide consisting of two hydrophobic amino acids (HH).

In certain embodiments, the peptide dendrimer is of formula 1a

(D³)₈-(B³-D²)₄-(B²-D¹)₂-B¹- X(Y¹) (1a),

wherein,
- X is selected from Lys or Glu, particularly X is Lys, which is coupled to B¹ via its N-terminus and which is coupled to Y via its side chain,
- Y¹ is selected from a -C(=O)-Cᵥ-alkyl, a -C(=O)-Cᵥ-alkenyl in case of X being Lys, and Y¹ is selected from a -(NH)-Cₐ-alkyl, a -(NH)-Cₐ-alkenyl, -(N)-(C_{d}-alkyl)₂ or a -(N)-(C_{d}-alkyl)₂ in case of X being Glu, wherein v is between 17 and 27 particularly between 19 and 27, more particularly between 21 and 25, wherein the sum of d is between 19 and 29, particularly between 21 and 29, more particularly between 23 and 27, and wherein a is between 19 and 29 particularly between 21 and 29, more particularly between 23 and 27, particularly Y¹ is selected from a -C(=O)-Cᵥ-alkyl, a -C(=O)-Cᵥ-alkenyl, wherein v is between 17 and 27 particularly between 19 and 27, more particularly between 21 and 25,
- each B is Lys
- each D independently from any other D is a dipeptide consisting of one hydrophobic amino acid and one cationic amino acid (HC or CH), consisting of two cationic amino acids (CC) or a dipeptide consisting of two hydrophobic amino acids (HH).

In certain embodiments, the peptide dendrimer is of formula 1b

(D³)₈-(B³-D²)₄-(B²-D¹)₂-B¹-X(Y²)X(Y²) (1b),

wherein,
- X is selected from Lys or Glu, particularly X is Lys, which is coupled to B¹ via its N-terminus and which is coupled to Y via its side chain,
- each Y² is independently selected from a -C(=O)-C_{w}-alkyl and a -C(=O)-C_{w}-alkenyl, in case of X being Lys, and Y² is selected from a -(NH)-C_{b}-alkyl, a -(NH)-C_{b}-alkenyl, - (N)-(Cₑ-alkyl)₂ or a -(N)-(Cₑ-alkyl)₂ in case of X being Glu, wherein the sum of w is between 18 and 36, particularly between 22 and 36, more particularly between 28 and 36, wherein the sum of e is between 22 and 40, particularly between 26 and 40, more particularly between 32 and 40, wherein the sum of b is between 22 and 40, particularly between 26 and 40, more particularly between 32 and 40, particularly each Y² is independently selected from a -C(=O)-C_{w}-alkyl and a -C(=O)-C_{w}-alkenyl, , wherein the sum of w is between 18 and 36, particularly between 22 and 36, more particularly between 28 and 36,
- each B is Lys
- each D independently from any other D is a dipeptide consisting of one hydrophobic amino acid and one cationic amino acid (HC or CH), consisting of two cationic amino acids (CC) or a dipeptide consisting of two hydrophobic amino acids (HH).

In certain embodiments, the number of carbon atoms of one moiety Y² equals the number of carbon atoms of the other moiety Y².

Particularly the DNA transfection efficiency may be further increased by using peptide dendrimers comprising a cysteine in the hydrophobic moiety Z. For example, Z may consist of an amino acid X (e.g. Lys or Glu) coupled via its side chain to a fatty acid or fatty amine Y and coupled via its C-terminus to a cysteine. The C-terminus of the cysteine may particularly be a carboxamide (-C(=O)-NH₂).

In certain embodiments, the peptide dendrimer is of formula 1c

(D³)₈-(B³-D²)₄-(B²-D¹)2-B¹-X(Y³)Cys (1c),

wherein,
- X is selected from Lys or Glu, particularly X is Lys, which is coupled to B¹ via its N-terminus and which is coupled to Y via its side chain,
- Y³ is selected from a -C(=O)-Cₓ-alkyl and a -C(=O)-Cₓ-alkenyl, in case of X being Lys, and Y³ is selected from a -(NH)-C_{c}-alkyl, a -(NH)-C_{c}-alkenyl, -(N)-(C_{f}-alkyl)₂ or a -(N)-(C_{f}-alkyl)₂ in case of X being Glu, wherein x is between 15 and 21, particularly between 15 and 17, wherein the sum of f is between 17 and 23, particularly between 17 and 19, wherein c is between 17 and 23, particularly between 17 and 19, particularly Y³ is selected from a -C(=O)-Cₓ-alkyl and a -C(=O)-Cₓ-alkenyl, wherein x is between 15 and 21, particularly between 15 and 17,
- each B is Lys
- each D independently from any other D is a dipeptide consisting of one hydrophobic amino acid and one cationic amino acid (HC or CH), consisting of two cationic amino acids (CC) or a dipeptide consisting of two hydrophobic amino acids (HH).

Peptide dendrimers comprising a hydrophobic peptide as moiety Z allow a straightforward synthesis as only amino acids are required as building blocks. Furthermore, such peptide dendrimers are more biocompatible than peptide dendrimers comprising a lipidated moiety Z.

In certain embodiments, the peptide dendrimer is of formula 1d,

(D³)₈-(B³-D²)₄-(B²-D¹)₂-B¹-HP (1d),

wherein,
- HP is a hydrophobic peptide consisting of 3 to 5 hydrophobic amino,
- each B is Lys
- each D independently from any other D is a dipeptide consisting of one hydrophobic amino acid and one cationic amino acid (HC or CH), consisting of two cationic amino acids (CC) or a dipeptide consisting of two hydrophobic amino acids (HH).

In certain embodiments, the hydrophobic peptide consists of hydrophobic amino acids independently selected from Cys, Leu, Trp, Phe, Nle (norleucine), Aoc (amino octanoic acid).

In certain embodiments, the hydropobic peptide consists of hydrophobic amino acids independently selected from Leu, Phe, Trp, Nle and Aoc.

In certain embodiments, the hydrophobic peptide consists of identical hydrophobic amino acids.

The C-terminus of the peptide dendrimer may be a carboxamide instead of a carboxylate. The carboxamide further adds to the hydrophobicity of the moiety Z whereas a negatively charged carboxylate would make the core less hydrophobic. As described above, transfection requires the self-assembly of peptide dendrimers which is mainly mediated by the hydrophobic core Z. A C-terminally carboxamide may be obtained by using a Rink-amide resin during the synthesis of the peptide dendrimer.

In certain embodiments, the C-terminus of the peptide dendrimer is a carboxamide.

As described above, the branching of the dendrimer mediates nanoparticle formation with other self-aggregated peptide dendrimers and nucleic acid molecules. For this, the ratio of cationic and hydrophobic amino acids is important.

In certain embodiments, each D independently from any other D is selected a dipeptide consisting of one hydrophobic amino acid and one cationic amino acid (HC or CH), a dipeptide consisting of two cationic amino acids (CC) or a dipeptide consisting of two hydrophobic amino acids (HH).

The branching moieties of the peptide dendrimer release the nucleic acids in pH-dependent manner. Particularly His may probably contribute to a proton sponge effect helping the release of the nucleic acids.

In certain embodiments, the dipeptide HH is selected from Leu and Nle, particularly Leu.

In certain embodiments, the dipeptide HC or CH, particularly CH, is selected from Leu and Nle, particularly Leu, for H and C is selected from Lys, Arg and His, particularly Lys.

In certain embodiments, the dipeptide CC is selected from Lys, Arg and His, wherein at least one C is His.

In certain embodiments, D¹ is the dipeptide HH and H is selected from Leu and Nle, particularly Leu.

In certain embodiments, D¹ is the dipeptide HC or CH, particularly CH, and H is selected from Leu and Nle, particularly Leu, and C is Lys.

In certain embodiments, D² is the dipeptide HC or CH, particularly CH, and H is Leu and C is selected from Lys, Arg and His, particularly Lys or Arg, more particularly Lys.

In certain embodiments, D² is the dipeptide CC, C is selected from Lys, Arg and His, particularly Lys, wherein at least one C is His.

In certain embodiments, D³ is the dipeptide HC or CH, particularly CH, and H is Leu and C is selected from Lys, Arg and His, particularly Lys or Arg, more particularly Lys.

In certain embodiments, D³ is the dipeptide CC, C is selected from Lys, Arg and His, particularly Lys, wherein at least one C is His.

In certain embodiments, each D independently from any other D is selected from a dipeptide consisting of one hydrophobic amino acid and one cationic amino (HC or CH, particularly CH), and a dipeptide consisting of two hydrophobic amino acids (HH).

In certain embodiments, D¹ is a dipeptide consisting of two hydrophobic amino acids (HH) and D² and D³ are each a dipeptide consisting of one cationic amino acid and one hydrophobic amino acid (CH). These compounds are particularly suited for siRNA transfection.

In certain embodiments, each D¹, D² and D³ are a dipeptide consisting of one cationic amino acid and one hydrophobic amino acid (CH). These compounds are particularly suited for DNA transfection

In certain embodiments, the amino acids are independently from each other selected from (L)-amino acids and (D)-amino acids.

In certain embodiments is the peptide homochiral.

The present invention relates also to a method for transfecting a cell *ex vivo* comprising the steps of
a. providing a transfection mix comprising a peptide dendrimer according to any one of the preceding claims and a nucleic acid,
b. contacting a cell with the transfection mix yielding a transfected cell.

In certain embodiments, is the transfection mix prepared by mixing a first solution comprising the peptide dendrimer according to any one of the preceding claims and a second solution comprising the nucleic acid.

In certain embodiments, is the nucleic acid is or RNA, particularly circular DNA (plasmid/vector), linear DNA (cDNA), linear RNA (siRNA, saRNA, miRNA, mRNA, long RNA).

In certain embodiments, is the solvent of the first and/or the second solution water or a cell culture medium.

In certain embodiments, is the final concentration of the nucleic acid in the transfection mix between 10 nM to 200 nM, particularly 20-100 nM, more particularly 40-80 nM.

In certain embodiments, is the final concentration of the peptide dendrimer in the transfection mix between 1.5 µg/ml and 30 µg/ml, particularly between 3 µg/ml and 15 µg/ml, more particularly between 6 µg/ml and 12 µg/ml.

In certain embodiments, is the N/P ratio (ratio of amine groups -NH₂ of the peptide dendrimer to phosphate groups of the nucleic acid) in the transfection mix between 3 and 100, particularly between 3 and 50, more particularly between 3 and 10.

In certain embodiments, is the transfection mix incubated after mixing the first and the second solution, particularly for 10 to 60 min, more particularly for 20 to 40 min, even more particularly for 25 to 35 min.

In certain embodiments, the transfection mix incubated at a temperature between 18 °C and 30 °C, particularly between 20 °C and 25 °C.

In certain embodiments, is the final concentration of the nucleic acid in the transfection mix between 10 nM to 200 nM, particularly between 20 nM to 100 nM, more particularly between 40 to 80 nM.

In certain embodiments, is the final concentration of the peptide dendrimer in the transfection mix between 1.5 µg/ml and 30 µg/ml, particularly between be 3-15 µg/mL, more particularly between 6-12 µg/mL.

In certain embodiments, comprises the transfection mix fetal calf serum, particularly up to 10 % (v/v) fetal calf serum.

In certain embodiments, is the cell a eukaryotic cell, particularly a mammalian cell.

In certain embodiments, is the cell contacted with the transfection mix for 4 h to 72 h.

In certain embodiments, are the cells contacted with the transfection mix at 37 °C in a humidified atmosphere comprising 5 % CO₂.

### Figures

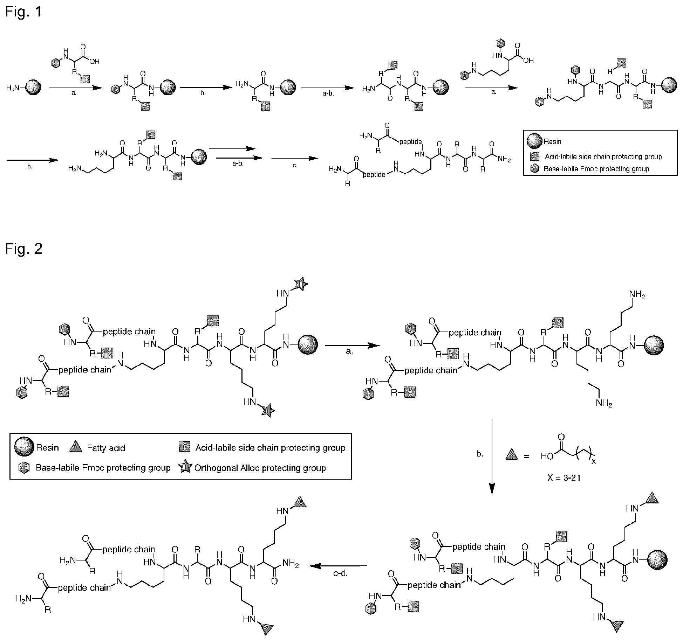

Fig. 1: shows a solid-phase peptide synthesis (SPPS) of peptide dendrimers. a. Coupling: 3eq/coupling-site Fmoc-amino acid, 3eq/coupling-site PyBOP and 5eq/coupling-site DIEA in NMP, 60 min-O.N. (manual synthesis), or 5eq/coupling-site Fmoc-amino acid, 5eq/coupling-site Oxyma, and 5eq/coupling-site DIC in NMP or DMF, 2-30 min (microwave synthesis). b. Fmoc-Deprotection: piperidine/DMF (1:4, v/v), 20 min. c. Cleavage: TFA (95%), TIS (2.5%), H₂O (2.5%) (Cys free peptide) or TFA (94%), TIS (1%), H₂O (2.5%), EDT (2.5%) (Cys containing peptide), 300 min.
Fig. 2: shows a aolid-phase peptide synthesis (SPPS) of peptide dendrimers starting with Alloc-protected lysine in the generation 0. a.Alloc-Deprotection: P(PPh₃)₄ (0.25eq/Alloc group), PhSiH₃ or (CH₃)₂NH·BH₃ (25eq/Alloc group), 2 x 60min. b. Coupling: Fatty acid (5eq/coupling-site), DIC (5eq/coupling-site), Oxyma (5eq/coupling-site) in NMP or DMF, 60 min-O.N. c. Fmoc-Deprotection: piperidine/DMF (1:4, v/v), 20 min. d. Cleavage: TFA (95%), TIS (2.5%), H₂O (2.5%) (Cys free peptide) or TFA (94%), TIS (1%), H₂O (2.5%), EDT (2.5%) (Cys containing peptide), 300 min.

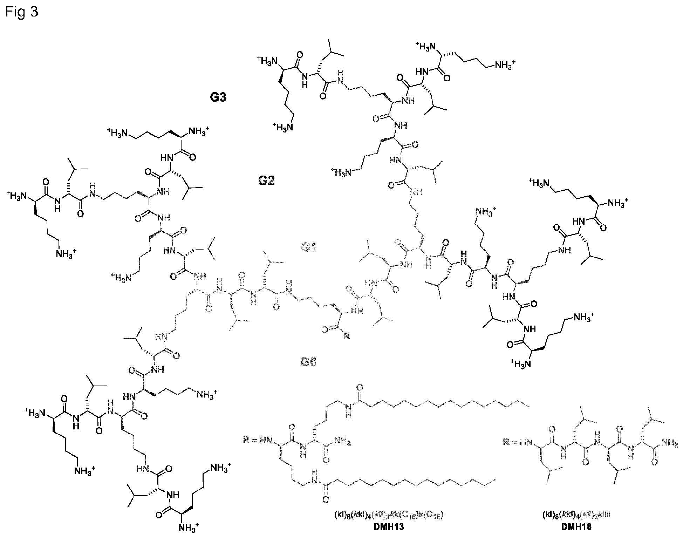

Fig. 3: Structural formulae and amino acid sequences of siRNA transfection dendrimers DMH13 and DMH18 (one letter codes for amino acids, branching lysines in italics).

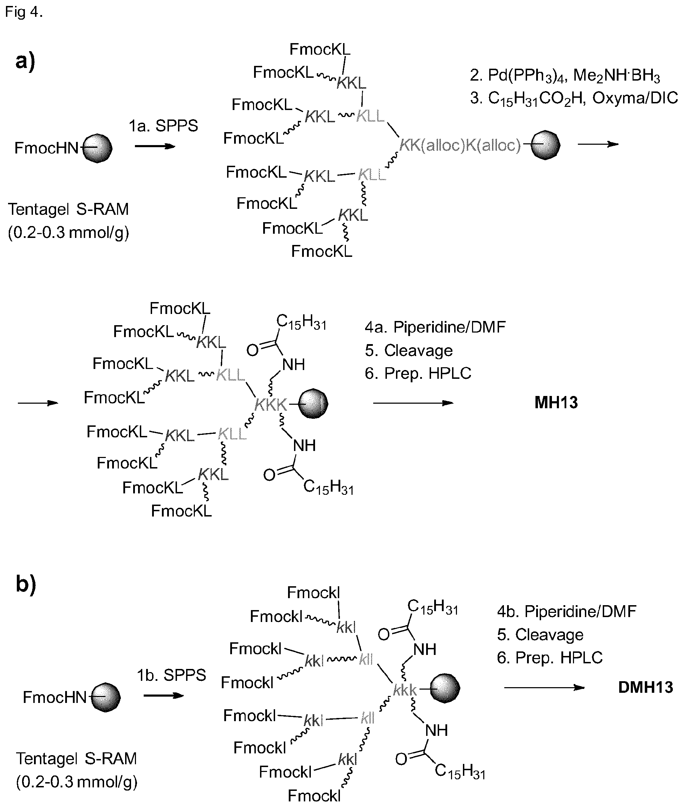

Fig. 4: Synthesis of transfection peptide dendrimers. (a) SPPS using orthogonal protection for lysine side chain acylation at the example of peptide dendrimer MH13. (b) High temperature SPPS in a microwave synthesizer using pre-acylated lysines at the dendrimer core. Conditions: 1a) i) 3eq. /coupling-site Fmoc-amino acid, 3 eq./coupling site PyBOP, 5 eq./coupling site i-Pr2NEt in NMP, 60 min-overnight; ii) Piperidine/DMF (1:4, v/v), 20 min. 1b) i) 5eq. /coupling-site Fmoc-amino acid, 5 eq./coupling site Oxyma, 5 eq./coupling site DIC in DMF, 1-5 min, 75-90 °C; ii) Piperidine/DMF (1:4, v/v), 2 min, 75-90 °C. 2) P(PPh3)4 (0.25 eq./alloc group), (CH3)2NH·BH3 (25 eq./alloc group), 2 x 60 min. 3) Palmitic acid (5 eq./coupling site), DIC (5 eq./coupling site), Oxyma (5 eq./coupling site) in NMP, 60 min-overnight. 4a) Piperidine/DMF (1:4, v/v), 20 min. 4b) Piperidine/DMF (1:4, v/v), 2 min, 75-90 °C. 5) CF3CO2H (95%), i-Pr3SiH (2.5%), H₂O (2.5%), 5 h, 25°C. 6) Reverse phase C18 preparative HPLC, gradient 0-70% CH3CN/H2O/0.1% TFA over 45 min.

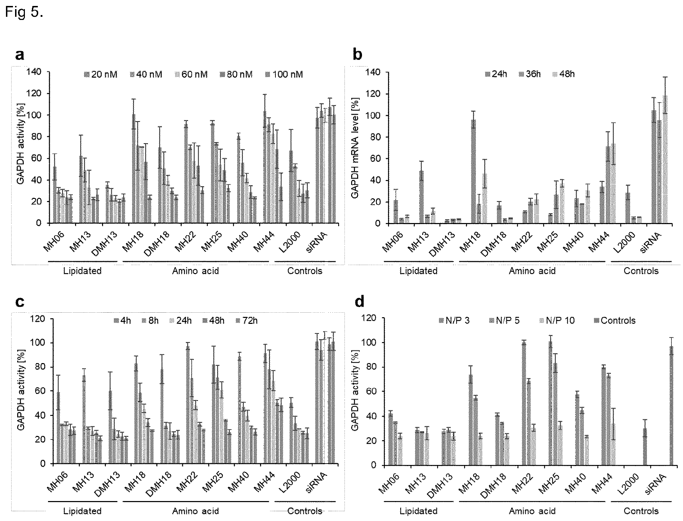

Fig. 5: Knock-down efficiency of peptide dendrimer/siRNA complexes under different conditions in HeLa cells. (a) GAPDH activity in HeLa cells after 4 h transfection by siRNA (20-100 nM) and peptide dendrimers (N/P 10, 0.42-2.1 µM, 3-15 µg/mL) or L2000 (2:1, 0.532-2.66 µg/mL) followed by 48 h incubation in DMEM supplemented with 10% FCS. (b) GAPDH mRNA level in HeLa cells by RT-PCR after 4 h transfection by siRNA (100 nM) and peptide dendrimers (N/P 10, 2.1 µM, 15 µg/mL) or L2000 (2:1, 2.66 µg/mL) completed by an incubation in DMEM supplemented with 10% FCS of up to 24, 36 or 48 h. Results were normalized to the level of untreated cells and each measurement to the level of 18S. (c) GAPDH activity in HeLa cells after 4 to 72 h transfection by siRNA (100 nM) and peptide dendrimers (N/P 10, 2.1 µM, 15 µg/mL) or L2000 (2:1, 2.66 µg/mL) in presence of 10% serum completed by an incubation in DMEM supplemented with 10% FCS of up to 72 h. (d) GADPH activity in HeLa cells after 4 h transfection by siRNA (100 nM) and peptide dendrimers (N/P 3-10, 0.63-2.1 µM, 4.5-15 µg/mL) or L2000 (2:1, 2.66 µg/mL) followed by 48 h incubation in DMEM supplemented with 10% FCS.

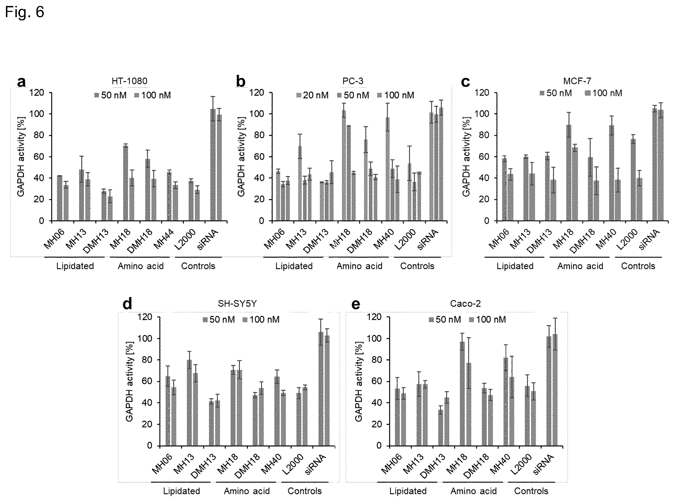

Fig. 6: Knock-down efficiency peptide dendrimers/siRNA complexes in different cell lines. GADPH activity in (a) HT-1080, (b) PC-3, (c) MCF-7, (d) SH-SY5Y and (e) Caco-2 cell lines after 4 h transfection in OptiMEM by siRNA (20-100 nM) and peptide dendrimers (N/P 10, 0.42-2.1 µM, 3-15 µg/mL) or L2000 (2:1, 0.532-2.66 µg/mL) followed by 48 h incubation in DMEM supplemented with 10% FCS. Results were normalized to parallel transfection with siNC (negative control). All experiments were carried in triplicate in three independent experiments.

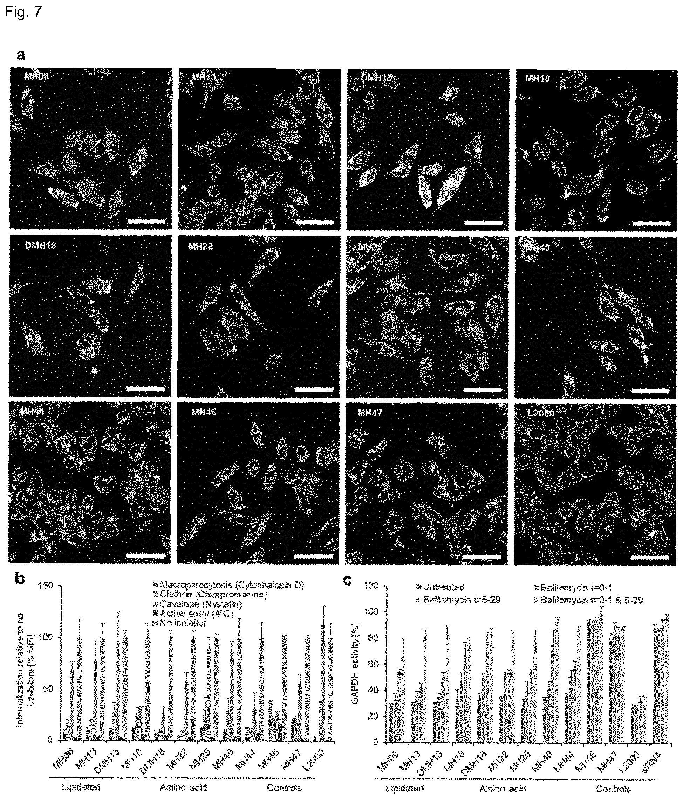

Fig. 7: Biological mechanism of dendrimer promoted siRNA transfection. (a) Live cells confocal microscopy of FAM-siRNA (green) in HeLa cells. HeLa cells were incubated with FAM-siRNA (100 nM) and peptide dendrimers (N/P 10, 2.1 µM, 15 µg/mL) or L2000 (2:1, 2.66 µg/mL) for 4 h in OptiMEM. The plasma membrane was stained with Cell mask deep red. White scale bars represent 50 µm (lense x40/1.3). (b) Internalization of dendrimer/FAM-siRNA nanoparticles in HeLa cells after 4 h transfection at 4 °C or preceded by 1 h incubation with inhibitors and measured by flow cytometry. siRNA (100 nM) was combined with peptide dendrimers (N/P 10, 2.1 µM, 15 µg/mL) or L2000 (2:1, 2.66 µg/mL). Error bars represent the standard deviation of two independent experiment and normalized to the internalization in normal conditions. (c) GAPDH activity in HeLa cells after 4 h transfection by siRNA (100 nM) and peptide dendrimers (N/P 10, 2.1 µM, 15 µg/mL) or L2000 (2:1, 2.66 µg/mL) with 1 h pretreatment (t=0-1), 24 h incubation post transfection (t=5-24) or both (t=0-1 & 5-29) with 200 nM Bafilomycin A1 followed by 24 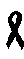 48 h incubation in DMEM supplemented with 10% FCS.

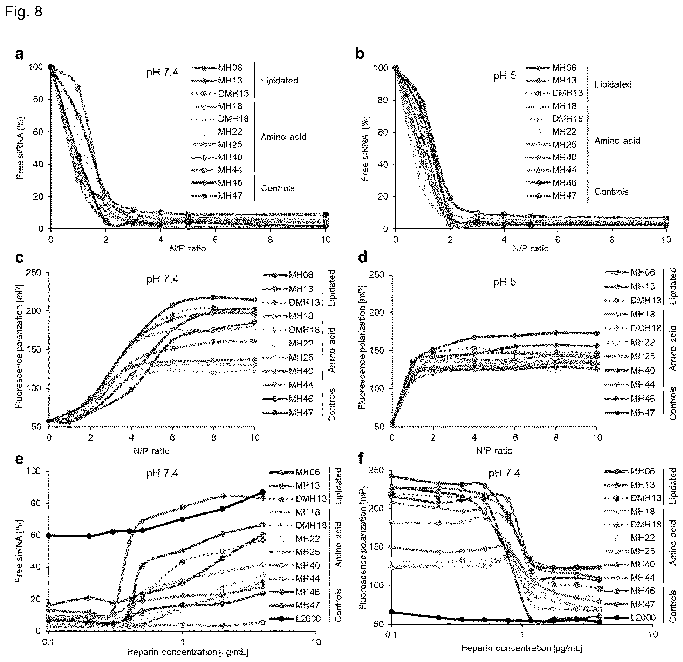

Fig. 8: Dendrimer/siRNA complex formation. (a) Free siRNA assay by intercalation of Quant-it^{™} microRNA in complexes of siRNA (20 nM) and peptide dendrimers (N/P 1-10, 42-420 nM, 0.3-3 µg/mL) in function of the N/P ratio at pH 7.4 in Quant-it^{™} microRNA buffer. (b) same as (a) at pH 5 in Quant-it^{™} microRNA buffer acidified with 10 mM acetate buffer. (c) Fluorescence polarization of FAM-siRNA (20 nM) in function of the N/P ratio of peptide dendrimers (N/P 1-10, 42-420 nM, 0.3-3 µg/mL) at pH 7.4 in DPBS. (d) Same as (c) at pH 5 in DPBS. (e) Displacement of siRNA from complexes formed of siRNA (20 nM) and peptide dendrimers (N/P 10, 420 nM, 3 µg/mL) or L2000 (2:1, 532 ng/mL) by addition of heparin (0-4 µg/mL) measured by tracking free siRNA by Quant-it^{™} microRNA assay at pH 7.4 in Quant-it^{™} microRNA buffer. Fluorescence normalized to siRNA alone set as 100%. (f) Same as (e) with heparin concentration (0-4 µg/mL) measured by FP at pH 7.4 in DPBS. All experiments were performed in triplicate.

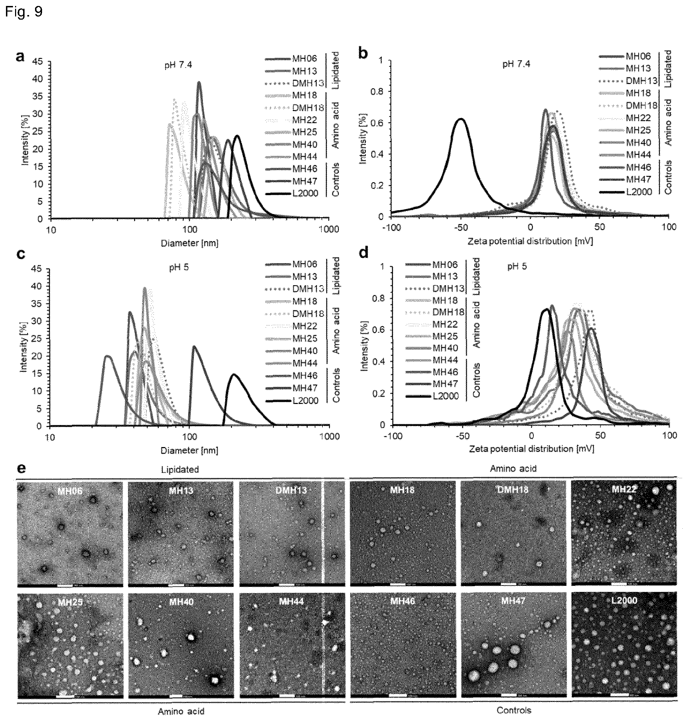

Fig. 9: Nanoparticle formation observed by DLS and TEM. (a,c) Dynamic light scattering and (b,d) zeta potential of siRNA (800 nM) and peptide dendrimers (N/P 10, 16.8 µM, 120 µg/mL) or L2000 (2:1, 21.3 µg/mL) complexes formed in PB at pH 7.4 and 5. All data shown have a polydispersity index (PDI) < 0.5. (e) Transmission electron microscopy of siRNA (800 nM) and peptide dendrimers (N/P 10, 16.8 µM, 120 µg/mL) or L2000 (2:1, 21.3 µg/mL) complexes formed in Milli-Q water deposited on glow discharged 400 mesh copper grids, dried and stained by uranyl acetate. White scale bars represent 100 nm.

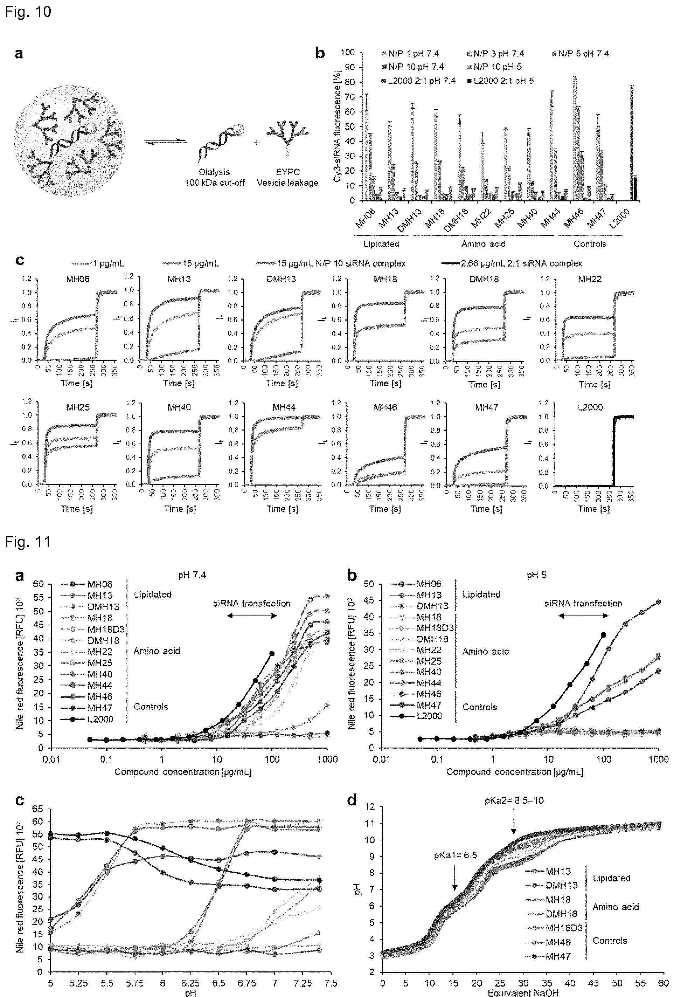

Fig. 10: Evidence for nanoparticle formation and aggregation with transfection peptide dendrimers. (a) Equilibrium between a dendrimer/Cy3-siRNA nanoparticle and its constituents. (b) Fluorescence of Cy3-siRNA (100 nM) after dialysis through a 100 kDa cutoff membrane in the presence of peptide dendrimers (N/P 1-10, 0.21-2.1 µM, 1.5 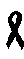 15 µg/mL) or L2000 (2:1, 2.66 µg/mL) in OptiMEM pH 7.4 or pH 5.0. Fluorescence intensity relative to untreated, filtered Cy3-siRNA is shown. (c) Fluorescein leakage assay from phosphatidyl choline lipid vesicles suspended in buffer (31 µM EYPC, 625 µM CF, 10 mM TRIS, 107 mM NaCl, pH 7.4). After 30 s peptide dendrimer (1-15 µg/mL), L2000 (1-15 µg/mL) or complexes formed with siRNA (100 nM) and peptide dendrimers (N/P 10, 2.1 µM, 15 µg/mL) or L2000 (2:1, 2.66 µg/mL) were added in the lipid vesicle solution. After 270 s Triton X-100 (0.07%) was added for full release of fluorescein. It = ratio of fluorescence intensity.
Fig.11:CMC and pKa determination with peptide dendrimers. (a) Critical aggregation concentration determination of peptide dendrimers and L2000 in PB pH 7.4, performed by serially diluting compounds starting from 1 mg/mL or 0.1 mg/mL respectively, added on dried Nile red (final concentration of 0.2 µM). (b) Same as (a) at PB pH 5 (c) Peptide dendrimer or L2000 (0.1 mg/mL) in PB at pH 5-7.4 added on dried Nile red (final concentration of 0.2 µM). Fluorescence measured at λex= 540 nm and λem= 615 nm. RFU = Relative fluorescence unit. (d) pH titration of 0.7-1 µmol (5-7 mg in 7-10 ml Milli-Q water, 100 µM) peptide dendrimers by 2 µL step of 0.1 M NaOH.

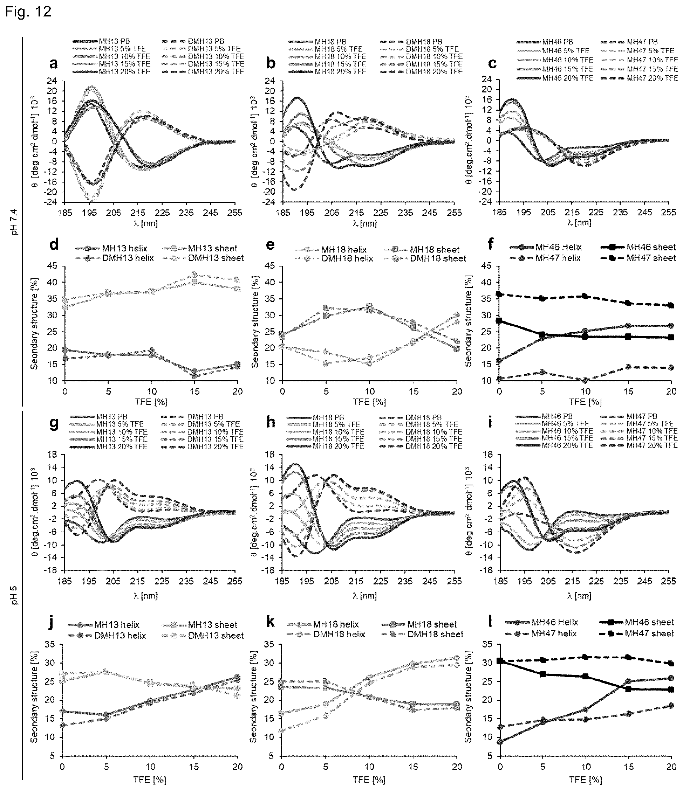

Fig.12: Conformation of transfection peptide dendrimers in solution at pH 7.4 and pH 5.0. Circular dichroism spectra of MH13, DMH13, MH18, DMH18, MH46 and MH47 (200 µg/mL) in 8 mM phosphate buffer (PB) at (a,b,c) pH 7.4 or (g,h,i) pH 5 with various concentration of trifluoroethanol. Percentage of α-helix and β-sheet as a function of the concentration of trifluoroethanol at (d,e,f) pH 7.4 and (j,k,l) pH 5 processed by Dichroweb using the CONTIN analysis program and reference set 3.

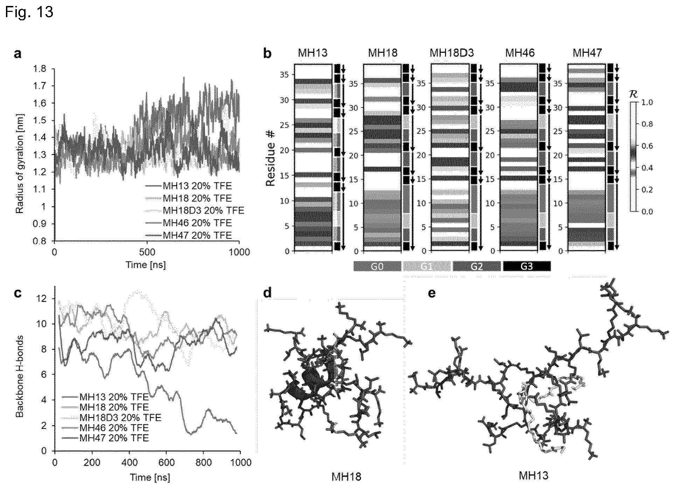

Fig. 13: Molecular dynamics studies at pH 5 in the presence of 20% TFE. (a) Radius of gyration over the course of the simulation. (b) Ramachandran number 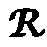 analysis of the internal secondary structures. Residues in α-helical conformation ( 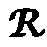 ≈ 0.34) are indicated in red, β-sheet ( 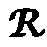 ≈ 0.52) in blue and loops ( 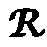 ≈ 0.62) in cyan. The position of each residue according to its generation is noted using the color code of the previous Figures. The arrows represent continuous β-peptide portions of the dendrimer. (c) Total backbone hydrogen bonds over the course of the simulation. Representative equilibrated structures obtained by clustering of the last 100 ns of the MD trajectory of peptide dendrimers (d) MH18 and (e) MH13 color-coded by residue type (blue = lysine, brown = leucine, gray = branching lysine, yellow = lipid chain).

### General Procedures

### Solid phase synthesis of peptide dendrimers

The Fmoc-SPPS is a widely-used method for the synthesis of all kind of peptides with building blocks based on either natural, unnatural, L- or D-amino acids (these latter denoted with capital and small letters respectively. In peptide dendrimers synthesis, the difference is on the use branching points consisting of diamino acids such as lysine, to have amide bond connection throughout the dendrimer structure (Fig. 1). The amino acids are attached with standard peptide coupling conditions and the Fmoc-protecting group removed under mild basic conditions, orthogonal to the acid labile side chain protecting groups. The chemical synthesis of the peptides starts at the C-terminus, and the peptide chain is growing towards the N-terminus while the biosynthesis occurs in the opposite direction.

After completion of these sequences the peptide dendrimers are cleaved from the resin with a cleavage cocktail based on TFA that also removes all acid-labile protecting groups from the side chains, precipitated in ether, purified with preparative RP-HPLC and characterized with MS, analytical LCMS and amino acid analysis.

### Manual solid phase synthesis of peptide dendrimers

Peptide dendrimers were synthesized by placing 300 mg Tentagel S RAM resin (0.22-0.25 mmol/g) in a 10 mL polypropylene syringe equipped as described previously. Stirring of the reaction mixture at any given step described below was performed by attaching the closed syringe to a rotating axis. The resin was swollen in DCM for 60 min. Then, the following conditions were used:
*Removal of the Fmoc protecting group:* At each step the Fmoc protecting group was removed with 8 mL of piperidine/DMF (1:4, v/v) for 2 x 10 min. After filtration the resin was washed with NMP (3 × 6 mL), MeOH (3 × 6 mL) and DCM (3 × 6 mL).
*Coupling of the Fmoc-protected amino acids:* 3 eq. of Fmoc-protected amino acid, 3 eq. of PyBOP (benzotriazol-1-yl-oxytripyrrolidinophosphonium hexafluorophosphate) and 5 eq. of DIPEA (N,N-Diisopropylethylamine) per reaction site in 8 mL of NMP/DCM (80:20, v/v) were added to the resin and the reaction was stirred for 60 min. Reaction were carried out according to the dendrimer generations with 1 h for the 0^{th} generation, 2 h for the 1^{st} generation, 3 h for the 2^{nd} generation and 4 h for the 3^{rd} generation. The resin was then washed with NMP (3 × 6 mL), MeOH (3 × 6 mL) and DCM (3 × 6 mL).

### Solid phase synthesis of peptides dendrimers by Biotage Initiator+ Alstra

Peptide dendrimers were also synthesized by Biotage Initiator Alstra using 300 mg of Tentagel S RAM resin (0.22-0.25 mmol/g). Stirring of the reaction mixture at any given step described below was performed by vortexing the vial. The resin was swollen in DMF for 60 min at R.T. Then, the following conditions were used:
*Removal of the Fmoc protecting group:* At each step the Fmoc protecting group was removed with 6 mL of piperidine/DMF (1:4, v/v) for 5 min at 75 °C. After filtration, the resin was washed for 8 min with DMF.
*Coupling of the Fmoc-protected amino acids:* - 5 eq. of Fmoc-protected amino acid, 5 eq. of Oxyma and 5 eq. of DIC all at a concentration of 0.5 M were used as coupling reagents in 5 mL of DMF. The reaction was stirred for 8 minutes at 75 °C. The resin was then washed with DMF for 8 min. The couplings were repeated according to the generations and performed once for the zero generation, twice for the first generation, four times for the second generation and seven times for the third generation.

### Solid phase synthesis of peptide dendrimers by CEM Liberty Blue

Peptide dendrimers were synthesized by CEM Liberty Blue (scale 0.10 mmol) using 300 mg of Tentagel S RAM resin (0.22-0.25 mmol/g). Stirring of the reaction mixture at any given step described below was performed by bubbling of N₂ in the vial. The resin was swollen in DMF/DCM 50:50 for 15 min at R.T. Then, the following conditions were used:
*Removal of the Fmoc protecting group:* At each step the Fmoc protecting group was removed with 5 mL of piperidine/DMF (1:4, v/v) for 2 min at 75 °C. After filtration, the resin was washed 5 times with 5 mL DMF.
*Coupling of the Fmoc-protected amino acids:* 5 eq. of Fmoc-protected amino acid, 5 eq. of Oxyma and 5 eq. of DIC all at a concentration of 0.2 M, were used as coupling reagents in 4 mL of DMF. The reaction was stirred for 5 minutes at 75 °C. The resin was then washed with 4 mL DMF 4 times. The couplings were repeated according to the generations and performed once for the zero generation, twice for the first generation, four times for the second generation and seven times for the third generation.
*Deprotection of Lys(Alloc) and coupling:* The resin was dried *in vacuo* and bubbled twice in dry DCM (8 ml) for 5 minutes with nitrogen. Solutions of Pd(PPh₃)₄ (0.1 eq., 10 mg) in dry DCM (3 mL) and (CH₃)₂NH·BH₃ (25 eq., 100 mg) in dry DCM (3 ml) were added to the resin and bubbled with nitrogen for 1h. The resin was washed with dry DCM (3 × 8 mL) and reaction repeated once for 2 h. The resin was washed with sodium diethyldithiocarbamate (0.02 M in DMF, 10 ml) for 20 min and NMP, MeOH and DCM (2 × 10 ml each). Then, the carboxylic acids were coupled according to the manual procedure.
*Last Fmoc deprotection:* After the last amino acid was coupled by the three different methods, and in some case the carboxylic acid coupled, Fmoc deprotection was performed with 8 mL of piperidine/DMF (1:4, v/v) for 20 min. After filtration, the resin was washed with NMP (3 × 6 mL), MeOH (3 × 6 mL) and DCM (3 × 6 mL).
*Cleavage and purification:* The cleavage was carried out by treating the resins with 7 mL of a TFA/DODT/TIS/H₂O (94:2.5:2.5:1, v/v/v/v) solution for 5 h. The peptide solutions were precipitated with 40 mL of TBME, centrifuged for 10 min at 3500 rpm (twice), evaporated and dried under high vacuum for 60 min. The crude was then dissolved in a H₂O/CH₃CN mixture with 0.1% TFA, some drops of MeOH added when needed and purified by preparative RP-HPLC. The fractions of the crudes were then lyophilized. Yields are given as SPPS total yields. In all cases, yields are calculated for the corresponding TFA salts.

The manual SPPS procedure is illustrated for the synthesis of dendrimer MH13 (Fig. 4a) and the high temperature SPPS in a microwave synthesizer with pre-acylated lysine building blocks for the dendrimer core, which resulted in higher preparative yields, is illustrated for D-enantiomeric dendrimer DMH13 (Fig 4b).

All products were purified by preparative reverse-phase HPLC and the purest fractions were isolated as trifluoroacetate salts after lyophilization and conditioned as 1 to 5 mg/mL stock solutions in water for further use.

### Transfection and mechanistic procedures

### siRNA

*Cell Culture, Transfection Reagents and siRNA* - HeLa, HEK-293, CHO, PC-3, HT-1080, SH-SY5Y, Caco-2 and MCF-7 cells (ATCC, Manassas, USA) were maintained in DMEM (Thermo Fisher Scientific, Reinach, CH) supplemented with 10% fetal calf serum (FCS, Thermo fisher Scientific) at 37 °C in a humidified atmosphere in 5% carbon dioxide. The sequence of the siRNA targeting GAPDH (AM 4631) for the sense strand is GGUCAUCCAUGACAACUUUdTdT and for the antisense strand AAAGUUGUCAUGGAUGACCdTdT. In the case of FAM-siRNA (AM 4650) or Cy3-siRNA (AM4649) targeting GAPDH, the sequences are identical with a 5-carboxyfluorescein or a Cyanine 3 attached to the 5' end of the sense strand. The sequence of the validated negative control siRNA (siNC, AM 4635) for the sense strand is AGUACUGCUUACGAUACGGdTdT and for the antisense strand CCGUAUCGUAAGCAGUACUdTdT. The siRNAs are consisting of phosphodiester bonds and containing two nucleotide overhangs (dTdT) at the 3'-end (Thermo Fisher Scientific). Lipofectamine^{®} 2000 (L2000) was obtained from Thermo Fisher Scientific and used as positive control with the transfection protocol in accordance with the manufacturer's instructions.

*siRNA Transfection and Protein Quantitation:* HeLa, CHO, PC3, SH-SY5Y, Caco-2 and MCF-7 cells were seeded in TPP 96-well plates (Faust Laborbedarf AG, Schaffhausen) at 5×10³ cells per well the day before transfection. The 96-well plates were coated with poly-L-Lysine (Sigma aldrich, Buchs, CH) for 1 h and dried under a flow of air in the fumehood at RT before plating HEK-293 and HT-1080 at 5x10³ cells per well the day before transfection. Cells were always used in their exponential phase for transfection and with a number of passages between 3 to 20. The siRNA transfection complexes were formed in OptiMEM by mixing siRNA (1-20 pmol, 0.02-0.4 µL from a 50 µM Milli-Q water solution in 6.25 µL OptiMEM) with peptide dendrimers (N/P ratio of 1-100, 21-420 pmol, 0.1-3.5 µl from a 1 mg/mL Milli-Q water solution in 6.25 µL OptiMEM) or L2000 (w/w ratio of 0.5:1-2:1, 0.0665-0.266 µg, 0.0665-0.266 µL from the 1 mg/mL commercial solution in 6.25 µL OptiMEM) at room temperature for 30 minutes (12.5 µL, concentration of 80-1600 nM siRNA and 1.68-33.6 µM i.e. 12-240 µg/mL peptide dendrimers or 5.32-21.28 µg/mL L2000). The complexes were then diluted in OptiMEM or in OptiMEM plus 10% FCS to a final volume of 100 µL per well (final concentration of 10-200 nM siRNA and 0.210-4.2 µM i.e. 1.5-30 µg/mL peptide dendrimers or 0.665-2.66 µg/mL L2000). Following removal of the complete medium from the cells, 100 µL of the transfection were added to each well. The plate was then incubated for 4-72 h at 37 °C in a humidified atmosphere in 5% carbon dioxide. The transfection complexes were replaced by full growth medium following the transfection. The siGAPDH gene knockdown assays were conducted 48 h or 72 h hours following transfection by measuring the level of GAPDH protein according to the manufacturer's instructions of the KDalert GAPDH assay kit (Thermo Fisher Scientific, Reinach, CH). Briefly, medium was removed, cells were lysed with 200 µL cold lysis buffer, 2-10 µL of lysis buffer from each well was taken to a new 96 well plate containing 90 µL Master Mix and fluorescence measured at λₑₓ= 560 nm and λₑₘ= 590 nm on a Tecan Infinite M1000 Pro plate reader.

siRNA Transfection in Presence of Bafilomycin: HeLa cells were used untreated or treated with Bafilomycin A1 (200 nM, Alfa Aesar, Karlsruhe, DE) in DMEM supplemented with 10% FCS for 1 h before transfection in 96-well TPP plates described above. After transfection, complexes were removed and replaced with DMEM supplemented with 10% FCS and incubated for 48 h or DMEM supplemented with 10% FCS containing 200 nM Bafilomycin A1, incubated for 24 h then replaced by DMEM supplemented with 10% FCS for 24 h supplementary incubation. Then, HeLa cells were treated for 1 h before transfection, 24 h following transfection or both with Bafilomycin A1. GAPDH protein level was assessed as described above after a total of 48 h.

Cellular Uptake by Flow Cytometry: HeLa, CHO and HEK-293 cells were seeded in 24-well TPP plates (Faust Laborbedarf AG, Schaffhausen) at 1×10⁵ cells per well 24 h prior to transfection. Complexes were formed with labelled FAM-siRNA (Thermo Fisher Scientific, Reinach, CH) (12-40 pmol, 0.24-0.8 µL from a 50 µM Milli-Q water solution in 25 µL OptiMEM) and peptide dendrimers (N/P ratio of 10, 252-840 pmol, 2.1-7 µL from a 1 mg/mL Milli-Q water solution in 25 µL OptiMEM) or L2000 (w/w ratio of 2:1, 0.319-1.064 µg, 0.319-1.064 µL from the 1 mg/mL commercial solution in 25 µL OptiMEM) and incubated at room temperature for 30 minutes (50 µL, concentration of 240-800 nM siRNA and 5.04-16.8 µM i.e. 36-120 µg/mL peptide dendrimers or 6.384-21.28 µg/mL L2000). The complexes were then diluted in OptiMEM to a final volume of 400 µL per well (final concentration of 30-100 nM FAM-siRNA and 0.63-2.1 µM i.e. 4.5-15 µg/mL peptide dendrimers or 0.798-2.66 µg/mL L2000). Following removal of the medium, the complexes were added to each well and incubated for 4 h at 37 °C in a humidified atmosphere in 5% carbon dioxide. Then the medium was removed, cells were washed with heparin in OptiMEM (2 mg/ml, 0.5 ml, 3 times), washed twice with PBS and the cell membrane was labeled with CellMask Deep Red plasma membrane stain (Thermo Fisher Scientific, Reinach, CH) at 0.5X in full DMEM growth medium (0.25 µL in 0.5 mL / well), 10 minutes at 37 °C. The cells were washed with PBS (1.0 mL / well, 3 times) and detached from the wells with 250.0 µL of 0.25% Trypsin / EDTA solution at 37 °C. Cells were harvested with 750.0 µL full DMEM growth medium, collected in 1.5 mL eppendorf tubes and centrifuged 5 minutes at 200 RCF. The trypsin solution was discarded, the pellet shaken and the cells were resuspended in 50.0 µL of PBS + 1% FCS. The fluorescence intensity of each cell sample was analyzed by a flow cytometer-microscope coupled ImageStream X Mark II (Merck Millipore) and processed with the IDEAS^{®} software.

Cellular Uptake in Presence of Inhibitors and at Lower Temperature: Cells were treated with Cytochalasin D (25 µg/mL), Nystatin (25 µg/mL) or Chlorpromazine (15 µg/mL) in DMEM supplemented with 10% FCS for 1 h and washed with PBS before transfection in 24-well TPP plates as described above. In the temperature dependent experiment, cells were transfected in the same condition as above for 4 h but at 4 °C. The cells were washed and processed as described above and the fluorescence intensity of each cell sample was analyzed by a flow cytometer-microscope coupled ImageStream X Mark II (Merck Millipore) and processed with the IDEAS^{®} software.

Quantitative RT-PCR: Cells were transfected in TPP 96-well plates as previously described. Following transfections, cells were washed with 50 µl cold PBS and the Cells-to-Ct kit (Thermo Fisher Scientific, Reinach, CH) was used. Briefly, cells were lysed with 49.5 µL lysis buffer + 0.5 µL DNase for 5 minutes, 5 µl of stop solution added and incubated for 2 minutes. Then, 2 µl of cell lysate was mixed with 18 µl of the Master mix containing 1 µL TaqMan GAPDH endogenous control (FAM/MGB, non-primer limited, 433764F, Thermo Fisher Scientific), 1 µL TaqMan 18S Endogenous control (VIC/MGB, primer limited, 4319413E, Thermo Fisher Scientific), 5 µL TaqMan 1-Step qRT-PCR Mix and 11 µL water. RT-PCR was performed in duplex, with GAPDH and 18S primers in the green and yellow channels respectively in a Corbett Rotorgene 6000 (Qiagen). Cycles: RT: 50 °C/5 min, RT inactivation: 95 °C/20 sec, Amplification: 95 °C/15sec and 60 °C/1 min for 40 cycles. mRNA levels were calculated by normalizing the Ct values of GAPDH to the Ct value of 18S and quantified by the 2^{-ΔΔCt} method.

Cell Viability by AlamarBlue^{®} Assay: Cells were transfected in TPP 96-well plates as previously described. Following transfections, the medium was removed and replaced with 10% AlamarBlue^{®} (Thermo Fisher Scientific, Reinach, CH) in DMEM supplemented with 10% FCS. Cells were incubated for 4-24 h at 37 °C in a humidified atmosphere in 5% carbon dioxide. Then, plates were measured on a Tecan Infinite M1000 Pro plate reader at λₑₓ= 560 nm and λₑₘ= 590 nm and value normalized to the one of untreated cells.

Confocal Microscopy: Nunc Lab-Tek II 8-well chambered coverglass plates (Faust Laborbedarf AG, Schaffhausen) were treated with poly-L-Lysine (Sigma Aldrich, Buchs, CH) for 1 h, dried at RT and the day prior transfection the cells were plated at 3×10⁴ cells per well. Complexes were formed with labelled FAM-siRNA (12-40 pmol, 0.24-0.8 µL from a 50 µM Milli-Q water solution in 25 µL OptiMEM) and peptide dendrimers (N/P ratio of 10, 252-840 pmol, 2.1-7 µL from a 1 mg/mL Milli-Q water solution in 25 µL OptiMEM) or L2000 (w/w ratio of 2:1, 0.319-1.064 µg, 0.319-1.064 µL from the 1 mg/mL commercial solution in 25 µL OptiMEM) and incubated at room temperature for 30 minutes (50 µL, concentration of 240-800 nM siRNA and 5.04-16.8 µM i.e. 36-15 µg/mL peptide dendrimers or 6.384-21.28 µg/mL L2000). The complexes were then diluted in OptiMEM to a final volume of 400 µL per well (final concentration of 30-100 nM FAM-siRNA and 0.63-2.1 µM i.e. 4.5-15 µg/mL peptide dendrimers or 0.798-2.66 µg/mL L2000). The complexes were added to each well and incubated 4 h at 37 °C in a humidified atmosphere in 5% carbon dioxide following the removal of the full growth medium. Then, the medium was removed, cells were washed with heparin in

OptiMEM (2 mg/ml, 0.5 ml, 3 times), washed twice with PBS and the cell membrane was labeled with CellMask Deep Red plasma membrane stain (Thermo Fisher Scientific, Reinach, CH) at 0.5X in full DMEM growth medium (0.25 µL in 0.5 mL / well), 10 minutes at 37 °C. The cells were washed with PBS (1.0 mL / well, 3 times), FluoroBrite DMEM (Thermo Fisher Scientific, Reinach, CH) was added and images were taken on a Zeiss LSM 880 confocal microscope with lense x40/1.3.

Free siRNA assay by Quant-iT^{™} microRNA: The complexes were formed in OptiMEM by mixing siRNA (10 pmol, 0.2 µL from a 50 µM Milli-Q water solution in 6.25 µL OptiMEM pH 5 or pH 7.4) with peptide dendrimers (N/P ratio of 1-10, 42-420 pmol, 0.15-1.5 µL from a 1 mg/mL Milli-Q water solution in 6.25 µL OptiMEM pH 5 or pH 7.4) or L2000 (w/w ratio of 0.2:1-8:1, 0.0266-1.064 µL from the 1 mg/mL commercial solution in 6.25 µL OptiMEM pH 7.4) for 30 min at room temperature (12.5 µL, concentration of 800 nM siRNA and 1.68-16.8 µM i.e. 12-120 µg/mL peptide dendrimers or 0.133-5.32 µg/mL L2000). Then, the Quant-iT^{™} microRNA Assay Kit (Thermo Fisher Scientific, Reinach, CH) was used following the manufacturer's protocol. Briefly, 1 µl of reagent was diluted in 200 µl of buffer (pH 5 by acidification by 10 mM acetate buffer or standard pH 7.4) and 195 µL added to the well of a TPP 96-well plate. Then, 5 µl of the complexes were added to the wells (200 µL, final concentration of 20 nM siRNA and 42-420 nM i.e. 0.3-3 µg/mL peptide dendrimers or 0.0532-2.128 µg/mL L2000) and fluorescence measured at λₑₓ= 500 nm and λₑₘ= 525 nm after 10 min on a Tecan Infinite M1000 Pro plate reader. The Quant-iT^{™} microRNA signal from the complexes were normalized against a « siRNA only » control to yield the percentage of the signal detected.

Competition assay by Quant-iT^{™} microRNA: The complexes were formed as in the free siRNA assay and 5 µL of the complexes were diluted in 190 µL of assay buffer as described in the part above. Then, 5 µL of heparin at different concentrations were added to the wells of a TPP 96-well plate and incubated for 30 min at room temperature (200 µL, final concentration of 0-4 µg/mL heparin, 20 nM siRNA and 420 nM i.e. 3 µg/mL peptide dendrimers or 532 ng/mL L2000). Fluorescence measured at λₑₓ= 500 nm and λₑₘ= 525 nm was performed on a Tecan Infinite M1000 Pro plate reader. The Quant-iT^{™} microRNA signal from the complexes were normalized against a « siRNA only » control to yield the percentage of the signal detected.

Free FAM-siRNA Assay by Fluorescence Polarization: The complexes were formed in DPBS (Thermofisher scientific, Reinach, CH) by mixing FAM-siRNA (2 pmol, 0.04 µL from a 50 µM Milli-Q water solution in 6.25 µL DPBS at pH 5 or pH 7.4) with the peptide dendrimers (N/P ratio of 1-10, 4.2-42 pmol, 0.03-0.3 µL from a 1 mg/mL Milli-Q water solution in 6.25 µL DPBS at pH 5 or pH 7.4) or L2000 (w/w ratio of 0.2:1-16:1, 0.00532-0.4256 µg, 0.00532-0.4256 µL from the 1 mg/mL commercial solution in 6.25 µL DPBS at pH 5 or pH 7.4) for 30 min at room temperature (12.5 µL, concentration of 160 nM FAM-siRNA and 0.336-3.36 µM i.e. 2.4-24 µg/mL peptide dendrimers or 0.4256 -34.048 µg/mL L2000). Complexes were then diluted in DPBS at pH 5 or pH 7.4 to 100 µL per well (final concentration of 20 nM FAM-siRNA and 42-420 nM i.e. 0.3-3 µg/mL peptide dendrimers or 0.0532-4.256 µg/mL L2000) and added to a Cellstar black, µClear, Greiner Bio one 96-well plate (Huberlab, Aesh, CH) and fluorescence anisotropy measured at λₑₓ= 470 nm and λₑₘ= 520 nm on a Tecan Infinite M1000 Pro plate reader. For each measurement, G-factor was calibrated with a solution of fluorescein at 1 nM in 10 mM NaOH set at 20 mP and for testing at pH 5, the polarization of FAM-siRNA alone was set to 55 mP to be comparable with pH 7.4.

Competition Assay by Fluorescence Polarization: The complexes were formed as in the free FAM-siRNA assay above in DPBS and 50 µL added to 50 µL of a serial dilution of 0 to 20 µg/mL heparin in DPBS in a Cellstar black, µClear, Greiner bio one 96-well plate (100 µL, final concentration of 0-4 µg/mL heparin, 20 nM FAM-siRNA and 420 nM i.e. 3 µg/mL peptide dendrimers or 532 ng/mL L2000). Plate was incubated for 2 h at RT and fluorescence anisotropy measured at λₑₓ= 470 nm and λₑₘ= 520 nm on a Tecan Infinite M1000 Pro plate reader. For each measurement, G-factor was calibrated with a solution of fluorescein at 1 nM in 10 mM NaOH set at 20 mP.

Dynamic Light Scattering (DLS): Complexes were formed in phosphate buffer (PB) at pH 5 or pH 7.4 with siRNA (80 pmol, 1.6 µL from a 50 µM Milli-Q water solution in 50 µL PB at pH 5 or pH 7.4) and peptide dendrimers (N/P ratio of 10, 1680 pmol, 10-14 µL from a 1 mg/mL Milli-Q water solution in 50 µL PB at pH 5 or pH 7.4) or L2000 (w/w ratio of 2:1, 2.128 µg, 2.128 µL from the 1 mg/mL commercial solution in 50 µL PB at pH 5 or pH 7.4) and incubated at room temperature for 30 minutes (100 µL, final concentration of 800 nM siRNA and 16.8 µM i.e. 120 µg/mL peptide dendrimers or 21.28 µg/mL L2000). Then, 50 µL was transferred to a low-volume Univette (Sigma aldrich, Buchs, CH). The dynamic light scattering and Zeta potential were then measured on an Anton Paar Litesizer 500 (Buchs, CH) and the data processed by the software provided by the manufacturer (Kalliope) using the "number of particles" parameter.

Transmission Electron Microscopy (TEM): The complexes were formed as previously described in Milli-Q water with siRNA (10 pmol, 0.2 µL from a 50 µM Milli-Q water solution in 6.25 µL Milli-Q water) and peptide dendrimers (N/P ratio of 10, 210 pmol, 1.25-1.75 µL from a 1 mg/mL Milli-Q water solution in 6.25 µL Milli-Q water) or L2000 (w/w ratio of 2:1, 21.28 µg/mL, 0.266 µL from the 1 mg/mL commercial solution in 6.25 µL Milli-Q water) for 30 min at room temperature (12.5 µL, final concentration of 800 nM siRNA and 16.8 µM i.e. 120 µg/mL peptide dendrimers or 21.28 µg/mL L2000). The carbon-coated copper TEM grid (400 mesh, Gloor Instruments AG, Kloten) were glow discharge on a CTA 010 Balzers Union. Then, 5 µL of the complex were dropped on the grid, incubated for 1 min at RT then dried with a filter paper. The grid was washed with water and dried 3 times with filter paper. The grid was then stained with uranyl acetate (2% in 50% alcoholic solution, 6 µl, Gloor Instruments AG, Kloten) for 10 secondes twice. Imaging was performed after stocking/air-dried for around 2 h on a FEI Tecnai spirit transmission electron microscope equipped with two digital cameras (Olympus-SIS Veleta CCD Camera, FEI Eagle CCD Camera).

Dialysis: Complexes were formed with labelled Cy3-siRNA in OptiMEM (80 pmol, 1.6 µL from a 50 µM Milli-Q water solution in 50 µL OptiMEM pH 5 or pH 7.4) and peptide dendrimers (N/P ratio of 1-10, 168-1680 pmol, 1-14 µL from a 1 mg/mL Milli-Q water solution in 50 µL OptiMEM pH 5 or pH 7.4) or L2000 (w/w ratio of 2:1, 0.218-2.128 µg, 0.218-2.128 µL from the 1 mg/mL commercial solution in 50 µL OptiMEM pH 5 or pH 7.4) and incubated at room temperature for 30 minutes (100 µL, concentration of 80-800 nM Cy3-siRNA and 1.68-16.8 µM i.e. 12-120 µg/mL peptide dendrimers or 2.66-21.28 µg/mL L2000). The complexes were then diluted in OptiMEM pH 5 or pH 7.4 to a final volume of 800 µL (final concentration of 100 nM Cy3-siRNA and 0.210-2.1 µM i.e. 1.5-15 µg/mL peptide dendrimers or 0.266-2.66 µg/mL L2000). 500 µL of the complexes were added to Amicon Ultra 0.5 mL dialysis Eppendorfs with a 100 KDa cut-off (Sigma aldrich, Buchs, CH) and centrifugated at 14000 g for 10 minutes. For the second experiment, following centrifugation, lower compartment was replaced, 500 µL of OptiMEM at pH 5 or pH 7.4 was added to the Amicon Ultra 0.5 mL dialysis Eppendorfs top compartment and centrifuged one more time at 14000 g for 10 minutes. Then, 100 µL of the dialysed solutions from before dialysis, after dialysis and supplementary wash were transferred to a TPP 96-well plate (Faust Laborbedarf AG, Schaffhausen) and fluorescence measured at λₑₓ= 547 nm and λₑₘ= 563 nm on a Tecan Infinite M1000 Pro plate reader.

Vesicle Leakage: Egg yolk phosphatidylcholine (EYPC, Avanti Polar Lipids, Alablaster, USA) thin lipid layer was prepared by evaporating a solution of 100 mg in 4 mL MeOH/CHCl₃ (1:1) on a rotary evaporator at room temperature and then *in vacuo* overnight. The resulting film was hydrated with 4 mL buffer A (50 mM 5(6)-carboxyfluorescein (CF, Sigma Aldrich, Buchs, CH), 10 mM TRIS, 10 mM NaCl, pH 7.4) for 30 min, subjected to freeze-thaw cycles (7 times) and extrusion (15 times) through a polycarbonate membrane (pore size 100 nm). Extra vesicular components were removed by gel filtration (Sephadex G-50) with buffer B (10 mM TRIS, 107 mM NaCl, pH 7.4). Final concentrations: ~ 2.5 mM EYPC; inside: 50 mM CF, 10 mM TRIS, 10 mM NaCl, pH 7.4 buffer; outside: 10 mM TRIS, 107 mM NaCl, pH 7.4 buffer.

EYPC stock solutions (10 µL) were diluted to 700-792 µL with the buffer B (10 mM TRIS, 107 mM NaCl, pH 7.4) and placed in a fluorescence cuvette, thermostated (25 °C) and gently stirred (final concentration of EYPC 31 µM, 625 µM CF, 10 mM TRIS and 107 mM NaCl). Complexes were formed with siRNA in buffer B (80 pmol, 1.6 µL from a 50 µM Milli-Q water solution in 50 µL buffer) and peptide dendrimers (N/P ratio of 10, 1680 pmol, 12 µL from a 1 mg/mL Milli-Q water solution in 50 µL buffer B) or L2000 (w/w ratio of 2:1, 2.128 µg, 2.128 µL from the 1 mg/mL commercial solution in 50 µL buffer B) and incubated at room temperature for 30 minutes (100 µL, concentration of 800 nM siRNA and 16.8 µM i.e. 120 µg/mL peptide dendrimers or 21.28 µg/mL L2000). CF efflux was monitored on an Agilent Cary Eclipse fluorescence spectrophotometer at λₑₓ= 492 nm and λₑₘ= 517 nm as a function of time for 360 s. At t= 30 s peptide dendrimers (0.8-12 µL from a 1 mg/mL Milli-Q water stock solution), L2000 (0.8-12 µL from the 1 mg/mL commercial solution) or complexes (100 µL from the complexes solutions described above) were added to the cuvette (final concentration of 0-100 nM siRNA and 0.14-2.1 µM / 1-15 µg/mL peptide dendrimers or 1-15 µg/mL L2000). At t= 270 s 1.2% Triton X-100 was added to the cuvette (50 µL, 0.07% final concentration). Fluorescence intensities were normalized to fractional emission intensity I(t) using I(t) = (It - I0)/(I∞ - I0) where I0 is the intensity before addition of the peptide dendrimer, I∞ is intensity at saturation after lysis by Triton X-100.

Critical Micellar Concentration (CMC): Nile red is known to have a higher fluorescence when being surrounded by a hydrophobic environment and this experiment is derived from known procedure but applied to 96-well plates. To see if the CMC determination is working in our hands with our assay, we first performed it with dodecylphosphocholine and found a value of 0.3125 mg/mL, corresponding to 0.9 mM which is in accordance with values between 0.9 and 1.1 mM from literature (data not shown). Briefly, Nile red (Sigma aldrich, Buchs, CH) was diluted in methanol at a concentration of 2 µM and 5 µL was added to each well of a TPP 96-well plate (Faust Laborbedarf AG, Schaffhausen) and dry under the fumehood air flow at room temperature for 1 h. Serial dilution of the peptide dendrimers, L2000 and dodecylphosphocholine (Avanti polar lipids, Alablaster, USA) were performed in 10 mM phosphate buffer (pH 5 or pH 7.4) starting from 1 mg/mL to 0.5 µg/mL and 50 µL was added to the plate containing the dried Nile red fluorophore (final concentration 0.2 µM). For the second experiment, solution of peptide dendrimers at 1 mg/mL in Milli-Q water were diluted to 0.1 mg/mL in 100 µL PB at pH ranging from 7.4 to 4 and added to dried Nile red. The plates were incubated for 2 h before measurement of fluorescence at λₑₓ= 540 nm and λₑₘ= 615 nm on a Tecan Infinite M1000 Pro plate reader. CMC values can be determined at the inflection point of the curves.

Diffusion NMR (DOSY) Measurements: Diffusion NMR experiments were performed using a Bruker DRX500 with solutions of dendrimer (15 mg/mL) in D₂O (pH 5 or 7.4, at 303 K). The gradient with a maximum strength of 50×10⁻⁴ T·cm⁻¹ was calibrated using the HOD proton signal in D₂O (99.997%). The diffusion time was 125 ms and the gradient duration 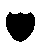 was 6 ms. Data analysis was performed by using the Bruker Simfit software and the diffusion coefficient D [m²s⁻¹] was derived from peak area and intensities. The hydrodynamic radii were calculated from the diffusion coefficient D using the Stokes-Einstein equation Rh= kT/6πηD with Boltzmann constant k= 1.380×10⁻²³ JK⁻¹, temperature T in K and viscosity η= 1.089 mPa. s for D₂O at 303 K.

Titration by NaOH: Peptide dendrimers (0.8-1 µmol, 5-7 mg) were diluted in 7-10 mL Milli-Q water (Final concentration of 100 µM) and acidified to pH 3 with 1 M HCl. Then, 0.1 M NaOH was added by step of 2 µL to the solution with a Dosimat plus (Metrohm, Zofingen, Switzerland) and pH measured on a 692 pH/ion meter (Metrohm).

Circular Dichroism (CD) Spectroscopic Measurements: The CD spectra were recorded using a Jasco J-715 spectrometer equipped with a PFD-350S temperature controller and a PS-150J power supply. All experiments were measured using a Hellma Suprasil R 100-QS 0.1 cm cuvette. Stock solution (1 mg/mL) of peptides dendrimers were freshly prepared in Milli-Q water. For the measurement, the peptides were diluted to a final concentration of 200 µg/mL with PBS buffer (pH = 7.4, 10 mM final concentration). For the solvent dependent studies, the peptides were diluted to 200 µg/mL with phosphate buffer (pH = 5 or 7.4, 8 mM final concentration) and TFE or ACN (0, 5, 10, 15, 20 or 40%). The range of measurement was 185-260 nm, scan rate was 10 nm/min or 20 nm/min, pitch 0.5 nm, response 16 sec. and band 1.0 nm. The nitrogen flow was kept above 8 L/min. The baseline (solvent) was recorded under the same conditions and subtracted manually. Each sample was subjected to two accumulations. The cuvettes were washed with 1M HCl, mQ-deionized H₂O and PBS or PB buffer before each measurement.

Molecular Dynamics: The dendrimer models were built by processing the GROMACS topologies of the linear peptides of the same sequence using in house software. The initial starting conformation was generated using PyMol (Molecular Graphics System, version 1.8 (Schrödinger, LLC)) by setting the (Φ, Ψ) angle pairs of all the residues in a helical conformation.

Molecular dynamics (MD) simulations were performed using GROMACS software version 2016.1 and the Gomos53a6 force field. A dodecahedral box was created around the dendrimer 1.0 nm from the edge of the dendrimer and filled with extended simple point charge water molecules. Sodium and chloride ions were added to produce an electroneutral solution at a final concentration of 0.15 M NaCl.

The energy was minimized using a steepest gradient method to remove any close contacts before the system was subjected to a two-phase position-restrained MD equilibration procedure. The system was first allowed to evolve for 100 ps in a canonical NVT (N is the number of particles, V the system volume, and T the temperature) ensemble at 300 K before pressure coupling was switched on and the system was equilibrated for an additional 100 ps in the NPT (P is the system pressure) ensemble at 1.0 bar.

All bond lengths were constrained to their equilibrium values by using the LINCS algorithm. The neighbor list for the calculation of nonbonded interactions was updated every five time steps with a cutoff of 1.0 nm with a step size of 2 fs. A twin range cutoff of 1.0 nm was used for both Coulomb and Lennard-Jones interactions. The system was split into two groups, "Protein" and "Non-Protein", which were coupled separately to a temperature bath using the V-rescale algorithm with a time constant of 0.1 ps while the pressure coupling was conducted using an isotropic Parrinello-Rahman barostat with a time constant of 2.0 ps.

The stability of the helical structure in the peptide dendrimers under different conditions was assessed by determining the unfolding kinetics as evidenced by the radius of gyration and RMSD values. After system pre-equilibration *(vide supra),* the structures were subjected to MD at 300 K during 1000 ns in water, 0.15 M NaCl with or without 20% v/v TFE. The unfolding the main α-peptide chain helix was followed by computing the RMSD of its backbone and the total number of i→i+4 H-bonds. The overall stability of the internal structure was assessed using the total number of backbone H-bonds over time. The spontaneous appearance of β-sheet and random coil secondary structures was evidenced by Ramachandran number analysis. The last 100 ns (10001 structures) of each 1 ms MD run were clustered using the GROMACS method and a cutoff of 0.3 nm and the central structure of the main cluster was used in the analysis. The Ramachandran numbers were computed using the python package as implemented by Mannige R.. That same structure was used as representative structure of the equilibrated dendrimer in each of the conditions simulated using the PyMol software for building the 3D models.

### DNA

*Cell lines, transfection reagents and plasmids:* The cells were maintained in DMEM medium with 10% (v/v) FCS and 1% (v/v) P/S in a humidified atmosphere in 5% CO₂ and 37 °C. The plasmid CRISPR-Cas9-GFP was purchased from DNA2.0. Lipofectamine 2000 (L2000) was obtained from Sigma-Aldrich. L2000 was used as positive control transfection agents, in accordance with the manufacturer's instructions.

*Transfection procedure:* 24 hours before transfection, cells were seeded in 96well plates in order to reach 70-90% confluence. Peptide dendrimers/DNA complexes were formed by mixing the dendrimers (6.25 µL, from 5 µg to 6.4 µg on N/P ratio 5) with plasmid DNA (250 ng; 6.25 µL). These mixtures were incubated in OptiMEM for 30 min at 25 °C. Transfection control complexes, L2000, was mixed with plasmid DNA (250 ng; 6.25 µL) at the respective manufacturers' recommended concentrations. Before overlaying the DNA complexes on the cells, OptiMEM was added to dilute the complexes, so that each complex contained 250 ng DNA in a total volume of 100 µL in one well of a 96well plate. After removing complete media from the cells, the complexes were added to the plates. The plates were incubated for 4 hours at 37 °C. Then, the transfection solutions were replaced by full growth media for 40 hours before transfection efficiency was assayed.

*Transgene expression assay:* The cells were washed twice with PBS and incubated with trypsin for 20 min at 37 °C. Then 100µL of PBS were added to each well and the transfection efficiency was assessed by FACS analysis (BD LSRFortessa).

### Results siRNA transfection

**Table 1:^{a)} One-letter code amino acids, including the unnatural amino acids 4-fluoro-phenylalanine (f₁), 3,4-Difluoro-phenylalanine (f₂) and Pentafluoro-phenylalanine (F₅ and f₅), Nle/nle are norleucine, aoc is 2-amino-octanoic acid, C₁₁F₁₇ is N-(4,4,5,5,6,6,7,7,8,8,9,9,10,10,11,11,11-Heptadecafluorundecyl) maleimide, K is the branching lysine residue, C-termini are carboxamide CONH₂, all N-termini are NH₂. K(Cₙ) or k(Cₙ) etc. are L- or D-lysine residues acylated at their side chains with a linear fatty acyl residue of the corresponding length, K(C₁₈) = Lys-ε-NHCO-(CH₂)₁₆CH₃. C(Cₙ) or c(Cₙ) are L- or D-cysteine residues with a linear thioalkane of the corresponding length attached with a disulfide bridge, C(C₁₆) = Cys-β-SS(CH₂)₁₅CH₃. ^{b)}Isolated yields as trifluoroacetate salt after preparative HPLC purification. ^{c)}ESI-MS. ^{d)}GAPDH activity in HeLa cells after 4 h transfection by siRNA (40-100 nM) and peptide dendrimers (N/P 10, 0.84-2.1 µM, 6-15 µg/mL) or L2000 (2:1, 1.064-2.66 µg/mL) followed by 48 h incubation in DMEM supplemented with 10% FCS and normalized to siNC (negative control). Experiments were carried out in triplicate in three independent experiments. n.d.= not determined. n.a.= not applicable**

| Short name | Sequence^{a} | Yield^{b} mg (%) | MS^{c} calc./obs. | GAPDH activity (%)^{d} | GAPDH activity (%)^{d} |
|---|---|---|---|---|---|
| | | | | 40 nM | 100 nM |
| L2000 | n.a. | n.a. | n.a. | 53±2 | 33±10 |
| G0 with one side chain acylated lysine: | | | | | |
| **MH02** | (KL)₈(*K*KL)₄(*K*LL)₂*K*K(C₁₈) | 6.0 (2) | 4654.53/4654.56 | n.d. | 39±15 |
| **MH03** | (KL)₈(*K*KL)₄(*K*LL)₂*K*K(C₂₀) | 4.1 (1) | 4682.56/4682.70 | n.d. | 35±7 |
| **MH04** | (KL)₈(*K*KL)₄(*K*LL)₂*K*K(C₂₂) | 3.2 (1) | 4710.59/4710.73 | n.d. | 33±4 |
| **MH05** | (KL)₈(*K*KL)₄(*K*LL)₂*K*K(C₂₄) | 4.5 (1) | 4738.63/4738.63 | n.d. | 35±2 |
| **MH06** | (KL)₈(*K*KL)₄(*K*LL)₂*K*K(C₂₆) | 3.1 (1) | 4766.66/4766.65 | n.d. | 30±6 |
| **MH07** | (KL)₈(*K*KL)₄(*K*LL)₂*K*K(C₂₈) | 3.1 (1) | 4794.69/4794.70 | n.d. | 39±18 |

| G0 with two side chain acylated lysines: | | | | | |
|---|---|---|---|---|---|
| **MH12** | (KL)₈(*K*KL)₄(*K*LL)₂*K*K(C₁₄)K(C₁₄) | 2.3 (1) | 4936.76/4936.90 | n.d. | 37±1 |
| **MH13** | (KL)₈(*K*KL)₄(*K*LL)₂*K*K(C₁₆)*K*(C₁₆) | 26.8 (4) | 4992.83/4992.82 | n.d. | 32±5 |
| **MH14** | (KL)₈(*K*KL)₄(*K*LL)₂*K*K(C₁₈)*K*(C₁₈) | 2.4 (1) | 5048.89/5048.91 | n.d. | 41±11 |
| **MH13D1** | (kl)₈(*K*KL)₄(*K*LL)₂*K*K(C₁₆)K(C₁₆) | 16.2 (2) | 4992.83/4992.85 | n.d. | 31±3 |
| **DMH13** | (kl)₈(*k*kl)₄(*k*ll)₂*k*k(C₁₆)k(Cₗ₆) | 128.4 (7) | 4992.83/4992.84 | n.d. | 31±2 |

| G0 with linear hydrophobic peptide: | | | | | |
|---|---|---|---|---|---|
| **MH18** | (KL)₈(*K*KL)₄(*K*LL)₂*K*LLLL | 52.7 (8) | 4712.51/4712.52 | n.d. | 28±9 |
| **MH19** | (KL)₈(*K*KL)₄(*K*LL)₂*K*LLLLL | 4.7 (1) | 4825.60/4825.61 | n.d. | 34±13 |
| **MH22** | (KL)₈(*K*KL)₄(*K*LL)₂*K*FFF | 13.7 (3) | 4701.38/4701.40 | n.d. | 32±3 |
| **MH25** | (KL)₈(*K*KL)₄(*K*LL)₂*K*WWWW | 4.5 (1) | 5004.49/5004.50 | n.d. | 34±3 |
| **MH26** | (KL)₈(*K*KL)₄(*K*LL)₂*K*WWWWW | 1.7 (1) | 5190.57/5190.63 | n.d. | 37±2 |
| **MH40** | (KL)₈(*K*KL)₄(*K*LL)₂*K*NIeNIeNIeNIe | 5.1 (1) | 4712.51/4712.53 | n.d. | 23±1 |
| **DMH18** | (kl)₈(*k*kl)₄(*k*ll)₂*k*llll | 73.6 (11) | 4712.51/4712.52 | n.d. | 28±9 |
| **kl3,2-fff** | (kl)₈(*k*kl)₄(*k*ll)₂*k*fff | 22.7 (5) | 4701.38/4701.41 | n.d. | 33±2 |
| **D4** | (kl)₈(*k*kl)₄(*k*ll)₂*k*lllc | 129.5 (21) | 4702.44/4702.48 | n.d. | 33±1 |
| **kl3,2-nlenlenlenle** | (kl)₈(*k*kl)₄(*k*ll)₂*k*nlenlenlenle | 19.3 (4) | 4712.51/4712.53 | 33±1 | n.d. |
| **kl3,2-aocaocaoc** | (kl)₈(*k*kl)₄(*k*ll)₂*k*aocaocaoc | 8.4 (2) | 4683.52/4683.52 | 37±2 | n.d. |
| **kl3,2-aocaocaocaoc** | (kl)₈(*k*kl)₄(*k*ll)₂*k*aocaocaocaoc | 13.3 (3) | 4824.64/4824.63 | 31±3 | n.d. |

| Variations in the branching: | | | | | |
|---|---|---|---|---|---|
| **MH44** | (RL)₈(KRL)₄(KLL)₂KLLLL | 25.9 (5) | 5048.59/5048.61 | n.d. | 34±13 |
| **rl3-kl2-nlenlenlenle** | (rl)₈(*k*kl)ₐ(*k*ll)₂*k*nlenlenlenle | 1.6 (1) | 4936.56/4936.56 | 37±1 | n.d. |
| **kl3-rl2-nlenlenlenle** | (kl)₈(*k*rl)ₐ(*k*ll)₂*k*nlenlenlenle | 17.3 (5) | 4824.53/4824.53 | 40±2 | n.d. |
| **kl3,2-nlenle1-C₁₆C₁₆** | (kl)₈(*k*kl)₄(*k*nlenle)₂*k*k(C₁₆)k(C₁₆) | 12.7 (2) | 4992.83/4992.83 | 37±3 | n.d. |

The two D-enantiomeric G3 peptide dendrimers, DMH13 and DMH18, acting with remarkable efficiency as single component reagents for siRNA transfection without helper lipid (Figure 3). These dendrimers combine the polycationic branches of our previously optimized siRNA co-transfection peptide dendrimers, now used in D-enantiomeric form, with a hydrophobic core consisting of either hydrophobic amino acids or fatty acids as replacements for lipofectin to promote endosome escape. Both dendrimers efficiently transfect siRNA in a variety of cell lines and conditions equally or better than the gold standard lipofectamine L2000 without significant toxicity to cells.

Quantifying GADPH knock-down by measuring residual enzyme activity with a NADH reporter assay in dendrimer/siRNA versus dendrimer/siNC treated cells showed that several dendrimers induced knock-down as strongly as the commercial reagent L2000 (33 ± 10 % residual activity relative to siNC treated cells, Table 1). Cells were not significantly affected by treatment with dendrimer/siNC complexes, which did not induce any GADPH knock-down or any decrease in cell viability compared to untreated cells. In terms of dendrimer structure, increasing G0 hydrophobicity increased transfection efficiency in the three dendrimers series up to an optimal hydrophobicity, which was reached with a C26 fatty acid for dendrimers with a single side chain acylated lysine in G0 (MH06, 30 ± 6 %), two C16 fatty acids with dendrimers with two side chain acylated lysines in G0 (MH13, 32 ± 5 %), and four leucines (MH18, 28 ± 9 %), three phenylalanines (MH22, 32 ± 3 %) and four tryptophans (MH25, 34 ± 3 %) for dendrimers with a linear peptide in G0. Surveying additional variations in G0 showed that transfection was also particularly efficient with a hydrophobic core consisting of four norleucines (MH40, 23 ± 1 %).

To further investigate the influence of dendrimer structure on transfection efficiency, we varied the composition of the G1-G3 branches around the tetra-leucine G0 core of MH18, resulting in analogs with different types or numbers of cationic and hydrophobic residues (Table 1). Replacing all cationic lysines in the branches of MH18 with histidines weakened siRNA binding and cellular uptake and abolished transfection. On the other hand, exhaustive substitution with arginines (MH44) led to an unusually strong siRNA binding (unbound siRNA: 1.6 ± 0.3 %) and cellular uptake (GMFI: 594 ± 92 %), while transfection efficiency remained stable (residual GADPH: 34 ± 13 %).

Altering the ratio and distribution of leucines and lysines in the G1-G3 branches also abolished transfection, e.g. by exhaustive deletion of leucines or by K-L exchanges in G1, G2 or G3, although Lys-Leu at G2 still showed strong siRNA binding and internalization into cells.

Considering that both our peptide dendrimers and siRNA are both homochiral, we additionally investigated if variations in amino acid chirality might have an impact on transfection. In the case of MH13 (2×C16 core), transfection efficiency was preserved upon switching to D-residues either in only G3 (MH13D1) or exhaustively (DMH13). In the case of MH18 (L4 core) by contrast, switching to D-amino acids in either G3 (MH18D1), G0 (MH18D2), or in the branching lysines (MH18D3), partially or completely abolished transfection. Surprisingly, diastereoisomer MH18D3 also lost siRNA binding and cellular uptake compared to MH18. Nevertheless, siRNA transfection was preserved in the enantiomer DMH18, showing that homochirality but not the absolute configuration was important for siRNA transfection in this dendrimer.

Transfecting HeLa cells with decreasing amounts of siRNA at a constant N/P = 10 showed that the two D-enantiomeric dendrimers DMH13 and DMH18 produced the most sustained GADPH knock-down after 48 h with as little as 40 nM siRNA (Figure 5a). In both cases, GADPH knock-down was essentially complete at 24 h, 36 h and 48 h as measured by the amount of remaining mRNA (Figure 5b). Remarkably, all dendrimers except the arginine containing MH44 were also effective in the presence of 10% serum between 8 h and 72 h incubation, suggesting possible use in complex environments (Figure 5c). Similar results were obtained on CHO and HEK-293 cells. When surveying different N/P ratios at constant siRNA (100 nM), we observed that the most active dendrimers, namely lipidated dendrimers MH06 (C26) and MH13/DMH13 (2xC16) and the D-enantiomeric leucine dendrimer DMH18, tolerated a wide range of N/P ratio (3 to 10) (Figure 5d). On the other hand, decreasing the concentration of siRNA to 10 and 20 nM at higher N/P ratio (respectively 100 and 50) only gave efficient transfection with dendrimers MH25 (Trp), MH40 (Nle), DMH13 (D-, 2xC16) and DMH18 (D-leu).

We performed transfection experiments with five additional cell lines using the most potent transfection dendrimers MH06, MH13, MH18, MH40, DMH13 and DMH18 in comparison with L2000. Quantifying GADPH knock-down showed that DMH13 and DMH18 and in part the other dendrimers effectively transfected all five cell lines, including SH-SY5Y and Caco-2 cells which are notoriously difficult to transfect (Figure 6). Cell viability was only marginally affected in all of these transfection experiments, with cell toxicity being observed mostly for L2000.

Taken together, these experiments showed that D-enantiomeric dendrimers DMH13 and DMH18 performed best across all conditions and cell lines tested. The better siRNA transfection of D- versus L-peptide dendrimers contrasts to reports of better performance of linear L- versus D-peptides for DNA transfection.

### Biological mechanism of cellular uptake

To understand how our dendrimers enabled siRNA transfection, we investigated cellular uptake of siRNA using the nine transfection dendrimers studied above, together with L2000 as positive control and two close analogs of MH18 showing either low (MH46, L-K in G1) or high (MH47, K-L in G2) internalization of siRNA but no significant GADPH knock-down as negative controls.

Live cell confocal microscopy images of fluorescence labeled siRNA showed that FAM-siRNA was mostly localized in intracellular compartments upon internalization in HeLa cells in all cases (Figure 7a). We observed fluorescence spread in the cell, indicative of endosomal release of siRNA, with lipidated dendrimers MH06, MH13 and DMH13 as well as with L2000, in line with the expected role of lipid components in promoting endosomal escape. Nevertheless, endosomal escape as observed in these live cell confocal images was not correlated with transfection efficiency, as exemplified with two of the best transfection dendrimers MH18 and DMH18 lacking significant siRNA fluorescence in the cytosol. In fact, part of the siRNA remained trapped in intracellular compartments even after 24 h with all dendrimers investigated as well as with L2000 independent of their transfection efficiency. The effect was most visible with transfecting arginine dendrimer MH44 and with non-transfecting control dendrimer MH47, which both showed much stronger cellular uptake than other dendrimers. Similar effects were also observed in CHO and HEK-293 cells.

Cellular uptake of siRNA was inhibited in the presence of various cell internalization inhibitors, as evidenced by flow cytometry (Figure 7b). Cellular uptake was entirely blocked at low temperature (+4 °C). Uptake was also strongly inhibited by cytochalasin D, which blocks actin filaments, indicative of macropinocytosis, as well as by chlorpromazine, indicative of clathrin mediated endocytosis. Furthermore, nystatin inhibited cellular uptake with dendrimers MH18/DMH18, its arginine analog MH44, and the inactive control dendrimer MH46, indicative of entry via caveolae in these cases. The other dendrimers were only partially (MH06, MH13, MH22, MH47) or not affected (DMH13, MH25, MH40) by nystatin.

To check if transfection required endosome acidification as hypothesized for most transfection reagents, we measured overall transfection efficiency in the presence of bafilomycin A1, which inhibits vacuolar ATP-ase and endosome acidification. While treating with bafilomycin for 1h prior to transfection had no effect, transfection was entirely inhibited when bafilomycin was present throughout the experiment, and was strongly reduced even if the compound was added at t = 4h after endocytosis had taken place, indicating that dendrimer mediated transfection required endosome acidification. By contrast, bafilomycin treatment had no effect on L2000 mediated transfection (Figure 7c).

Taken together, the imaging and inhibition experiments above were in agreement with findings reported for various non-viral transfection reagents occurring via energy dependent endocytosis followed by acidification dependent endosomal escape, and suggested that peptide dendrimer mediated siRNA delivery should involve the formation of dendrimer/siRNA nanoparticles.

### Nanoparticle formation

Measuring the extent of siRNA binding by quantifying free siRNA with the Quant-it^{™} assay as function of N/P ratio showed that all eleven selected dendrimers bound siRNA completely independent of their transfection efficiency at N/P > 2, both at pH 7.4 corresponding to the extracellular medium and at pH 5.0 corresponding to the acidified endosome (Figure 8a/b). To test if siRNA was incorporated into nanoparticles upon dendrimer complexation, we tracked binding of FAM-siRNA using fluorescence polarization (FP). Indeed, a strong FP signal was recorded upon addition of the various dendrimers, implying that FAM-siRNA was incorporated into higher molecular weight aggregates, presumed to be nanoparticles. At pH 7.4, the FP signal intensity reached a maximum above N/P = 5, suggesting that the full complexation of siRNA at N/P = 2 was followed by further aggregation to larger nanoparticles by incorporating additional dendrimer molecules (Figure 8c). At pH 5.0 by contrast, the FP signal reached saturation at N/P = 2 and remained stable upon addition of more dendrimer, suggesting that the formed dendrimer/siRNA nanoparticles did not incorporate additional dendrimer at pH 5.0 (Figure 8d).

Measuring displacement of FAM-siRNA from dendrimer complexes formed at N/P = 10 upon addition of polyanionic heparin tracking either free siRNA (Figure 8e) or by the decrease in FP signal intensity (Figure 8f) required in both cases a threshold concentration of heparin before the signal started to change, probably reflecting displacement of excess dendrimer from the nanoparticles until the stoichiometry corresponding to N/P = 2 was reached. Above that threshold, the extent of displacement varied strongly between the different dendrimers. FAM-siRNA was most tightly bound by polyarginine dendrimer MH44 and non-transfecting control MH47, which both showed very high cellular uptake, suggesting that these dendrimers form very tight siRNA complexes and cannot release their siRNA cargo. Heparin displacement was also incomplete with amino acid only dendrimers (MH18, MH22, MH25, MH40, MH46, DMH18), and was easiest with lipidated dendrimers, implying that these formed significantly weaker complexes with siRNA (MH06, MH13, DMH13). Note that the commercial reagent lipofectamine L2000 released siRNA immediately at the lowest heparin concentration used, showing a much weaker complexation compared to our dendrimers.

We further confirmed the formation of nanoparticles using dynamic light scattering (DLS), which showed that dendrimer/siRNA complexes formed nanoparticles in the size range of 80 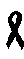 200 nm at pH 7.4 (Figure 9a). Nanoparticles were significantly smaller at pH 5.0, confirming the effect seen by FP (Figure 9c). The largest particles were formed by dendrimer MH47, which bound and internalized siRNA strongly but did not transfect. All dendrimer/siRNA nanoparticles had positive zeta potential values between +10.4 mV (MH46) and +17.7 mV (DMH13) indicative of an overall positive charge, in line with FP measurements indicating further aggregation of dendrimers above N/P = 2 (Figure 9b). Zeta potential values were higher and more spread at pH 5.0, ranging between +13.3 mV (MH46) and +40.3 mV (DMH13), possibly indicating increased nanoparticle stability at that pH (Figure 9d).55 Note that our dendrimers behaved very differently from L2000, which formed larger aggregates with comparable size at both pH values, but with zeta potential values changing from strongly negative at pH 7.4 (-50 mV) to slightly positive at pH 5.0 (+10.7 mV). Transmission electron microscopy (TEM) further confirmed the formation of nanoparticles (Figure 9e). TEM images were obtained by combining dendrimers as their trifluoroacetate salts with siRNA in pure water, resulting in approximately pH = 5, and showed a wide range of sizes for dendrimer/siRNA complexes in line with the distribution of sizes observed by DLS at pH 5.0.

We performed two additional experiments showing that siRNA and dendrimers were both tightly bound within nanoparticles. In the first experiment, we extensively dialyzed complexes formed between a fluorescence labeled Cy3-siRNA and our dendrimers using dialysis membranes with a molecular weight cut-off of 100 kDa, which should separate uncomplexed Cy3-siRNA from dendrimer/Cy3-siRNA nanoparticles (Figure 9a). A strong fluorescence was detected in the dialysis filtrate in the absence of dendrimer, indicating that free Cy3-siRNA was able to diffuse through the membrane, but decreased to a non-detectable level upon increasing dendrimer concentration up to N/P = 10, indicating that Cy3-siRNA was entirely incorporated into aggregates larger than 100 kDa. siRNA was also bound tightly by our dendrimers at pH = 5.0, while L2000 only bound siRNA tightly at pH 5.0 but only weakly at pH 7.4 (Figure 9b).

In a second experiment we measured leakage of fluorescein from vesicles composed of phosphatidyl choline, a zwitterionic lipid forming neutral membranes related to the membrane of a eukaryotic cell. In this experiment, fluorescence is auto-quenched under the high fluorescein concentration within vesicles but recovers by dilution if fluorescein is released from the vesicles, which occurs upon addition of membrane disrupting compounds. Indeed, we observed a strong fluorescence increase upon addition of free dendrimers at 15 µg/mL (red curves, figure 10c). The effect was significant with all transfecting dendrimers even at 1 µg/mL (yellow curves, figure 10c), as could be expected from their cationic/hydrophobic composition, but much weaker with the two negative controls MH46 and MH47 which have a higher respectively lower ratio of cationic/hydrophobic side chains compared to transfecting dendrimers. Remarkably, adding siRNA to 15 µg/mL dendrimer (cyan curves, figure 10c) either entirely blocked fluorescein release from the vesicles (MH06, MH22), or reduced it very strongly (e.g. MH13/DMH13, MH40) or at least to the level observed with only 1 µg/mL free dendrimer (e.g. MH18, MH44), indicating that the dendrimers were almost completely incorporated into siRNA complexes, which themselves did not have significant membrane disruptive activity. This effect is striking since with N/P = 10 peptide dendrimers are in large excess compared to siRNA. In contrast to our dendrimers, L2000 did not cause any measurable vesicle leakage with or without siRNA (black curve, Figure 10c).

### pH dependent aggregation of peptide dendrimers

The amphiphilic topology of our dendrimers and the effects of hydrophobic core size and composition on siRNA binding and transfection suggested that dendrimer-siRNA interactions might be mediated by self-aggregation of the dendrimers prior to siRNA binding. Such self-aggregation in the absence of siRNA would involve intermolecular interactions potentially explaining the much larger differences observed in siRNA binding between diastereoisomers (MH18/MH18D3) than between enantiomers (MH18/DMH18).

Measuring aggregation using the Nile Red assay57 showed that all peptide dendrimers with strong siRNA binding properties formed aggregates at pH 7.4, which critical micellar concentration (CMC) value below the concentration range used in our transfection experiments (Figure 11a). By contrast dendrimers that weakly bound siRNA, such as MH18D3 and MH46, did not show any measurable aggregation at that pH across the entire concentration range tested. At pH = 5 there was a sharp drop in aggregation, indicated by higher or non-detectable CMC, across all dendrimers except the negative control dendrimer MH47 which bound but did not release siRNA and whose CMC were the same at pH = 7.4 and pH = 5 (Figure 11b). CMC and siRNA binding correlated with dendrimer hydrophobicity when comparing the optimal transfection dendrimer MH18 with negative controls MH46 (double L-K exchange in G1 of MH18 reducing hydrophobicity, no self-aggregation, no siRNA binding) and MH47 (four-fold K-L exchange in G2 of MH18 increasing hydrophobicity, strong self-aggregation at both pH and strong siRNA binding without transfection). The effect was also visible when investigating aggregation as function of pH, which showed that lipidated dendrimer MH06/MH13/DMH13 aggregated at a lower pH than amino acid only dendrimers (Figure11).

Dendrimer aggregation was further evidenced by measuring the hydrodynamic radii (Rh) by DOSY NMR at pH 7.4 and 5.0 in the case of MH13, DMH13, MH18, DMH18, MH18D3, MH46 and MH47 (Table 3). While MH18D3 and MH46 gave comparable values of Rh = 2.42 / 2.52 nm and 1.98 / 2.06 nm compatible with a monomeric state at both pH values despite of the relatively high concentration of the NMR measurement (15 mg/mL), MH18 and DMH18 were monomeric at pH 5.0 (Rh = 2.52 nm and 2.42 nm) but aggregated at pH 7.4 (Rh = 3.21 nm and 3.11 nm), while MH13 and DMH13 were aggregated at both pH value (Rh = 4.66 / 4.97 nm and Rh = 4.14 / 4.95 nm).

**Table 3:^{a)} The hydrodynamic radii (Rₕ) were calculated from diffusion coefficients D, which are the median values from area and intensity fit analysis of one ¹H measurement in D₂O pH 5 or pH 7.4 and the Stokes-Einstein equation Rₕ=kT/6πηD, with Boltzmann constant k=1.380x10⁻²³ J.K⁻¹, temperature T=303 K, and viscosity η=1.089 mPa.s for D₂O. ^{b)}Number of peptide dendrimers are calculated by dividing the particles volume by the volume of a monomeric dendrimer having a 2 nm radius. Dendrimer MH47 could not be measured due to limited solubility.**

| Compound | | pH 5 | | | pH 7.4 | |
|---|---|---|---|---|---|---|
| | D [m².s⁻¹].10⁻¹¹ | Rₕ [nm]^{a} | dendrimer/ aggregate^{b} | D [m².s⁻¹].10⁻¹¹ | Rₕ [nm]^{a} | dendrimer/ aggregate^{b} |
| **MH13** | 4.92±0.04 | 4.14±0.03 | ~ 9 | 4.17±0.34 | 4.95±0.44 | ~ 15 |
| **DMH13** | 4.37±0.04 | 4.66±0.05 | ~ 12 | 4.10±0.08 | 4.97±0.09 | ~ 15 |
| **MH18** | 8.10±0.12 | 2.52±0.04 | ~ 2 | 6.41±0.59 | 3.21±0.27 | ~ 4 |
| **DMH18** | 8.42±0.09 | 2.42±0.03 | ~ 2 | 6.56±0.29 | 3.11±0.13 | ~ 4 |
| **MH18D3** | 8.44±0.12 | 2.42±0.04 | ~ 2 | 8.15±0.86 | 2.52±0.28 | ~ 2 |
| **MH46** | 10.27±0.02 | 1.98±0.03 | ~ 1 | 9.89±0.04 | 2.06±0.07 | ~ 1 |

The pH dependence of aggregation correlated with the acid-based properties of the dendrimers. Titration of dendrimers MH13, DMH13, MH18, DMH18, MH18D3, MH46 and MH47 showed two apparent pKa values with relative stoichiometry consistent with ionization of amino termini at acidic pH (pKa1 = 6.5) and of lysine side chains at higher pH (pKa2 = 8.5-10) (Figure 11d). Protonation of amino termini at pKa1 = 6.5, leading to an increase by eight positive charges in the dendrimer, readily explained the switch in aggregation between pH 7.4 and pH 5. On the other hand, pKa2 values for lysine side chain protonation decreased in the series MH18D3/MH46 > MH18/DMH18 > MH13/DMH13, following the order of increased self-aggregation propensity, showing that aggregation decreased the basicity of lysine side chains. MH47 precipitated during acid-base titration, precluding determination of its pKa2 value.

Taken together, CMC, DOSY-NMR and acid-base titration experiments showed a consistent picture of pH dependent self-aggregation of our dendrimers enabling siRNA complexation, including the surprising diastereoselective complexation of siRNA by the self-aggregating MH18/DMH18 compared to their diastereoisomer MH18D3, which neither bound siRNA nor aggregated. However, while these effects primarily followed overall dendrimer hydrophobicity, hydrophobicity failed to explain the difference between MH18 and diastereoisomer MH18D3.

### Circular dichroism

Since stereochemistry should influence conformation, we measured the secondary structure content of our dendrimers by circular dichroism (CD) in aqueous medium containing up to 20% v/v trifluoroethanol (TFE) as an inducer of secondary structures (Figure 12).

CD spectra of lipidated dendrimers MH13/DMH13 at pH = 7.4 showed a significant β-sheet content, which increased from ~35 % in water to ~40% upon addition of 20 % v/v trifluoroethanol (TFE) at the expense of α-helix content despite of the fact that TFE normally induces α-helices (Figure 12a/d). β-Sheet content also increased at pH 7.4 with transfection dendrimer MH18/DMH18 up to 10% v/v TFE (25%-33% β-sheet) but decreased again with 20% v/v TFE (22% β-sheet) in favor of an α-helix (30% α-helix), probably reflecting the α-helix propensity the linear tetra-leucine core (Figure 12b/e). Increases in β-sheet content without induction of α-helix in transfection dendrimers were similarly observed upon addition of acetonitrile as cosolvent. β-sheet content also increased as function of hydrophobic core size in dendrimers with G0 containing one (MH01-MH07) or two (MH08-MH15) side-chain acylated lysines, or zero to five leucines (MH30, MH16-MH20), a trend which followed their ability to aggregate, as well as their siRNA binding and cell internalization. On the other hand, dendrimer MH47, which aggregated and bound siRNA tightly but did not transfect, kept an overall stable α-helix and β-sheet content upon addition of 20% v/v TFE (11%-14% α-helix, 36%-33% β-sheet, (Figure 12c/f). Furthermore, at pH 7.4 the β-sheet content of dendrimer MH46, which neither aggregated nor bound siRNA, decreased from pure water to 20% v/v TFE (28%-20% β-sheet) in favor of the α-helix content (16%-26% α-helix).

CD spectra recorded at pH 5.0, under which conditions dendrimers were not or only partially aggregated, showed predominantly α-helical conformations with enhancement of α-helix content upon addition of TFE for all dendrimers, with the exception of the strongly aggregating and siRNA binding non-transfecting dendrimer MH47 which retained a predominantly β-sheet conformation.

The β-sheet propensity of our transfection dendrimers was surprising to us because previous conformational studies with structurally related antimicrobial peptide dendrimers (AMPDs) showed a strong tendency towards α-helical conformations in CD spectra. These AMPDs were significantly less hydrophobic and did not form any aggregates in water, suggesting that the β-sheet conformations observed in our transfection dendrimers might reflect intermolecular interactions in the aggregated state.

### Molecular dynamics

To test whether our transfection dendrimers might contain β-sheet conformations in their monomeric state we preformed molecular dynamics (MD) simulations with transfection dendrimers MH13 and MH18 and non-transfecting controls MH18D3, MH46 and MH47 in explicit water with or without 20 % v/v TFE as an inducer of secondary structures. As starting conformations, we built 3D-models using PyMol (Version 1.8, Schrödinger, LLC) with all dendrimer branches in a pre-folded α-helical conformation and fully protonated dendrimers to simulate pH 5.0 and dendrimers with protonated side chains and amino termini as free bases to simulate pH 7.4. We ran simulations of monomeric species over the course of one microsecond using GROMACS.

The simulations gave comparable results in pure water at both pH values, under which conditions all dendrimers rapidly unfolded and rearranged to a compact conformation with an almost constant radius of gyration across the entire trajectory. Clustering of the last 100 ns produced well defined 3D-models which we analyzed as the average structure as 3D models and in terms of individual residue conformation across the dendrimer using the Ramachandran number. In almost all cases residues along the central α-peptide chain adopted an α-helical conformation from G0 to G2, while residues in branches starting with isopeptide bonds were either disordered or in β-sheet conformation. In the presence of TFE dendrimers similarly unfolded and rearranged, however the dendrimers were more open and flexible and showed a slightly larger radius or gyration than in water, however the number of backbone H-bonds was similar if not higher with TFE compared to water (Figure 13a/c). This analysis showed a conserved α-helix in the central α-peptide chain at the level of G0/G1/G2, as illustrated for the case of transfection dendrimer MH18 at pH 5 (Figure 13b/d). One notable exception was MH13 at pH 5, under which conditions the dendrimer opened up completely and had β-sheet conformations across the entire structure (Figure 13b/e).

The present MD simulations above only very poorly reproduced CD data in terms of variations in α-helix and β-sheet contents between different dendrimers, pH and solvent conditions. We consider this discrepancy as additional evidence that our transfection dendrimers directly interact with each other within the aggregated state, most likely by forming intermolecular β-sheets. Although this situation cannot be easily modelled, we have previously observed β-sheet cross-links between peptide dendrimers in the crystal structure of a lectin-bound glycopeptide dendrimer engaging in trimeric aggregates.

### Mechanistic model of dendrimer promoted siRNA transfection

In view of cellular uptake, nanoparticle formation, pH-dependent aggregation, CD and MD data presented above, we propose the following overall mechanistic model for dendrimer mediated siRNA transfection. First, transfection dendrimers presenting a favorable arrangement of cationic and hydrophobic groups self-aggregate at pH 7.4 via intermolecular β-sheet cross-links in a stereoselective process accessible to homochiral dendrimers such as MH18/DMH18 but not their diastereoisomer MH18D3. These dendrimer aggregates then complex siRNA to form stable nanoparticles accumulating an excess of dendrimer over siRNA up to N/P > 6. In a third step, these nanoparticles enter cells by endocytosis and localize in endosomes. Endosome acidification then induces protonation of dendrimer amino termini, which triggers disaggregation, presumably by electrostatic repulsion.

Disaggregation results in dissolution of the intermolecular β-sheet cross-links between dendrimers and rearrangement to a monomeric α-helical conformation, which releases excess free monomeric dendrimer, leaving behind smaller dendrimer/siRNA nanoparticles with a lower N/P ratio. Protonation of the amino termini probably also causes a proton sponge effect enabling endosome escape as also postulated for many transfection reagents. The process is only very partial as evidenced by confocal images showing endosome localization of FAM-siRNA in almost all cases up to 24 h, in line with studies of siRNA transfection lipids showing that less than 4 % of the siRNA actually escapes the endosome. In the case of MH13/DMH13 for which a broader spread of FAM-siRNA into the cytosol is observed we postulate that endosome escape is further assisted by disruption of the endosomal membrane by the liberated dendrimer which probably exists in an open conformation exposing its hydrophobic groups for membrane disruption similar to the model obtained by MD for MH13 at pH 5 in the presence of TFE.

For the few dendrimer/siRNA nanoparticles reaching the cytosol after endosome escape, equilibration to pH 7.4 would be expected to partly liberate siRNA since the N/P ratio of these nanoparticles would be lower. To model this process, we repeated the dialysis filtration experiment at pH 5 to form rearranged dendrimer/siRNA nanoparticles, and re-equilibrated to pH 7.4 to mimic entry into the cytosol and performed a new dialysis filtration at that pH value. While the release from the tight binding transfecting MH44 and non-transfecting dendrimer MH47 was very low in this experiment, transfecting dendrimers such as MH18/DMH18, MH22, MH25 and MH40 released a significant amount of FAM-siRNA. Note that transition to pH 5 was necessary to induce siRNA release. Non-transfecting dendrimer MH46 also released its siRNA, however the dendrimer did not significantly internalize into cells. Finally, the amount of siRNA liberated from lipidated dendrimers MH06 and MH13/DMH13 was lower, however this is probably compensated by a much more extensive endosome escape of the nanoparticles in these cases.

Gene knock-down is most efficient with dendrimers showing an intermediate level siRNA binding. The most efficient peptide dendrimers are D-enantiomeric peptide dendrimers DMH13 and DMH18.

Structure-activity relationships and mechanistic studies provide an unprecedented insight into the transfection mechanism. Efficient transfection depends on a favorable ratio and arrangement of hydrophobic and cationic groups in the dendrimers and the ability to aggregate in a stereoselective process involving intermolecular β-sheet cross-links to enable the formation of dendrimer/siRNA nanoparticles. Stereoselectivity is highlighted by the lack of aggregation and siRNA binding with MH18D3, a diastereoisomer of the best transfection dendrimer DMH18. Protonation of dendrimer amino termini (pKa ~ 6.5) upon endosome acidification is also essential in enabling nanoparticle rearrangement and possibly endosome escape by a proton sponge effect.

The described dendrimers are easily obtained as pure products from commercial building blocks by SPPS followed by preparative HPLC purification, ensuring reproducibility of biological results and transferability of the reagents to other laboratories. The possibility to fine-tune their activity by modifying their amino acid sequence and the nature of the lipidated core opens the door to further optimization towards targeted applications

### Results DNA transfection

For example, a study for transfecting plasmid DNA using similar peptide dendrimers and a large plasmid DNA coding for CRISPR-Cas9-GFP (~9Kbp). The Cas9-2A-GFP vector allows the co-expression of Cas9 and GFP from the same mRNA. The self-cleavable peptide 2A allows the release of GFP reporter in the cytoplasm after the expression, so that the transfection efficiency was determined by tracking the expression of GFP (green fluorescent protein) in various cell lines upon exposure to the corresponding plasmid complexes with dendrimers at N/P = 5. We tracked transfection efficiency by fluorescence activated cell sorting, and counted the percentage of successfully transfected cells, which appeared fluorescent. The results showed that DNA transfection requires dendrimer branches of composition (KL)₈(*K*KL)₄(*K*KL)₂*K* combined with a hydrophobic core according to the example shown in the table below. Most remarkably, dendrimer mediated transfection is found to be far more efficient than with the reference reagent L2000. We noticed that the presence of a cysteine residue was favourable as one of the hydrophobic residues in the dendrimer core, and found that the corresponding disulfide bridged dimeric dendrimers had similar transfection ability. However, cysteine containing monomers also transfected in the presence of reducing agents preventing dimer formation, showing that disulfide bridge formation is not required for DNA transfection. Mechanistic studies on aggregation and nanoparticle formation show that, similarly to siRNA transfection dendrimers, DNA transfection dendrimers also undergo self-aggregation and nanoparticle formation as a necessary step for DNA transfection.

**Table 2:^{a)} One-letter code amino acids, K is the branching lysine residue, C-termini are carboxamide CONH₂, all N-termini are free. K(Cₙ) or k(Cₙ) etc. are L- or D-lysine residues acylated at their side chains with a linear fatty acyl residue of the corresponding length, K(C₁₈) = Lys-ε-NHCO-(CH₂)₁₆CH₃. ^{b)} Isolated yields as trifluoroacetate salt after preparative HPLC purification. ^{c)} (+) ESI-MS. ^{d)} Transfection efficiency measured by FACS analysis and expressed as percentage of fluorescent cells counting 20K events. ^{e)} FI refers to the Fold Increase in transfection efficiency in comparison to L2000. n.a.= not applicable**

| Short name | Sequence^{a} | Yield^{b} mg (%) | MS^{c} calc/obs | Transfection efficiency (%)^{d} | | | | | |
|---|---|---|---|---|---|---|---|---|---|
| | | | | DU 145 | FI^{e} | NIH 3T3 | FI^{e} | HEK | FI^{e} |
| L2000 | n.a. | n.a. | n.a. | 8±1 | | 2.7±0.6 | | 42.6±1 | |
| KL1,2,3-C18C18 | (KL)₈(*K*KL)₄(*K*KL)₂*K*K(C₁₈)K(C₁₈) | 2.5 (1) | 5078.91/5078.90 | 40.8±11 | 5 | | | | |
| KL1,2,3-OleicAcid-C | (KL)₈(*K*KL)₄(*K*KL)₂*K*K(OleicAcid)C | 2.3 (2) | 4785.55/4785.56 | 50.6±9 | 6 | | | | |
| KL1,2,3-C18-C | (KL)₈(*K*KL)₄(*K*KL)₂*K*K(C₁₈)C | 2.5 (2) | 4787.56/4787.57 | 53.7±16 | 7 | 19.8±3 | 7 | | |
| KH2,3-KL1-OleicAcid-OleicAcid | (KH)₈(*K*KH)₄(*K*KL)₂*K*K(OleicAcid)K (OleicAcid) | 2.7 (3) | 5362.58/5362.58 | 42±14 | 5 | | | | |
| (KL1,2,3-C18-C)2 | ((KL)₈(*K*KL)₄(*K*KL)₂*K*K(C₁₈)C)₂ | 1.9 (2) | 9573.10/9573.10 | 49.8114 | 6 | 21±5 | 8 | | |
| (KL2,3-LLLC)2 | ((KL)₈(*K*KL)₄(*K*LL)₂*K*K(C₁₈)KLLLC)₂ | 1.9 (7) | 9409.10/9407.87 | 44.7±5 | 6 | | | | |
| KL1,2,3-C22 | (KL)₈(*K*KL)₄(*K*KL)₂*K*K(C₂₂) | 11.4 (8) | 4740.62/4740.63 | 30.6±4 | 4 | 16.2±4 | 6 | | |
| KL1,2,3-C16C16 | (KL)₈(*K*KL)₄(*K*KL)₂*K*K(C₁₆)K(C₁₆) | 39.1 (8) | 5022.85/5022.86 | 70.6±8 | 9 | 24.3±8 | 9 | | |
| kl1,2,3-C16C16 | (kl)₈(*k*kl)₄(*k*kl)₂*k*k(C₁₆)k(C₁₆) | 24.9 (5) | 5022.85/5022.88 | 72.1±7 | 9 | 27.4±4 | 10 | | |
| kl1,2,3-C16-c | (kl)₈(*k*kl)₄(*k*kl)₂*k*k(C₁₆)c | 6.04 (3) | 4759.53/4759.54 | | | | | 60.7±2 | 1 |
| kl1,2,3-C18-c | (kl)₈(*k*kl)₄(*k*kl)₂*k*k(C₁₈)c | 3.1 (3) | 4787.56/4787.57 | | | | | 54.5±6 | 1 |
| kl1,2,3-OleicAcid-c | (kl)₈(*k*kl)₄(*k*kl)₂*k*k(OleicAcid)c | 5.85 (4) | 4786.00/4785.55 | | | | | 52.5±4 | 1 |
| (kl1,2,3-C16-c)₂ | ((kl)₈(*k*kl)₄(*k*kl)₂*k*k(C₁₆)c)₂ | 1.64 (3) | 9517.06/9518.07 | | | | | 53±5 | 1 |
| (kl1,2,3-C18-c)₂ | ((kl)₈(*k*kl)₄(*k*kl)₂*k*k(C₁₈)c)₂ | 2.18 (3) | 9573.12/9573.13 | | | | | 61.5±4 | 1 |

Further examples for DNA transfection are given below.

| Short name | Sequence^{a} | Yield^{b} mg(%) | MS^{c} calc/obs | Transfection efficiency (%)^{d} HEK | Transfection efficiency (%)^{d} HeLa |
|---|---|---|---|---|---|
| L2000 | n.a. | n.a. | n.a. | 44.5 ±6 | 34.3 ±10 |
| kl1,2,3-c18-a | (kl)₈(*k*kl)₄(*k*kl)₂*k*k(C₁₈)a | 114 (18) | 4755.59/4755.59 | 54.6±12 | 20 ± 5 |
| kl1,2,3-c18 | (kl)₈(*k*kl)₄(*k*kl)₂*k*k(C₁₈) | 20 (23) | 4684.55/4684.56 | 57.7 ± 11 | 15.6 ± 5 |
| kl1,2,3-cl6 | (kl)₈(*k*kl)₄(*k*kl)₂*k*k(C₁₆) | 22 (16) | 4656.52/4656.52 | 52.8 ± 12 | 16.3 ± 6 |
| rl1,2,3-c18 | (rl)₈(*k*rl)₄(*k*rl)₂*k*k(C₁₈) | 13 (15) | 5076.64/5076.63 | 84.2 ±5.9 | 41 ± 4 |
| rl1,2,3-c16 | (rl)₈(*k*rl)₄(*k*rl)₂*k*k(C₁₆) | 14 (11) | 5048.61/5048.60 | 81.6 ±8 | 30.3 ±12 |
| rl1,2,3-c18-c | (rl)₈(*k*rl)₄(*k*rl)₂*k*k-(C₁₈)c | 26.6 (14) | 5179.65/5179.66 | 62.5 ± 9 | 47.3 ± 9 |
| rl1,2,3-c16-c | (rl)₈(*k*rl)₄(*k*rl)₂*k*k-(C₁₆)c | 9 (4) | 5151.62/5151.62 | 60.6 ± 6 | 41.6 ± 10 |
| rl1,2,3-c18-a | (rl)₈(*k*rl)₄(*k*rl)₂*k*k(C₁₈)a | 25 (12) | 5147.68/5147.68 | 73.2 ± 5 | 52.9 ± 3 |

| | | | | | |
|---|---|---|---|---|---|
| ^{a)} One-letter code amino acids, *K* is the branching lysine residue, C-termini are carboxamide CONH₂, all N-termini are free. K(Cₙ) or k(Cₙ) etc. are L- or D-lysine residues acylated at their side chains with a linear fatty acyl residue of the corresponding length, K(C₁₈) = Lys-ε-NHCO-(CH₂)₁₆CH₃, K(C₁₆) = Lys-ε-NHCO-(CH₂)₁₄CH₃ ^{b)} Isolated yields as trifluoroacetate salt after preparative HPLC purification. ^{c)} (+) ESI-MS. ^{d)} Transfection efficiency measured by FACS analysis and expressed as percentage of fluorescent cells counting 10K events. | | | | | |

### Examples

**MH02 ((KL)₈(*K*KL)₄(*K*LL)₂*K*K(C₁₈))** was obtained from the CEM Liberty Blue synthesizer as foamy colourless solid after preparative RP-HPLC (6.0 mg, 0.86 µmol, 2%). Analytical RP-HPLC: t_{R} = 3.44 min (100% A to 100% D in 7.5 min, λ= 214 nm). MS (ESI+): C₂₃₄H₄₅₃N₅₇O₃₇ calc./obs. 4654.53/4654.56 [M+H⁺].

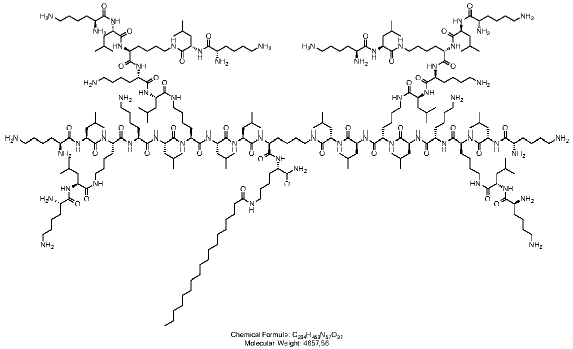

**MH03 ((KL)₈(*K*KL)₄(*K*LL)₂*K*K(C₂₀))** was obtained after manual synthesis as foamy colourless solid after preparative RP-HPLC (4.1 mg, 0.59 µmol, 1%). Analytical RP-HPLC: t_{R} = 4.01 min (100% A to 100% D in 7.5 min, λ= 214 nm). MS (ESI+): C₂₃₆H₄₅₇N₅₇O₃₇ calc./obs. 4682.56/4682.70 [M+H⁺].

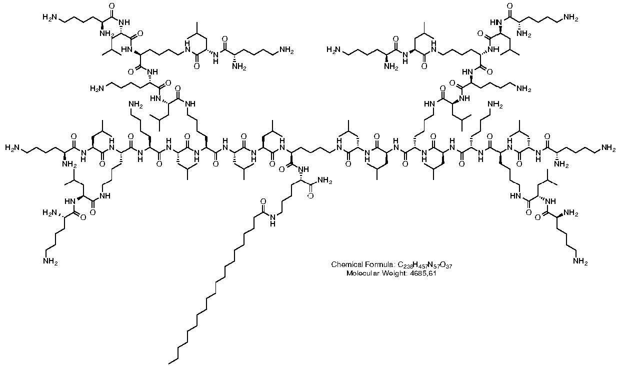

**MH04 ((KL)₈(*K*KL)₄(*K*LL)₂*K*K(C₂₂))** was obtained after manual synthesis as foamy colourless solid after preparative RP-HPLC (3.2 mg, 0.46 µmol, 1%). Analytical RP-HPLC: t_{R} = 4.20 min (100% A to 100% D in 7.5 min, λ= 214 nm). MS (ESI+): C₂₃₈H₄₆₁N₅₇O₃₇ calc./obs. 4710.59/4710.73 [M+H⁺].

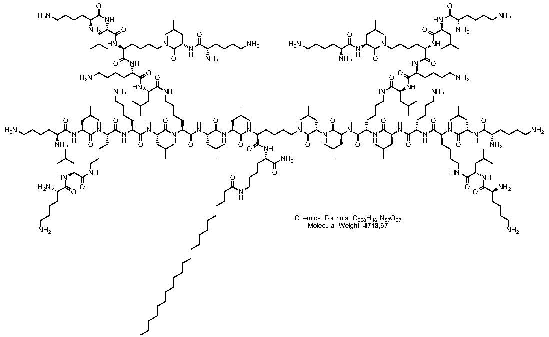

**MH05 ((KL)₈(*K*KL)₄(*K*LL)₂*K*K(C₂₄))** was obtained after manual synthesis as foamy colourless solid after preparative RP-HPLC (4.5 mg, 0.6 µmol, 1%). Analytical RP-HPLC: t_{R} = 4.40 min (100% A to 100% D in 7.5 min, λ= 214 nm). MS (ESI+): C₂₄₀H₄₆₅N₅₇O₃₇ calc./obs. 4738.63/4738.63 [M+H⁺]

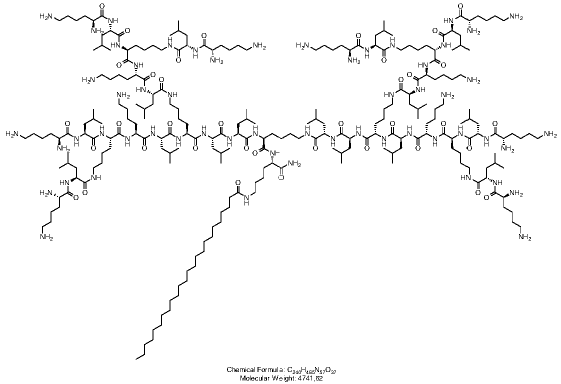

**MH06 ((KL)₈(*K*KL)₄(*K*LL)₂*K*K(C₂₆))** was obtained after manual synthesis as foamy colourless solid after preparative RP-HPLC (3.1 mg, 0.44 µmol, 1%). Analytical RP-HPLC: t_{R} = 4.67 min (100% A to 100% D in 7.5 min, λ= 214 nm). MS (ESI+): C₂₄₂H₄₆₉N₅₇O₃₇ calc./obs. 4766.66/4766.65 [M+H⁺].

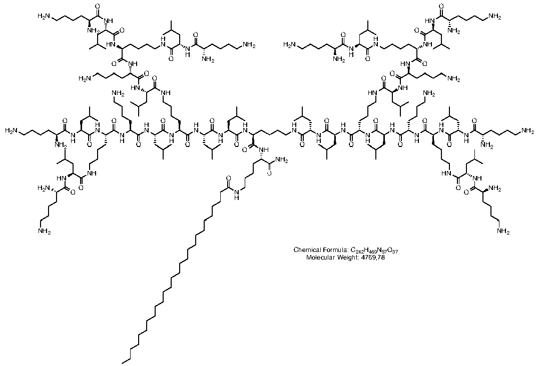

**MH07 ((KL)₈(*K*KL)₄(*K*LL)₂*K*K(C₂₈))** was obtained after manual synthesis as foamy colourless solid after preparative RP-HPLC (3.1 mg, 0.44 µmol, 1%). Analytical RP-HPLC: t_{R} = 4.81 min (100% A to 100% D in 7.5 min, λ= 214 nm). MS (ESI+): C₂₄₄H₄₇₃N₅₇O₃₇ calc./obs. 4794.69/4794.70 [M+H⁺].

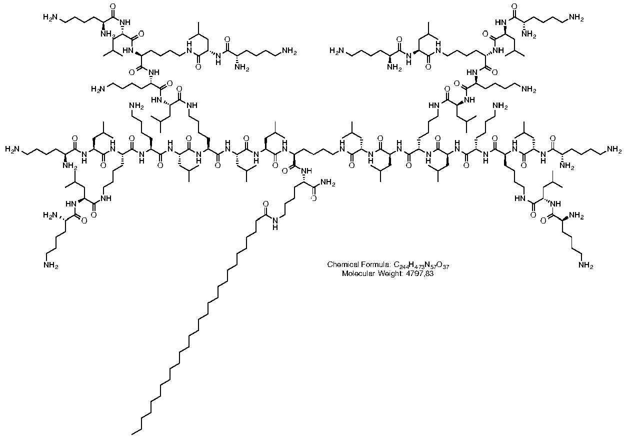

**MH12 ((KL)₈(*K*KL)₄(*K*LL)₂*K*K(C₁₄)K(C₁₄))** was obtained after manual synthesis as foamy colourless solid after preparative RP-HPLC (2.3 mg, 0.32 µmol, 1%). Analytical RP-HPLC: t_{R} = 4.27 min (100% A to 100% D in 7.5 min, λ= 214 nm). MS (ESI+): C₂₅₀H₄₈₃N₅₉O₃₉ calc./obs. 4936.76/4936.90 [M+H⁺].

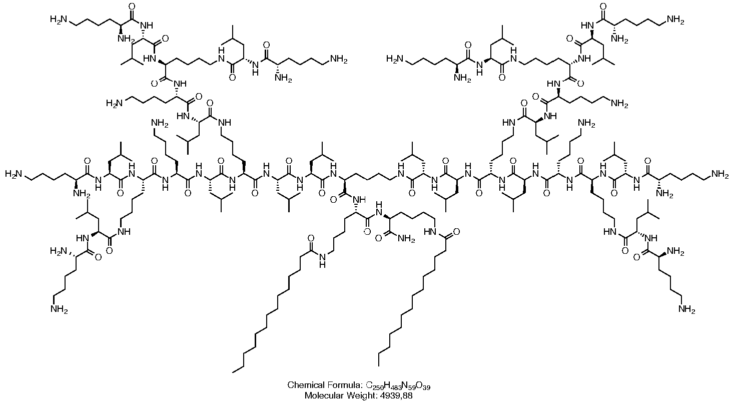

**MH13** (**(KL)₈(*K*KL)₄(*K*LL)₂*K*K(C₁₆)K(C₁₆))** was obtained from the Biotage Initiator+ Alstra synthesizer as foamy colourless solid after preparative RP-HPLC (26.8 mg, 3.68 µmol, 4%). Analytical RP-HPLC: t_{R} = 4.68 min (100% A to 100% D in 7.5 min, λ= 214 nm). MS (ESI+): C₂₅₄H₄₉₁N₅₉O₃₉ calc./obs. 4992.83/4992.82 [M+H⁺].

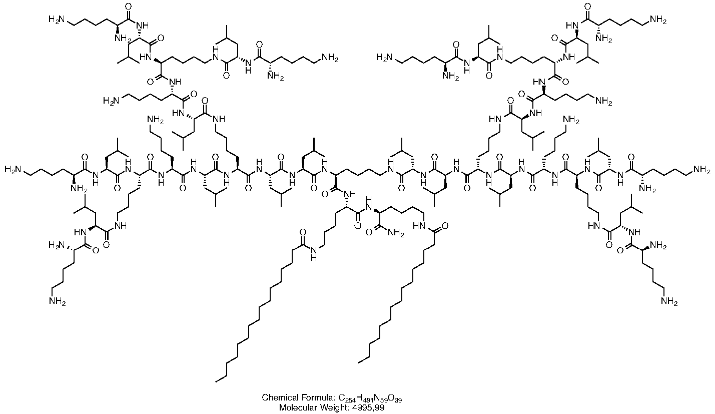

**MH14 ((KL)₈(*K*KL)₄(*K*LL)₂*K*K(C₁₈)K(C₁₈))** was obtained after manual synthesis as foamy colourless solid after preparative RP-HPLC (2.4 mg, 0.3 µmol, 1%). Analytical RP-HPLC: t_{R} = 5.08 min (100% A to 100% D in 7.5 min, λ= 214 nm). MS (ESI+): C₂₅₈H₄₉₉N₅₉O₃₉ calc./obs. 5048.89/5048.91 [M+H⁺].

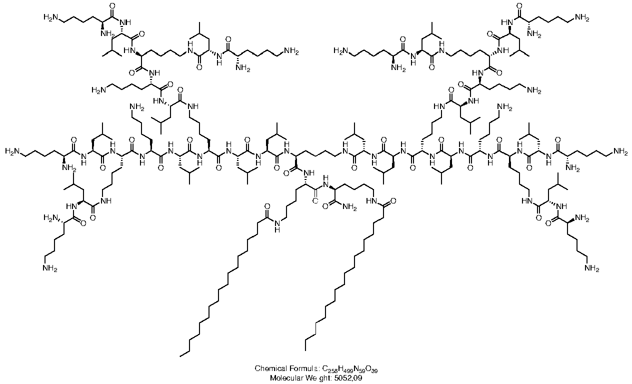

**MH13D1 ((kl)₈(*K*KL)₄(*K*LL)₂*K*K(C₁₆)K(C₁₆))** was obtained from the CEM Liberty Blue synthesizer as foamy colourless solid after preparative RP-HPLC (16.2 mg, 2.23 µmol, 2%). Analytical RP-HPLC: t_{R} = 3.81 min (100% A to 100% D in 7.5 min, λ= 214 nm). MS (ESI+): C₂₅₄H₄₉₁N₅₉O₃₉ calc./obs. 4992.83/4992.85 [M+H⁺].

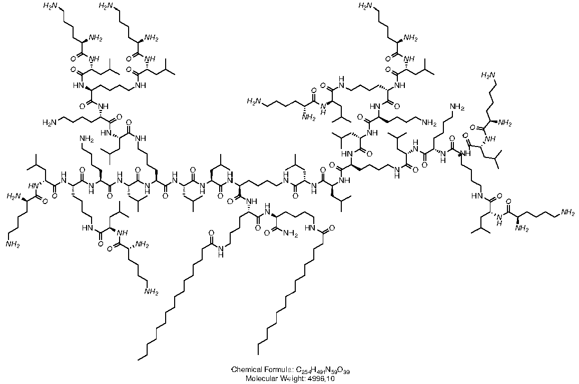

**DMH13 ((kl)₈(*k*kl)₄(*k*ll)₂*k*k(C₁₆)k(C₁₆))** was obtained from the CEM Liberty Blue synthesizer as foamy colourless solid after preparative RP-HPLC (128.4 mg, 17.65 µmol, 7%). Analytical RP-HPLC: t_{R} = 4.67 min (100% A to 100% D in 7.5 min, λ= 214 nm). MS (ESI+): C₂₅₄H₄₉₁N₅₉O₃₉ calc./obs. 4992.83/4992.84 [M+H⁺].

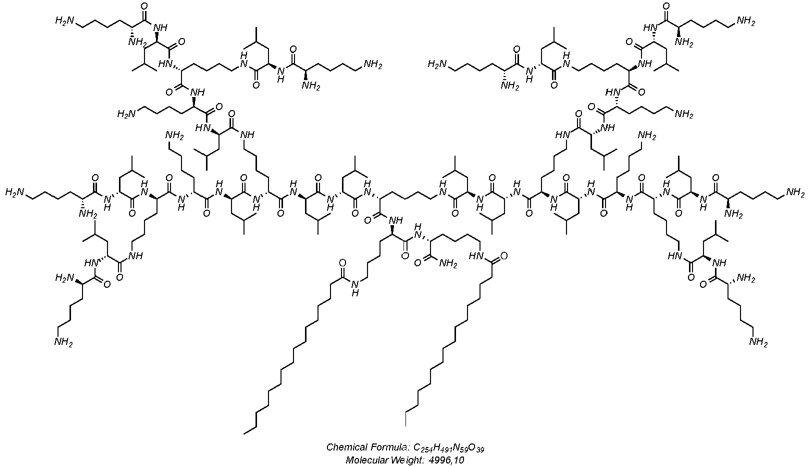

**MH18 ((KL)₃(*K*KL)₄(*K*LL)₂*K*LLLL)** was obtained from the CEM Liberty Blue synthesizer as foamy colourless solid after preparative RP-HPLC (52.7 mg, 7.53 µmol, 8%). Analytical RP-HPLC: t_{R} = 3.29 min (100% A to 100% D in 7.5 min, λ= 214 nm). MS (ESI+): C₂₃₄H₄₅₁N₅₉O₃₉ calc./obs. 4712.51/4712.52 [M+H⁺].

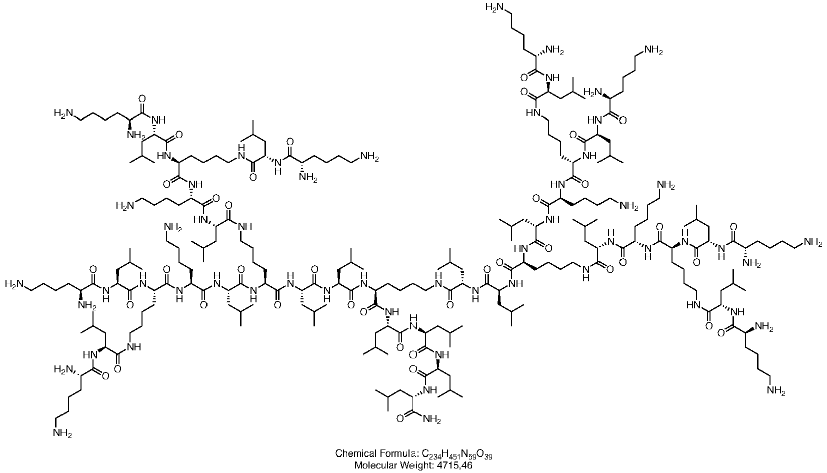

**MH19 ((KL)₈(*K*KL)₄(*K*LL)₂*K*LLLLL)** was obtained after manual synthesis as foamy colourless solid after preparative RP-HPLC (4.7 mg, 0.66 µmol, 1%). Analytical RP-HPLC: t_{R} = 3.36 min (100% A to 100% D in 7.5 min, λ= 214 nm). MS (ESI+): C₂₄₀H₄₆₂N₆₀O₄₀ calc./obs. 4825.60/4825.61 [M+H⁺].

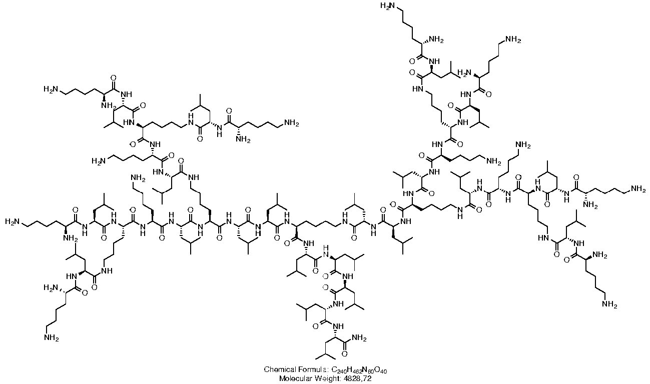

**MH22 ((KL)₈(*K*KL)₄(*K*LL)₂*K*FFF)** was obtained after manual synthesis as foamy colourless solid after preparative RP-HPLC (13.7 mg, 1.96 µmol, 3%). Analytical RP-HPLC: t_{R} = 3.30 min (100% A to 100% D in 7.5 min, λ= 214 nm). MS (ESI+): C₂₃₇H₄₃₄N₅₈O₃₈ calc./obs. 4701.38/4701.40 [M+H⁺].

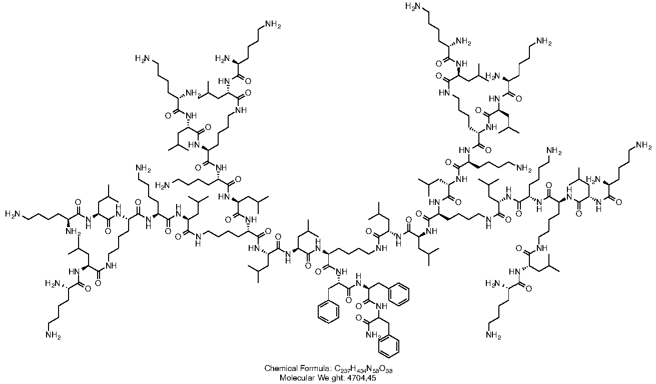

**MH25 ((KL)₈(*K*KL)₄(*K*LL)₂*K*WWWW)** was obtained after manual synthesis as foamy colourless solid after preparative RP-HPLC (4.5 mg, 0.6 µmol, 1%). Analytical RP-HPLC: t_{R} = 3.27 min (100% A to 100% D in 7.5 min, λ= 214 nm). MS (ESI+): C₂₅₄H₄₄₆N₆₂O₄₀ calc./obs. 5004.49/5004.50 [M+H⁺].

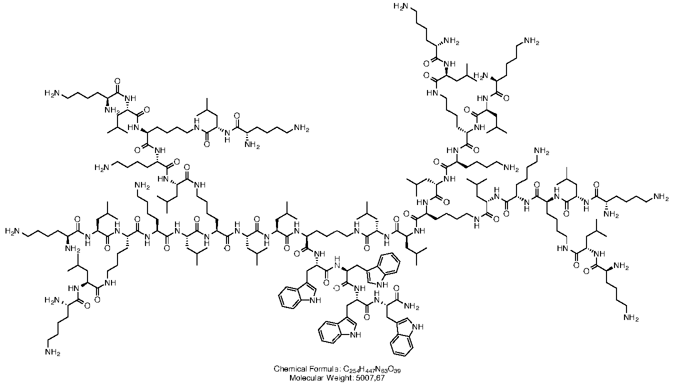

**MH26 ((KL)₈(*K*KL)₄(*K*LL)₂*K*WWWWW)** was obtained after manual synthesis as foamy colourless solid after preparative RP-HPLC (1.7 mg, 0.23 µmol, 1%). Analytical RP-HPLC: t_{R} = 3.33 min (100% A to 100% D in 7.5 min, λ= 214 nm). MS (ESI+): C₂₆₅H₄₅₇N₆₅O₄₀ calc./obs. 5190.57/5190.63 [M+H⁺].

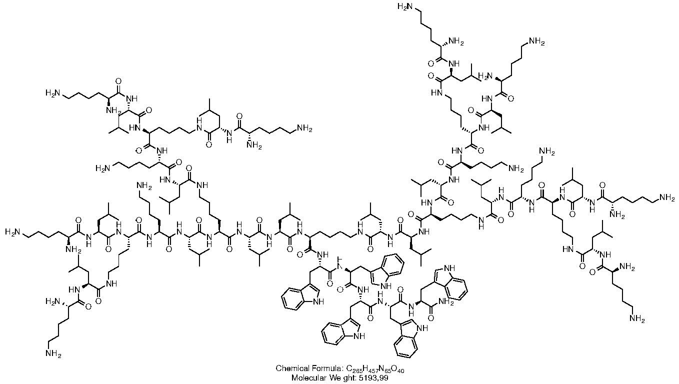

**MH40 ((KL)₈(*K*KL)₄(*K*LL)₂*K*NleNleNleNle)** was obtained after manual synthesis as foamy colourless solid after preparative RP-HPLC (5.1 mg, 0.73 µmol, 1%). Analytical RP-HPLC: t_{R} = 3.34 min (100% A to 100% D in 7.5 min, λ= 214 nm). MS (ESI+): C₂₃₄H₄₅₁N₅₉O₃₉ calc./obs. 4712.51/4712.53 [M+H⁺].

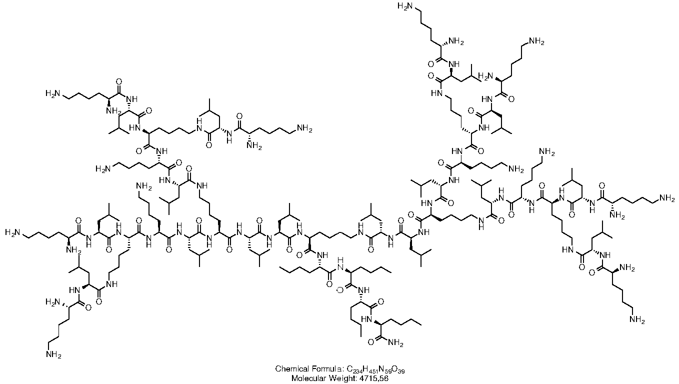

**MH46 ((KL)₈(*K*KL)₄(*K*KL)₂*K*LLLL)** was obtained after manual synthesis as foamy colourless solid after preparative RP-HPLC (8.5 mg, 1.17 µmol, 2%). Analytical RP-HPLC: t_{R} = 3.11 min (100% A to 100% D in 7.5 min, λ= 214 nm). MS (ESI+): C₂₃₄H₄₅₃N₆₁O₃₉ calc./obs. 4742.53/4742.55 [M+H⁺].

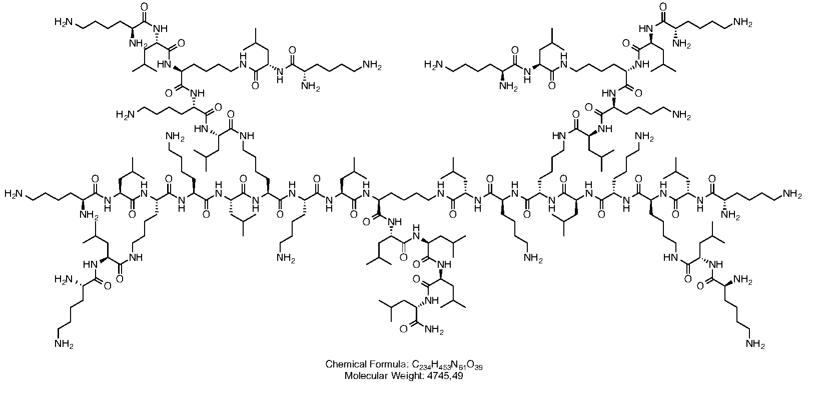

**MH47 ((KL)8(KLL)4(KLL)2KLLLL)** was obtained after manual synthesis as foamy colourless solid after preparative RP-HPLC (10.1 mg, 1.56 µmol, 2%). Analytical RP-HPLC: t_{R} = 4.15 min (100% A to 100% D in 7.5 min, λ= 214 nm). MS (ESI+): C₂₃₄H₄₄₇N₅₅O₃₉ calc./obs. 4652.47/4652.49 [M+H⁺].

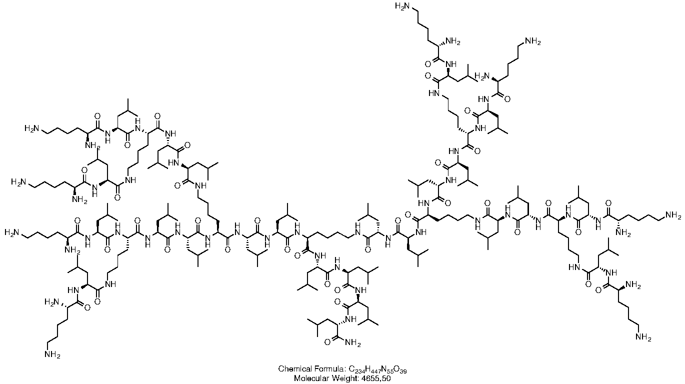

**DMH18 ((kl)₈(*k*kl)₄(*k*ll)₂kllll)** was obtained from the Biotage Initiator+ Alstra synthesizer as foamy colourless solid after preparative RP-HPLC (73.6 mg, 10.5 µmol, 11%). Analytical RP-HPLC: t_{R} = 3.27 min (100% A to 100% D in 7.5 min, λ= 214 nm). MS (ESI+): C₂₃₄H₄₅₁N₅₉O₃₇ calc./obs. 4712.51/4712.52 [M+H⁺].

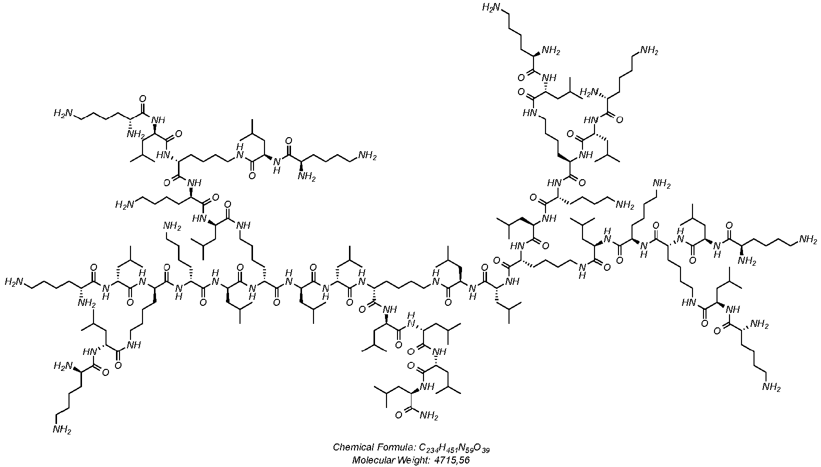

**kl3,2-fff ((kl)₈(*k*kl)₄(*k*ll)₂*k*fff)** was obtained from the CEM Liberty Blue synthesizer as foamy colourless solid after preparative RP-HPLC (22.7 mg, 3.25 µmol, 5 %). Analytical RP-HPLC: t_{R} = 3.27 min (100 % A to 100 % D in 7.5 min, λ= 214 nm). MS (ESI+): C₂₃₇H₄₃₄N₅₈O₃₈ calc/found 4701.38/4701.41 [M+H⁺].

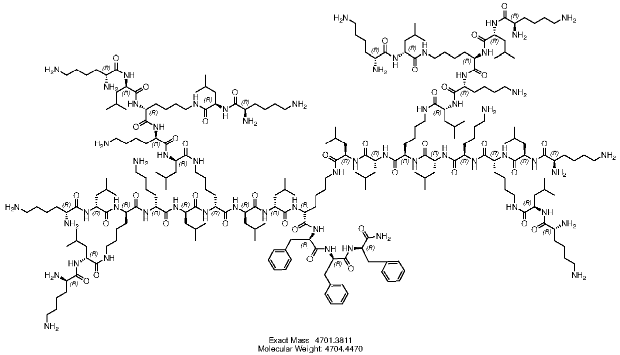

**D4 ((kl)₈(*k*kl)₄(*k*ll)₂*k*lllc)** was obtained from the CEM Liberty Blue synthesizer as foamy colourless solid after preparative RP-HPLC (129.5 mg, 18.5 µmol, 21%). Analytical RP-HPLC: t_{R} = 2.97 min (100% A to 100% D in 7.5 min, λ= 214 nm). MS (ESI+): C₂₃₁H₄₄₅N₅₉O₃₉S calc/found 4702.44/4702.48 [M+H⁺].

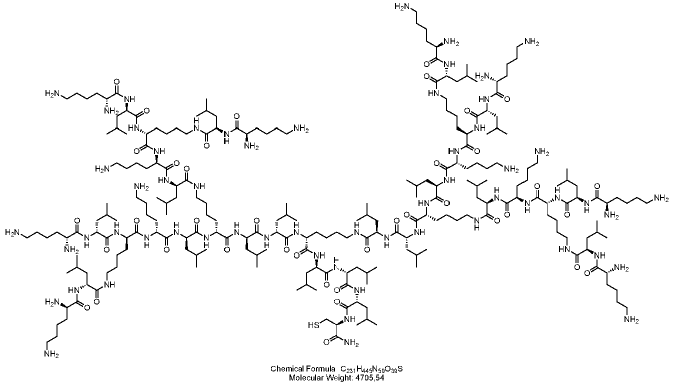

**kl3,2-nlenlenlenle ((kl)₈(*k*kl)₄(*k*ll)₂*k*nlenlenlenle)** was obtained from the CEM Liberty Blue synthesizer as foamy colourless solid after preparative RP-HPLC (19.3 mg, 2.76 µmol, 4 %). Analytical RP-HPLC: t_{R} = 3.31 min (100 % A to 100 % D in 7.5 min, λ= 214 nm). MS (ESI+): C₂₃₄H₄₅₁N₅₉O₃₉ calc/found 4712.51/4712.53 [M+H⁺].

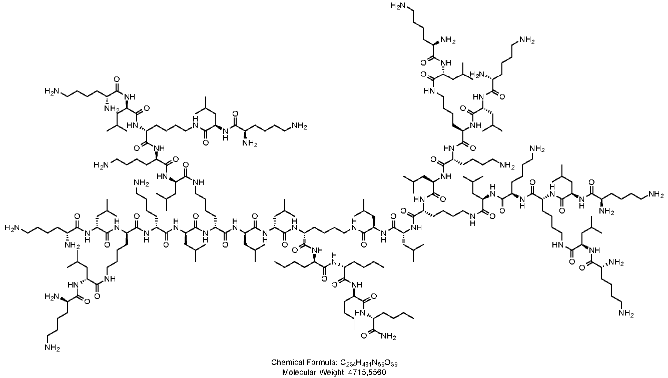

**kl3,2-aocaocaoc ((kl)₈(*k*kl)₄(*k*ll)₂*k*aocaocaoc)** was obtained from the CEM Liberty Blue synthesizer as foamy colourless solid after preparative RP-HPLC (8.4 mg, 1.21 µmol, 2 %). Analytical RP-HPLC: t_{R} = 3.48 min (100 % A to 100 % D in 7.5 min, λ= 214 nm). MS (ESI+): C₂₃₄H₄₅₂N₅₈O₃₈ calc/found 4683.52/4683.52 [M+H⁺].

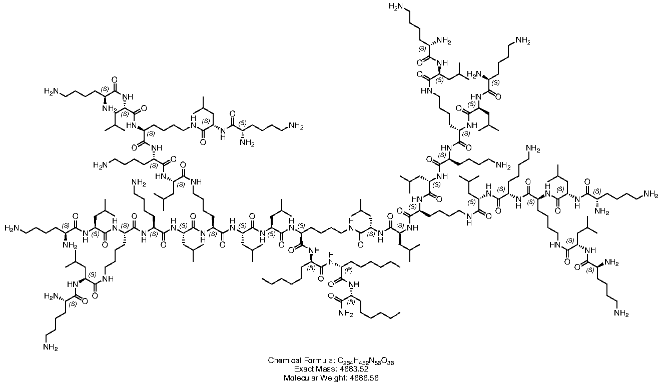

**kl3,2-aocaocaocaoc ((kl)₈(*k*kl)₄(*k*ll)₂kaocaocaocaoc)** was obtained from the CEM Liberty Blue synthesizer as foamy colourless solid after preparative RP-HPLC (13.3 mg, 1.87 µmol, 3 %). Analytical RP-HPLC: t_{R} = 3.56 min (100 % A to 100 % D in 7.5 min, λ= 214 nm). MS (ESI+): C₂₄₂H₄₆₇N₅₉O₃₉ calc/found 4824.64/4824.63 [M+H⁺].

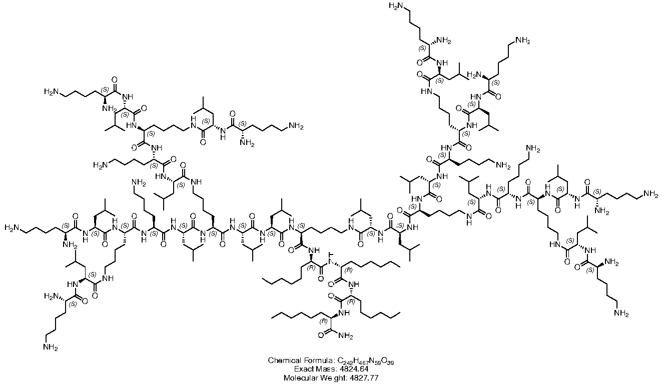

**MH44 ((RL)₈(*K*RL)₄(*K*LL)₂*K*LLLL)** was obtained from the Biotage Initiator+ Alstra synthesizer as foamy colourless solid after preparative RP-HPLC (25.9 mg, 3.53 µmol, 5%). Analytical RP-HPLC: t_{R} = 3.40 min (100% A to 100% D in 7.5 min, λ= 214 nm). MS (ESI+): C₂₃₄H₄₅₀N₈₂O₄₀ calc./obs. 5048.59/5048.61 [M+H⁺].

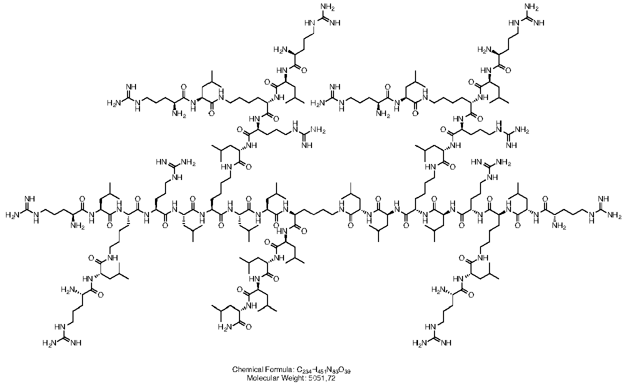

**rl3-kl2-nlenlenlenle ((rl)₈(*k*kl)₄(*k*ll)₂*k*nlenlenlenle)** was obtained from the CEM Liberty Blue synthesizer as foamy colourless solid after preparative RP-HPLC (1.6 mg, 0.22 µmol, 1 %). Analytical RP-HPLC: t_{R} = 3.40 min (100 % A to 100 % D in 7.5 min, λ= 214 nm). MS (ESI+): C₂₃₄H₄₅₁N₇₅O₃₉ calc/found 4936.56/4936.56 [M+H⁺].

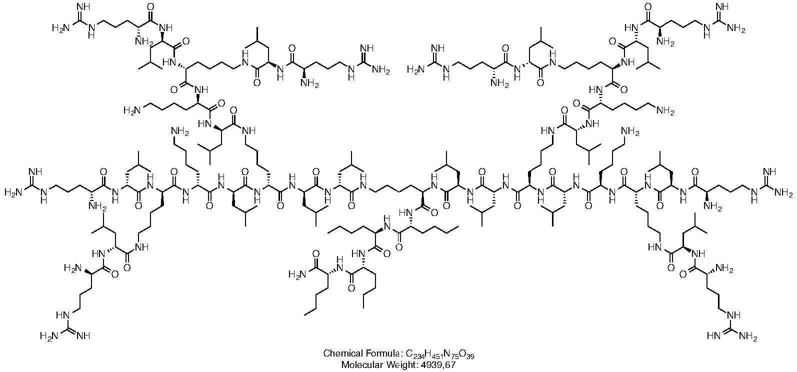

**kl3-rl2-nlenlenlenle ((kl)₈(*k*rl)₄(*k*ll)₂*k*nlenlenlenle)** was obtained from the CEM Liberty Blue synthesizer as foamy colourless solid after preparative RP-HPLC (17.3 mg, 2.43 µmol, 5 %). Analytical RP-HPLC: t_{R} = 3.35 min (100 % A to 100 % D in 7.5 min, λ= 214 nm). MS (ESI+): C₂₃₄H₄₅₁N₅₇O₃₉ calc/found 4824.53/4824.53 [M+H⁺].

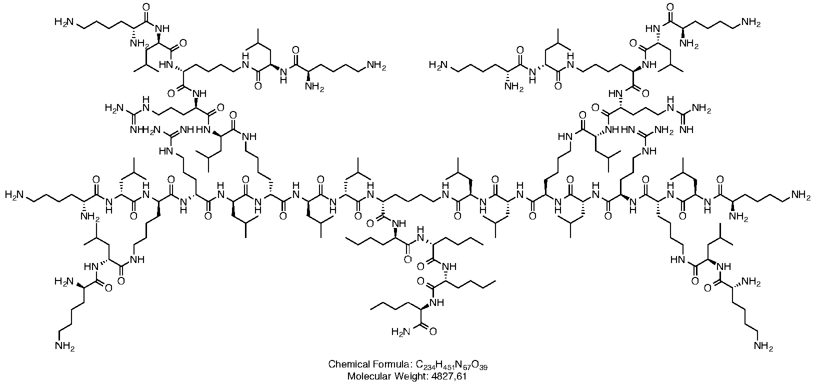

**kl3,2-nlenle1-C₁₆C₁₆ ((kl)₈(*k*kl)₄(*k*nlenle)₂*k*k(C₁₆)k(C₁₆))** was obtained from the CEM Liberty Blue synthesizer as foamy colourless solid after preparative RP-HPLC (12.7 mg, 1.69 µmol, 3 %). Analytical RP-HPLC: t_{R} = 4.47 min (100 % A to 100 % D in 7.5 min, λ= 214 nm). MS (ESI+): C₂₅₄H₄₉₁N₅₉O₃₉ calc/found 4992.8251/4992.8246 [M+H⁺].

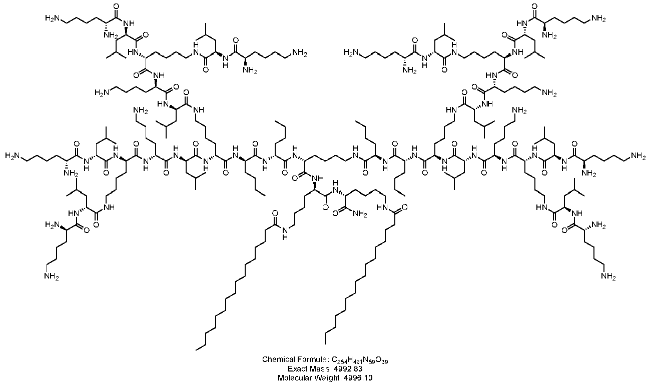

**((KL)ₛ(*K*KL)₄(*K*KL)₂*K*K(C₁₈)K(C₁₈))** was obtained as foamy colorless solid after preparative RP-HPLC (2.5 mg, 0.49 µmol, 1%). Analytical RP-HPLC: t_{R} = 5.06 min (100% A to 100% D in 7.5 min, λ= 214 nm). MS (ESI+): C₂₅₈H₅₀₁N₆₁O₃₉ calc/found 5078.91/5078.90 [M+H⁺].

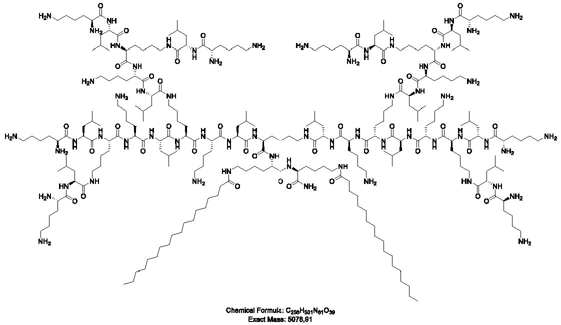

**((KL)₈(*K*KL)₄(*K*KL)₂*K*K(Oleic Acid)C)** was obtained as foamy colorless solid after preparative RP-HPLC (2.3 mg, 0.48 µmol, 2%). Analytical RP-HPLC: t_{R} = 3.70 min (100% A to 100% D in 7.5 min, λ= 214 nm). MS (ESI+): C₂₃₇H₄₅₈N₆₀O₃₈S calc/found 4785.55/4785.56 [M+H⁺].

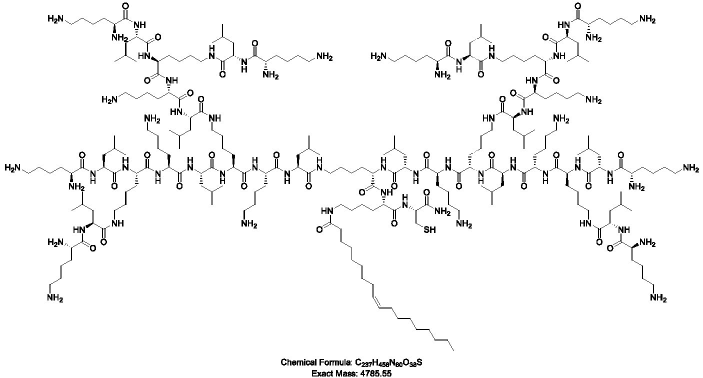

**((KL)₃(*K*KL)₄(*K*KL)₂*K*K(C₁₃)C)** was obtained as foamy colorless solid after preparative RP-HPLC (2.5 mg, 0.52 µmol, 2%). Analytical RP-HPLC: t_{R} = 3.83 min (100% A to 100% D in 7.5 min, λ= 214 nm). MS (ESI+): C₂₃₇H₄₆₀N₆₀O₃₈S calc/found 4787.56/4787.57 [M+H⁺].

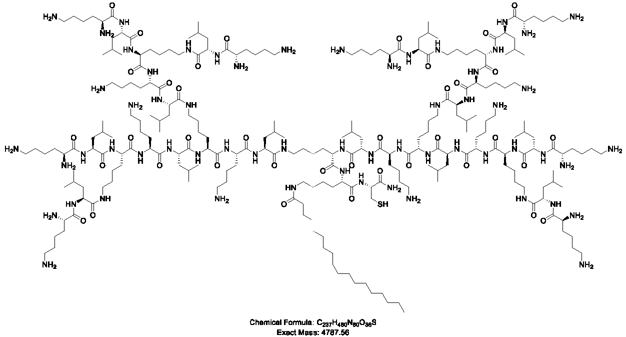

**((KH)₈(*K*KH)₄(*K*KL)₂*K*K(Oleic Acid)K(Oleic Acid))** was obtained as foamy colorless solid after preparative RP-HPLC (2.7 mg, 0.50 µmol, 3%). Analytical RP-HPLC: t_{R} = 4.48 min (100% A to 100% D in 7.5 min, λ= 214 nm). MS (ESI+): C₂₅₈H₄₄₉N₈₅O₃₉ calc/found 5362.58/5362.58 [M+H⁺].

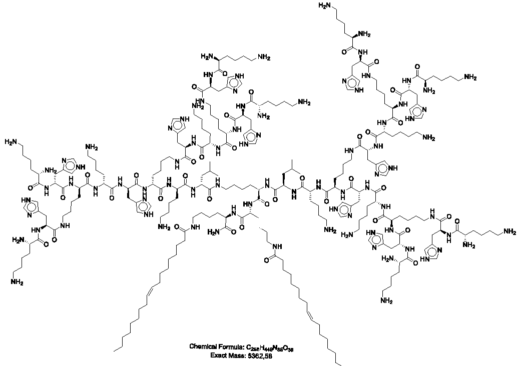

**((KL)₈(*K*KL)₄(*K*LL)₂*K*K(C₂₂))** was obtained as foamy colorless solid after preparative RP-HPLC (11.4 mg, 0.24 µmol, 8%). Analytical RP-HPLC: t_{R} = 4.18 min (100% A to 100% D in 7.5 min, λ= 214 nm). MS (ESI+): C₂₃₈H₄₆₃N₅₉O₃₇ calc/found 4740.62/4740.63 [M+H⁺].

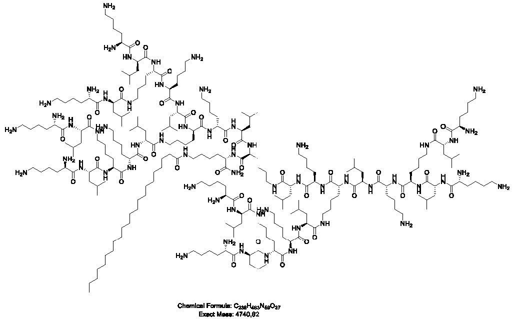

**((KL)_{S}(*K*KL)₄(*K*KL)₂*K*K(C₁₆)K(C₁₆))** was obtained as foamy colourless solid after preparative RP-HPLC (39.1 mg, 5.18 µmol, 8 %). Analytical RP-HPLC: t_{R} = 4.35 min (100 % A to 100 % D in 7.5 min, λ= 214 nm). MS (ESI+): C₂₅₄H₄₉₃N₆₁O₃₉ calc/found 5022.85/5022.86 [M+H⁺].

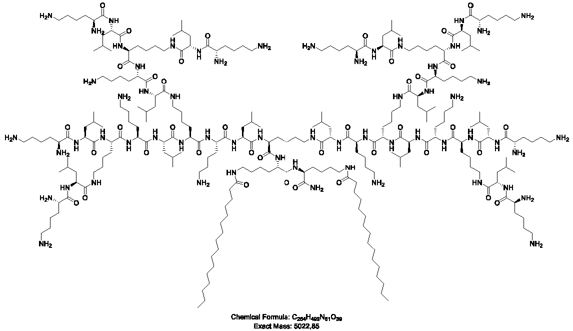

**((kl)₈(*k*kl)₄(*k*kl)₂*k*k(C₁₆)*k*(C₁₆))** was obtained as foamy colourless solid after preparative RP-HPLC (24.9 mg, 3.3 µmol, 5 %). Analytical RP-HPLC: t_{R} = 4.45 min (100 % A to 100 % D in 7.5 min, λ= 214 nm). MS (ESI+): C₂₅₄H₄₉₃N₅₁O₃₉ calc/found 5022.85/5022.88 [M+H⁺].

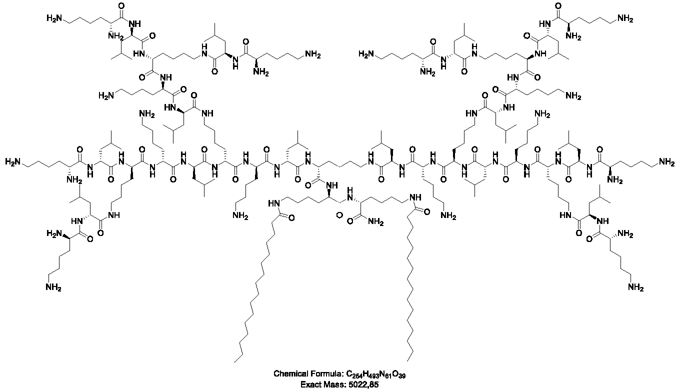

**((kl)₈(*k*kl)₄(*k*kl)₂*k*k(C₁₆)c)** was obtained as foamy green solid after preparative RP-HPLC (6.04 mg, 0.13 µmol, 3%). Analytical RP-HPLC: t_{R} = 3.65 min (100% A to 100% D in 7.5 min, λ= 214 nm). MS (ESI+): C₂₃₅H₄₅₆N₆₀O₃₈S calc/found 4759.53/4759.54 [M+H⁺].

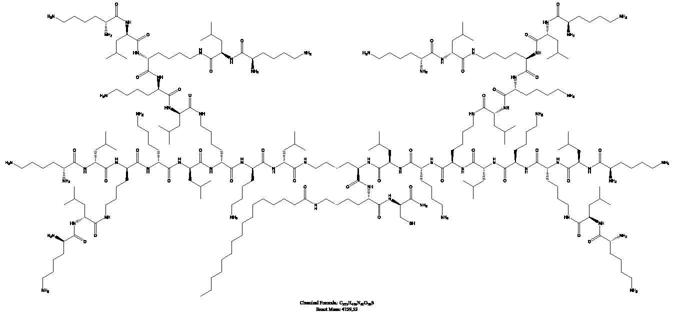

**((kl)₈(*k*kl)₄(*k*kl)₂*k*k(C₁₈)c)** was obtained as foamy colorless solid after preparative RP-HPLC (3.1 mg, 0.65 µmol, 3%). Analytical RP-HPLC: t_{R} = 3.73 min (100% A to 100% D in 7.5 min, λ= 214 nm). MS (ESI+): C₂₃₇H₄₆₀N₆₀O₃₈S calc/found 4787.56/4787.57 [M+H⁺].

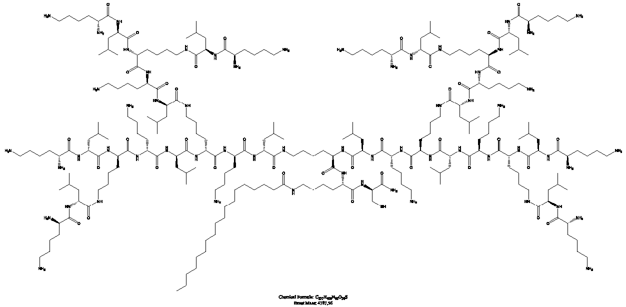

**((kl)₈(*k*kl)₄(*k*kl)₂*k*k(Oleic Acid)c)** was obtained as foamy solid after preparative RP-HPLC (5.85 mg, 0.12 µmol, 4%). Analytical RP-HPLC: t_{R} = 3.41 min (100% A to 100% D in 7.5 min, λ= 214 nm). MS (ESI+): C₂₃₇H₄₅₈N₆₀O₃₈S calc/found 4786.00/4785.55 [M+H⁺].

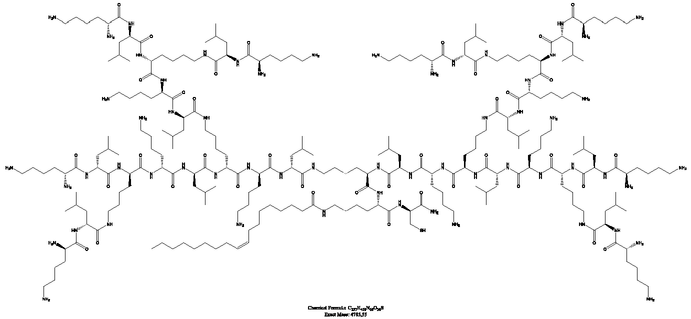

**((kl)₈(*k*kl)₄(*k*kl)₂*k*k(C18)a)** was obtained as foamy green solid after preparative RP-HPLC (114 mg, 24 µmol, 18%). Analytical RP-HPLC: t_{R} = 3.66 min (100% A to 100% D in 7.5 min, λ= 214 nm). MS (ESI+): C₂₃₇H₄₆₀N₆₀O₃₈ calc/found 4755.59/4755.59.

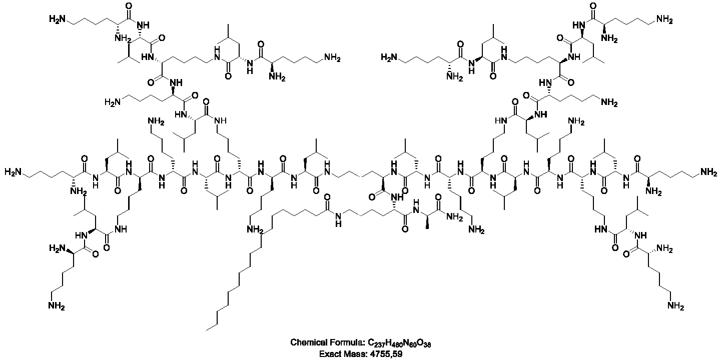

**((kl)₈(*k*kl)₄(*k*kl)₂*k*k(C18))** was obtained as foamy green solid after preparative RP-HPLC (20 mg, 4.1 µmol, 23%). Analytical RP-HPLC: t_{R} = 3.76 min (100% A to 100% D in 7.5 min, λ= 214 nm). MS (ESI+): C₂₃₄H₄₅₅N₅₉O₃₇ calc/found 4684.55/4684.56.

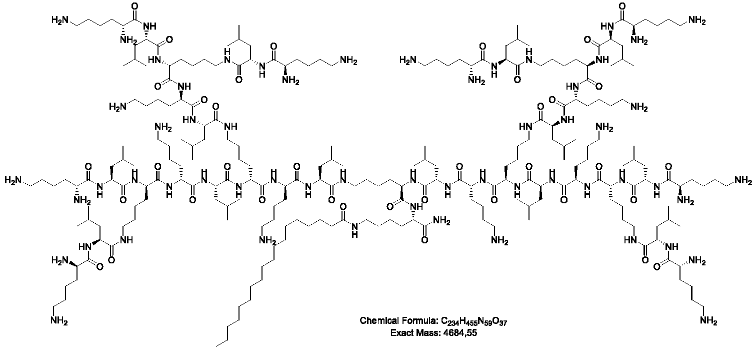

**((kl)₈(*k*kl)₄(*k*kl)₂*k*k(C16))** was obtained as foamy green solid after preparative RP-HPLC (22 mg, 4.6 µmol, 16%). Analytical RP-HPLC: t_{R} = 3.60 min (100% A to 100% D in 7.5 min, λ= 214 nm). MS (ESI+): C₂₃₂H₄₅₁N₅₉O₃₇ calc/found 4656.52/4656.52.

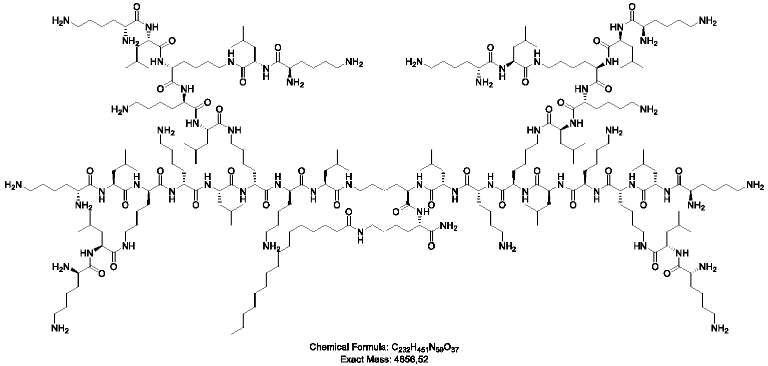

**((rl)₈(*k*rl)₄(*k*rl)₂*k*k(C18))** was obtained as foamy green solid after preparative RP-HPLC (13 mg, 2.5 µmol, 15%). Analytical RP-HPLC: t_{R} = 3.90 min (100% A to 100% D in 7.5 min, λ= 214 nm). MS (ESI+): C₂₃₄H₄₅₅N₃₇O₃₇ calc/found 5076.64/5076.63.

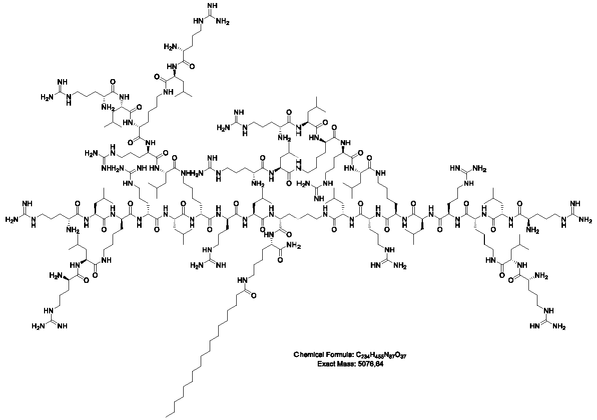

**((rl)₈(*k*rl)₄(*k*rl)₂*k*k(C16))** was obtained as foamy green solid after preparative RP-HPLC (14 mg, 2.7 µmol, 11%). Analytical RP-HPLC: t_{R} = 3.69 min (100% A to 100% D in 7.5 min, λ= 214 nm). MS (ESI+): C₂₃₂H₄₅₁N₈₇O₃₇ calc/found 5048.61/5048.60.

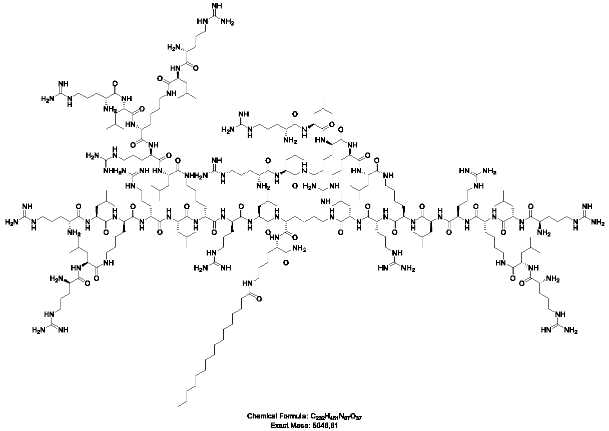

**((rl)₈(*k*rl)₄(*k*rl)₂*k*k(C₁₈)c)** was obtained after manual synthesis as foamy colorless solid after preparative RP-HPLC (26.6 mg, 5.1 µmol, 14%). Analytical RP-HPLC: t_{R}= 3.99 min (100% A to 100% D in 7.5 min, λ= 214 nm). MS (ESI+):C₂₃₇H₄₆₀N₈₈O₃₈S calc./obs. 5179.65/5179.66.

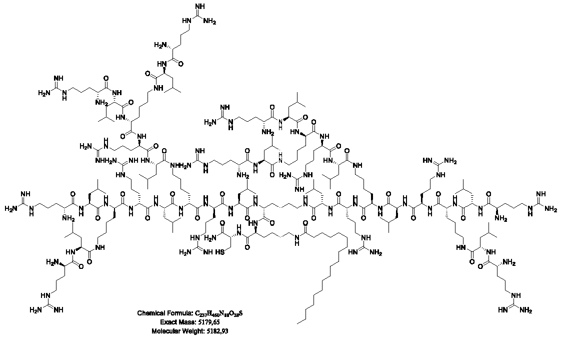

**((rl)₈(*k*rl)₄(*k*rl)₂*k*k(C₁₆)c)** was obtained after manual synthesis as foamy colorless solid after preparative RP-HPLC (9 mg, 1.6 µmol, 4%). Analytical RP-HPLC: t_{R}= 3.77 min (100% A to 100% D in 7.5 min, λ= 214 nm). MS (ESI+):C₂₃₅H₄₅₆N₈₈O₃₈S calc./obs. 5151.62/5151.62

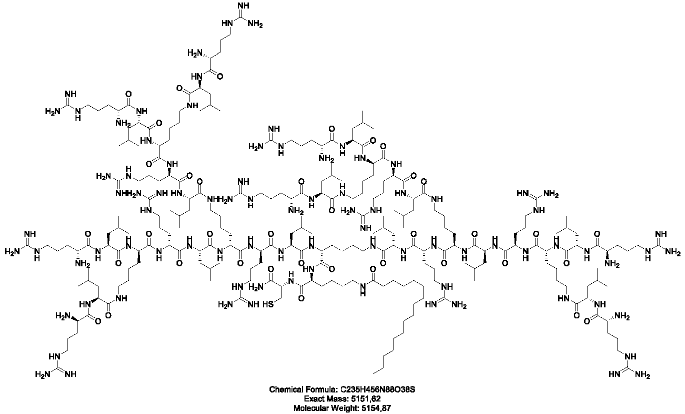

**((rl)₈(*k*rl)₄(*k*rl)₂*k*k(C18)a)** was obtained as foamy green solid after preparative RP-HPLC (25 mg, 4.9 µmol, 12%). Analytical RP-HPLC: t_{R} = 3.90 min (100% A to 100% D in 7.5 min, λ= 214 nm). MS (ESI+): C₂₃₇H₄₆₀N₈₈O₃₈ calc/found 5147.68/5147.68.

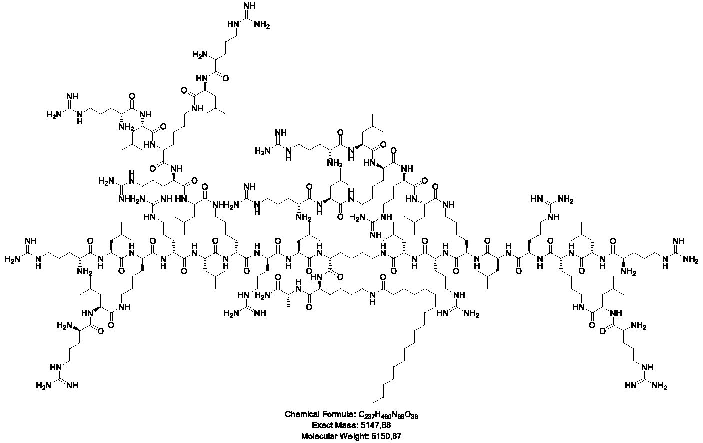

## Claims

1. A peptide dendrimer of formula 1,
(D³)₈-(B³-D²)₄-(B²-D¹)₂-B¹-Z (1)
with Z being -X(Y¹) (1a), -X(Y²)X(Y²) (1b), -X(Y³)Cys (1c), -HP (1d), or -X(Y⁴)Ala, in particular with Z being -X(Y¹) (1a), -X(Y²)X(Y²) (1b), -X(Y³)Cys (1c) or -HP (1d),
wherein,
- X is selected from Lys, Orn, DAB, DAP, Glu or Asp, which is coupled to B¹ via its N-terminus and which is coupled to Y via its side chain,
- Y¹ is selected from a -C(=O)-Cᵥ-alkyl, a -C(=O)-Cᵥ-alkenyl in case of X being Lys, Orn, DAB or DAP, and v is
- between 15 and 27, particularly between 17 and 27, more particularly between 19 and 27, even more particularly between 21 and 25, in case of X being Lys,
- between 18 and 28, particularly between 20 and 28, more particularly between 22 and 26, in case of X being Orn,
- between 19 and 29, particularly between 21 and 29, more particularly between 23 and 27, in case of X being DAB,
- between 20 and 30, particularly between 22 and 30, more particularly between 24 and 28, in case of X being DAP, or
Y¹ is selected from a -(NH)-Cₐ-alkyl, a -(NH)-Cₐ-alkenyl, -(N)-(C_{d}-alkyl)₂ or a -(N)-(C_{d}-alkenyl)₂ in case of X being Glu or Asp, wherein
- the sum of d is between 19 and 29, particularly between 21 and 29, more particularly between 23 and 27 in case of X being Glu,
- the sum of d is between 20 and 30, particularly between 22 and 30, more particularly between 24 and 28, in case of X being Asp, and wherein
- a is between 19 and 29 particularly between 21 and 29, more particularly between 23 and 27, in case of X being Glu, or
- a is between 20 and 30, particularly between 22 and 30, more particularly between 24 and 28, in case of X being Asp,
- each Y² is independently selected from a -C(=O)-C_{w}-alkyl and a -C(=O)-C_{w}-alkenyl, in case of X being Lys, Orn, DAB or DAP and the sum of w is
- between 18 and 36, particularly between 22 and 36, more particularly between 28 and 36, in case of X being Lys,
- between 20 and 38, particularly between 24 and 38, more particularly between 30 and 38, in case of X being Orn,
- between 22 and 40, particularly between 26 and 40, more particularly between 32 and 40, in case of X being DAB,
- between 24 and 42, particularly between 28 and 42, more particularly between 34 and 42, in case of X being DAP, or
Y² is selected from a -(NH)-C_{b}-alkyl, a -(NH)-C_{b}-alkenyl, -(N)-(Cₑ-alkyl)₂ or a -(N)-(Cₑ-alkenyl)₂ in case of X being Glu or Asp, wherein
the sum of e or b is between 22 and 40, particularly between 26 and 40, more particularly between 32 and 40 in case of X being Glu, wherein
the sum of e or b is between 24 and 42, particularly between 28 and 42, more particularly between 34 and 42, in case of X being Asp,
- Y³ and Y⁴ are independently selected from a -C(=O)-Cₓ-alkyl and a -C(=O)-Cₓ-alkenyl, in case of X being Lys, Orn, DAB or DAP and x is
- between 15 and 21, particularly between 15 and 17, in case of X being Lys,
- between 16 and 22, particularly between 16 and 18, in case of X being Orn
- between 17 and 23, particularly between 17 and 19, in case of X being DAB between 18 and 24, particularly between 18 and 20, in case of X being DAPY³ is selected from a -(NH)-C_{c}-alkyl, a -(NH)-C_{c}-alkenyl, -(N)-(C_{f}-alkyl)₂ or a -(N)-(C_{f}-alkenyl)₂ in case of X being Glu or Asp, wherein
- the sum of f is between 17 and 23, particularly between 17 and 19, in case of X being Glu,
- the sum of f is between 18 and 24, particularly between 18 and 20, in case of X being Asp,
- c is between 17 and 23, particularly between 17 and 19, in case of X being Glu,
- c is between 18 and 24, particularly between 18 and 20, in case of X being Asp,
- HP is a hydrophobic peptide consisting of 3 to 5 hydrophobic amino acids,
- each B is Lys
- each D independently from any other D is a dipeptide consisting of one hydrophobic amino acid and one cationic amino acid (HC or CH), a dipeptide consisting of two cationic amino acids (CC) or a dipeptide consisting of two hydrophobic amino acids (HH).

2. The peptide dendrimer according to claim 1, wherein X is Lys.

3. The peptide dendrimer according to any one of the preceding claims, wherein the hydrophobic peptide consists of hydrophobic amino acids independently selected from Cys, Leu, Trp, Phe, Nle (norleucine), Aoc (amino octanoic acid), particularly from Leu, Phe, Trp, Nle, Aoc and Cys, more particularly from Leu, Phe, Trp, Nle and Aoc.

4. The peptide dendrimer according to any one of the preceding claims, wherein the hydrophobic peptide consists of identical hydrophobic amino acids.

5. The peptide dendrimer according to any one of the preceding claims, wherein the C-terminus of the peptide dendrimer is a carboxamide.

6. The peptide dendrimer according to any one of the preceding claims, wherein in case of HH, CH and HC
a. the hydrophobic amino acid in D¹ is selected from Leu and Nle, and the cationic amino acid in D¹ is Lys.
b. the hydrophobic amino acid in D² is Leu, and
the cationic amino acid in D² is selected from Lys, Arg and His.
c. the hydrophobic amino acid in D³ is Leu, and
the cationic amino acid in D³ is selected from Lys, Arg and His and in case of CC
the cationic amino acid in D² or D³ is selected from Lys, Arg and His, wherein at least one C is His.

7. The peptide dendrimer according to any one of the preceding claims, wherein
- D¹ is a dipeptide consisting of two hydrophobic amino acids (HH) and D² and D³ are each a dipeptide consisting of one cationic amino acid and one hydrophobic amino acid (CH), or
- each D¹, D² and D³ are a dipeptide consisting of one cationic amino acid and one hydrophobic amino acid (CH).

8. The peptide dendrimer according to any one of the preceding claims, wherein the amino acids are independently from each other selected from (L)-amino acids and (D)-amino acids.

9. The peptide dendrimer according to any one of the preceding claims, wherein the peptide is homochiral.

10. A method for transfecting a cell *ex vivo* comprising the steps of
a. providing a transfection mix comprising a peptide dendrimer according to any one of the preceding claims and a nucleic acid,
b. contacting a cell with the transfection mix yielding a transfected cell.

11. The method according to claim 10, wherein the transfection mix is prepared by mixing a first solution comprising the peptide dendrimer according to any one of the preceding claims and a second solution comprising the nucleic acid.

12. The method according to any one of claims 10 to 11, wherein the nucleic acid is DNA or RNA, particularly circular DNA (plasmid/vector), linear DNA (cDNA), linear RNA (siRNA, saRNA, miRNA, mRNA, long RNA).

13. The method according to any one of claims 10 to 12, wherein the solvent of the first and/or the second solution is water or a cell culture medium.

14. The method according to any one of claims 10 to 13, wherein the final concentration of the nucleic acid in the transfection mix is between 10 nM to 200 nM, particularly 20-100 nM, more particularly 40-80 nM.

15. The method according to any one of claims 10 to 14, wherein the final concentration of the peptide dendrimer in the transfection mix is between 1.5 µg/ml and 30 µg/ml, particularly between 3 µg/ml and 15 µg/ml, more particularly between 6 µg/ml and 12 µg/ml.

## Patentansprüche

1. Peptid-Dendrimer der Formel 1,
(D³)₈-(B³-D²)₄-(B²-D¹)₂-B¹-Z (1),
wobei Z -X(Y¹) (1a), -X(Y²)X(Y²) (1b), -X(Y³)Cys (1c), -HP (1d) oder -X(Y⁴)Ala ist, insbesondere wobei Z -X(Y¹) (1a), -X(Y²)X(Y²) (1b), -X(Y³)Cys (1c) oder -HP (1d) ist, wobei
- X ausgewählt ist aus Lys, Orn, DAB, DAP, Glu oder Asp, welche(s) über seinen/ihren N-Terminus mit B¹ verbunden ist und welche(s) über seine/ihre Seitenkette mit Y verbunden ist,
- Y¹ ausgewählt ist aus einem -C(=O)-Cᵥ-Alkyl, einem -C(=O)-Cᵥ-Alkenyl, im Falle, dass X Lys, Orn, DAB oder DAP ist, und v beträgt:
- zwischen 15 und 27, insbesondere zwischen 17 und 27, spezieller zwischen 19 und 27, noch spezieller zwischen 21 und 25, im Falle, dass X Lys ist,
- zwischen 18 und 28, insbesondere zwischen 20 und 28, spezieller zwischen 22 und 26, im Falle, dass X Orn ist,
- zwischen 19 und 29, insbesondere zwischen 21 und 29, spezieller zwischen 23 und 27, im Falle, dass X DAB ist,
- zwischen 20 und 30, insbesondere zwischen 22 und 30, spezieller zwischen 24 und 28, im Falle, dass X DAP ist, oder
Y¹ ausgewählt ist aus einem -(NH)-Cₐ-Alkyl, einem -(NH)-Cₐ-Alkenyl, einem -(N)-(C_{d}-Alkyl)₂ oder einem -(N)-(C_{d}-Alkenyl)₂, im Falle, dass X Glu oder Asp ist, wobei
- die Summe von d zwischen 19 und 29, insbesondere zwischen 21 und 29, spezieller zwischen 23 und 27 beträgt, im Falle, dass X Glu ist,
- die Summe von d zwischen 20 und 30, insbesondere zwischen 22 und 30, spezieller zwischen 24 und 28 beträgt, im Falle, dass X Asp ist, und wobei
- a zwischen 19 und 29, insbesondere zwischen 21 und 29, spezieller zwischen 23 und 27 beträgt, im Falle, dass X Glu ist, oder
- a zwischen 20 und 30, insbesondere zwischen 22 und 30, spezieller zwischen 24 und 28 beträgt, im Falle, dass X Asp ist,
- jedes Y² unabhängig ausgewählt ist aus einem -C(=O)-C_{w}-Alkyl und einem -C(=O)-C_{w}-Alkenyl, im Falle, dass X Lys, Orn, DAB oder DAP ist, und die Summe von w beträgt:
- zwischen 18 und 36, insbesondere zwischen 22 und 36, spezieller zwischen 28 und 36, im Falle, dass X Lys ist,
- zwischen 20 und 38, insbesondere zwischen 24 und 38, spezieller zwischen 30 und 38, im Falle, dass X Orn ist,
- zwischen 22 und 40, insbesondere zwischen 26 und 40, spezieller zwischen 32 und 40, im Falle, dass X DAB ist,
- zwischen 24 und 42, insbesondere zwischen 28 und 42, spezieller zwischen 34 und 42, im Falle, dass X DAP ist, oder
Y² ausgewählt ist aus einem -(NH)-C_{b}-Alkyl, einem -(NH)-C_{b}-Alkenyl, einem -(N)-(Cₑ-Alkyl)₂ oder einem -(N)-(Cₑ-Alkenyl)₂, im Falle, dass X Glu oder Asp ist, wobei
die Summe von e, oder b zwischen 22 und 40, insbesondere zwischen 26 und 40, spezieller zwischen 32 und 40 beträgt, im Falle, dass X Glu ist, wobei
die Summe von e, oder b zwischen 24 und 42, insbesondere zwischen 28 und 42, spezieller zwischen 34 und 42 beträgt, im Falle, dass X Asp ist,
- Y³ und Y⁴ unabhängig voneinander ausgewählt sind aus einem -C(=O)-Cₓ-Alkyl und einem -C(=O)-Cₓ-Alkenyl, im Falle, dass X Lys, Orn, DAB oder DAP ist, und x beträgt:
- zwischen 15 und 21, insbesondere zwischen 15 und 17, im Falle, dass X Lys ist,
- zwischen 16 und 22, insbesondere zwischen 16 und 18, im Falle, dass X Orn ist,
- zwischen 17 und 23, insbesondere zwischen 17 und 19, im Falle, dass X DAB ist,
- zwischen 18 und 24, insbesondere zwischen 18 und 20, im Falle, dass X DAP ist, Y³ ausgewählt ist aus einem -(NH)-C_{c}-Alkyl, einem -(NH)-C_{c}-Alkenyl, einem -(N)-(C_{f}-Alkyl)₂ oder einem -(N)-(C_{f}-Alkenyl)₂, im Falle, dass X Glu oder Asp ist, wobei
- die Summe von f zwischen 17 und 23, insbesondere zwischen 17 und 19 beträgt, im Falle, dass X Glu ist,
- die Summe von f zwischen 18 und 24, insbesondere zwischen 18 und 20 beträgt, im Falle, dass X Asp ist,
- c zwischen 17 und 23, insbesondere zwischen 17 und 19 beträgt, im Falle, dass X Glu ist,
- c zwischen 18 und 24, insbesondere zwischen 18 und 20 beträgt, im Falle, dass X Asp ist,
- HP ein aus 3 bis 5 hydrophoben Aminosäuren bestehendes hydrophobes Peptid ist,
- jedes B Lys ist,
- jedes D unabhängig von einem anderen D ein aus einer hydrophoben Aminosäure und einer kationischen Aminosäure bestehendes Dipeptid (HC oder CH), ein aus zwei kationischen Aminosäuren bestehendes Dipeptid (CC) oder ein aus zwei hydrophoben Aminosäuren bestehendes Dipeptid (HH) ist.

2. Peptid-Dendrimer nach Anspruch 1, wobei X Lys ist.

3. Peptid-Dendrimer nach einem der vorangehenden Ansprüche, wobei das hydrophobe Peptid aus hydrophoben Aminosäuren besteht, die unabhängig voneinander ausgewählt sind aus Cys, Leu, Trp, Phe, Nle (Norleucin), Aoc (Aminooctansäure), insbesondere aus Leu, Phe, Trp, Nie, Aoc und Cys, spezieller aus Leu, Phe, Trp, Nle und Aoc.

4. Peptid-Dendrimer nach einem der vorangehenden Ansprüche, wobei das hydrophobe Peptid aus identischen hydrophoben Aminosäuren besteht.

5. Peptid-Dendrimer nach einem der vorangehenden Ansprüche, wobei der C-Terminus des Peptid-Dendrimers ein Carboxamid ist.

6. Peptid-Dendrimer nach einem der vorangehenden Ansprüche, wobei im Falle von HH, CH and HC
a. die hydrophobe Aminosäure in D¹ aus Leu und Nle ausgewählt ist, und
die kationische Aminosäure in D¹ Lys ist,
b. die hydrophobe Aminosäure in D² Leu ist, und
die kationische Aminosäure in D² aus Lys, Arg und His ausgewählt ist,
c. die hydrophobe Aminosäure in D³ Leu ist, und
die kationische Aminosäure in D³ aus Lys, Arg und His ausgewählt ist, und im Falle von CC
die kationische Aminosäure in D² oder D³ aus Lys, Arg und His ausgewählt ist, wobei mindestens ein C His ist.

7. Peptid-Dendrimer nach einem der vorangehenden Ansprüche, wobei
- D¹ ein aus zwei hydrophoben Aminosäuren bestehendes Dipeptid (HH) ist und D² und D³ jeweils ein aus einer kationischen Aminosäure und einer hydrophoben Aminosäure bestehendes Dipeptid (CH) sind, oder
- jedes D¹, D² und D³ ein aus einer kationischen Aminosäure und einer hydrophoben Aminosäure bestehendes Dipeptid (CH) ist.

8. Peptid-Dendrimer nach einem der vorangehenden Ansprüche, wobei die Aminosäuren unabhängig voneinander aus L-Aminosäuren und D-Aminosäuren ausgewählt sind.

9. Peptid-Dendrimer nach einem der vorangehenden Ansprüche, wobei das Peptid homochiral ist.

10. Verfahren zum Transfizieren einer Zelle *ex vivo,* umfassend die Schritte
a. Bereitstellen eines Transfektionsgemisches, umfassend ein Peptid-Dendrimer nach einem der vorangehenden Ansprüche und eine Nukleinsäure,
b. Inkontaktbringen einer Zelle mit dem Transfektionsgemisch unter Hervorbringen einer transfizierten Zelle.

11. Verfahren nach Anspruch 10, wobei das Transfektionsgemisch durch Mischen einer ersten Lösung, die das Peptid-Dendrimer nach einem der vorangehenden Ansprüche umfasst, und einer zweiten Lösung, die die Nukleinsäure umfasst, hergestellt wird.

12. Verfahren nach einem der Ansprüche 10 bis 11, wobei die Nukleinsäure DNA oder RNA, insbesondere ringförmige DNA (Plasmid/Vektor), lineare DNA (cDNA), lineare RNA (siRNA, saRNA, miRNA, mRNA, lange RNA), ist.

13. Verfahren nach einem der Ansprüche 10 bis 12, wobei das Lösungsmittel der ersten und/oder der zweiten Lösung Wasser oder ein Zellkulturmedium ist.

14. Verfahren nach einem der Ansprüche 10 bis 13, wobei die Endkonzentration der Nukleinsäure in dem Transfektionsgemisch zwischen 10 nM bis 200 nM, insbesondere 20-100 nM, spezieller 40-80 nM beträgt.

15. Verfahren nach einem der Ansprüche 10 bis 14, wobei die Endkonzentration des Peptid-Dendrimers in dem Transfektionsgemisch zwischen 1,5 µg/ml und 30 µg/ml, insbesondere zwischen 3 µg/ml und 15 µg/ml, spezieller zwischen 6 µg/ml und 12 µg/ml beträgt.

## Revendications

1. Dendrimère peptidique de formule 1,
(D³)₈-(B³-D²)₄-(B²-D¹)₂-B¹-Z (1)
avec Z qui est -X(Y¹) (1a), -X(Y²)X(Y²) (1b), -X(Y³)Cys (1c), -HP (1d) ou -X(Y⁴)Ala, en particulier avec Z qui est -X(Y¹) (1a), -X(Y²)X(Y²) (1b), -X(Y³)Cys (1c) ou -HP (1d),
dans lesquelles
- X est sélectionné parmi Lys, Orn, DAB, DAP, Glu ou Asp, qui est couplé à B¹ par le biais de sa terminaison N et qui est couplé à Y par le biais de sa chaîne latérale,
- Y¹ est sélectionné parmi un -C(=O)-Cᵥ-alkyle, un -C(=O)-Cᵥ-alkényle dans le cas de X qui est Lys, Orn, DAB ou DAP, et v est
- entre 15 et 27, en particulier entre 17 et 27, plus particulièrement entre 19 et 27, encore plus particulièrement entre 21 et 25, dans le cas de X qui est Lys,
- entre 18 et 28, en particulier entre 20 et 28, plus particulièrement entre 22 et 26, dans le cas de X qui est Orn,
- entre 19 et 29, en particulier entre 21 et 29, plus particulièrement entre 23 et 27, dans le cas de X qui est DAB,
- entre 20 et 30, en particulier entre 22 et 30, plus particulièrement entre 24 et 28, dans le cas de X qui est DAP, ou
Y¹ est sélectionné parmi un -(NH)-Cₐ-alkyle, un -(NH)-Cₐ-alkényle, -(N)-(C_{d}-alkyle)₂ ou un -(N)-(C_{d}-alkényle)₂ dans le cas de X qui est Glu ou Asp, dans lesquelles
- la somme de d est entre 19 et 29, en particulier entre 21 et 29, plus particulièrement entre 23 et 27 dans le cas de X qui est Glu,
- la somme de d est entre 20 et 30, en particulier entre 22 et 30, plus particulièrement entre 24 et 28, dans le cas de X qui est Asp, et dans lesquelles
- a est entre 19 et 29, en particulier entre 21 et 29, plus particulièrement entre 23 et 27, dans le cas de X qui est Glu, ou
- a est entre 20 et 30, en particulier entre 22 et 30, plus particulièrement entre 24 et 28, dans le cas de X qui est Asp,
- chaque Y² est indépendamment sélectionné parmi un -C(=O)-C_{w}-alkyle et un - C(=O)-C_{w}-alkényle, dans le cas de X qui est Lys, Orn, DAB ou DAP, et la somme de w est
- entre 18 et 36, en particulier entre 22 et 36, plus particulièrement entre 28 et 36, dans le cas de X qui est Lys,
- entre 20 et 38, en particulier entre 24 et 38, plus particulièrement entre 30 et 38, dans le cas de X qui est Orn,
- entre 22 et 40, en particulier entre 26 et 40, plus particulièrement entre 32 et 40, dans le cas de X qui est DAB,
- entre 24 et 42, en particulier entre 28 et 42, plus particulièrement entre 34 et 42, dans le cas de X qui est DAP, ou
Y² est sélectionné parmi un -(NH)-C_{b}-alkyle, un -(NH)-C_{b}-alkényle, -(N)-(Cₑ-alkyle)₂ ou un -(N)-(Cₑ-alkényle)₂ dans le cas de X qui est Glu ou Asp, dans lesquelles la somme de e ou b est entre 22 et 40, en particulier entre 26 et 40, plus particulièrement entre 32 et 40 dans le cas de X qui est Glu, dans lesquelles la somme de e ou b est entre 24 et 42, en particulier entre 28 et 42, plus particulièrement entre 34 et 42, dans le cas de X qui est Asp,
- Y³ et Y⁴ sont indépendamment sélectionnés parmi un -C(=O)-Cₓ-alkyle et un - C(=O)-Cₓ-alkényle, dans le cas de X qui est Lys, Orn, DAB ou DAP, et x est
- entre 15 et 21, en particulier entre 15 et 17, dans le cas de X qui est Lys,
- entre 16 et 22, en particulier entre 16 et 18, dans le cas de X qui est Orn,
- entre 17 et 23, en particulier entre 17 et 19, dans le cas de X qui est DAB,
- entre 18 et 24, en particulier entre 18 et 20, dans le cas de X qui est DAP,
Y³ est sélectionné parmi un -(NH)-C_{c}-alkyle, un -(NH)-C_{c}-alkényle, -(N)-(C_{f}-alkyle)₂ ou un - (N)-(C_{f}-alkényle)₂ dans le cas de X qui est Glu ou Asp, dans lesquelles
- la somme de f est entre 17 et 23, en particulier entre 17 et 19, dans le cas de X qui est Glu,
- la somme de f est entre 18 et 24, en particulier entre 18 et 20, dans le cas de X qui est Asp,
- c est entre 17 et 23, en particulier entre 17 et 19, dans le cas de X qui est Glu,
- c est entre 18 et 24, en particulier entre 18 et 20, dans le cas de X qui est Asp,
- HP est un peptide hydrophobe constitué de 3 à 5 acides aminés hydrophobes,
- chaque B est Lys,
- chaque D indépendamment de tout autre D est un dipeptide constitué d'un acide aminé hydrophobe et d'un acide aminé cationique (HC ou CH), un dipeptide constitué de deux acides aminés cationiques (CC) ou un dipeptide constitué de deux acides aminés hydrophobes (HH).

2. Dendrimère peptidique selon la revendication 1, dans lequel X est Lys.

3. Dendrimère peptidique selon l'une quelconque des revendications précédentes, dans lequel le peptide hydrophobe est constitué d'acides aminés hydrophobes indépendamment sélectionnés parmi Cys, Leu, Trp, Phe, Nle (norleucine), Aoc (acide amino-octanoïque), en particulier parmi Leu, Phe, Trp, Nle, Aoc et Cys, plus particulièrement parmi Leu, Phe, Trp, Nle et Aoc.

4. Dendrimère peptidique selon l'une quelconque des revendications précédentes, dans lequel le peptide hydrophobe est constitué d'acides aminés hydrophobes identiques.

5. Dendrimère peptidique selon l'une quelconque des revendications précédentes, dans lequel la terminaison C du dendrimère peptidique est un carboxamide.

6. Dendrimère peptidique selon l'une quelconque des revendications précédentes, dans lequel en cas de HH, CH et HC
a. l'acide aminé hydrophobe dans D¹ est sélectionné parmi Leu et Nle, et
l'acide aminé cationique dans D¹ est Lys,
b. l'acide aminé hydrophobe dans D² est Leu, et
l'acide aminé cationique dans D² est sélectionné parmi Lys, Arg et His,
c. l'acide aminé hydrophobe dans D³ est Leu, et
l'acide aminé cationique dans D³ est sélectionné parmi Lys, Arg et His, et en cas de CC
l'acide aminé cationique dans D² ou D³ est sélectionné parmi Lys, Arg et His, dans lequel au moins un C est His.

7. Dendrimère peptidique selon l'une quelconque des revendications précédentes, dans lequel
- D¹ est un dipeptide constitué de deux acides aminés hydrophobes (HH) et D² et D³ sont chacun un dipeptide constitué d'un acide aminé cationique et d'un acide aminé hydrophobe (CH), ou
- chaque D¹, D² et D³ sont est un dipeptide constitué d'un acide aminé cationique et d'un acide aminé hydrophobe (CH).

8. Dendrimère peptidique selon l'une quelconque des revendications précédentes, dans lequel les acides aminés sont sélectionnés indépendamment l'un de l'autre parmi des acides (L)-aminés et acides (D)-aminés.

9. Dendrimère peptidique selon l'une quelconque des revendications précédentes, dans lequel le peptide est homochiral.

10. Procédé de transfection d'une cellule *ex vivo* comprenant les étapes consistant à
a. fournir un mélange de transfection comprenant un dendrimère peptidique selon l'une quelconque des revendications précédentes et un acide nucléique,
b. mettre en contact une cellule avec le mélange de transfection donnant une cellule transfectée.

11. Procédé selon la revendication 10, dans lequel le mélange de transfection est préparé en mélangeant une première solution comprenant le dendrimère peptidique selon l'une quelconque des revendications précédentes et une seconde solution comprenant l'acide nucléique.

12. Procédé selon l'une quelconque des revendications 10 à 11, dans lequel l'acide nucléique est de l'ADN ou ARN, en particulier de l'ADN circulaire (plasmide/vecteur), de l'ADN linéaire (ADNc), de l'ARN linéaire (ARNsi, ARNsa, ARNmi, ARNm, ARN long).

13. Procédé selon l'une quelconque des revendications 10 à 12, dans lequel le solvant de la première et/ou la seconde solution est de l'eau ou un milieu de culture cellulaire.

14. Procédé selon l'une quelconque des revendications 10 à 13, dans lequel la concentration finale en l'acide nucléique dans le mélange de transfection est entre 10 nM et 200 nM, en particulier 20 et 100 nM, plus particulièrement 40 et 80 nM.

15. Procédé selon l'une quelconque des revendications 10 à 14, dans lequel la concentration finale du dendrimère peptidique dans le mélange de transfection est entre 1,5 µg/ml et 30 µg/ml, en particulier entre 3 µg/ml et 15 µg/ml, plus particulièrement entre 6 µg/ml et 12 µg/ml.
